# EUROPEAN PATENT SPECIFICATION

(11) **EP 1 926 505 B1**
(45) Date of publication and mention of the grant of the patent: **01.02.2012**
(21) Application number: 06815019.2
(22) Date of filing: 21.09.2006
(51) Int. Cl.: A61L 27/20, A61L 27/54, A61K 9/00

(54) **IN VIVO FORMED MATRICES INCLUDING NATURAL BIODEGRADABLE POLYSACCHARIDES AND OPHTHALMIC USES THEREOF**
IN VIVO GEFORMTE MATRIZEN MIT NATÜRLICHEN BIOLOGISCH ABBAUBAREN POLYSACCHARIDEN UND IHRE OPHTHALMISCHEN VERWENDUNGEN
MATRICES FORMEES IN VIVO CONTENANT DES POLYSACCHARIDES BIODEGRADABLES NATURELS ET LEURS UTILISATIONS OPHTALMIQUES

(30) Priority: 21.09.2005 US 719466 P; 10.04.2006 US 791086 P
(43) Date of publication of application: 04.06.2008
(73) Proprietor: SurModics, Inc., Eden Prairie, MN 55344 (US)
(72) Inventor: VARNER, Signe, E., Los Angeles, CA 90065 (US); BEELEY, Nathan, R., F., Wynnewood, PA 19096 (US); CHUDZIK, Stephen, J., St. Paul, MN 55101 (US); BURKSTRAND, Michael, J., Richfield, MN 55423 (US)
(74) Representative: Stoner, Gerard Patrick
(86) International application number: PCT/US2006/036632
(87) International publication number: WO 2007/038126

(56) References cited:
- EP-A2- 0 244 178
- WO-A-93/09176
- WO-A-2005/034875
- WO-A2-99/07418
- US-A1- 2003 077 242
- US-A1- 2005 136 091

## Description

### Technical Field

The present invention relates to *in vivo* formed matrices comprising a natural biodegradable polymeric material. Bioactive agents included in the *in vivo* formed matrices provide a therapeutic effect to a patient. The formed matrices provide medical articles for implantation in the eye.

### Background

In recent years, much attention has been given to site-specific delivery of drugs within a patient. Although various drugs have been developed for treatment of a wide variety of ailments and diseases of the body, in many instances, such drugs cannot be effectively administered systemically without risk of detrimental side effects. Site-specific drug delivery focuses on delivering the drugs locally, i.e., to the area of the body requiring treatment. One benefit of the local release of bioactive agents is the avoidance of toxic concentrations of drugs that are at times necessary, when given systemically, to achieve therapeutic concentrations at the site where they are required.

Site-specific drug delivery can be accomplished by injection and/or implantation of an article or device that releases the drug to the treatment site. Injection of drugs can have limitations, for example, by requiring multiple administrations, increasing risk of complications (such as infection), and patient discomfort. Implantation of an article or device that delivers drug to the treatment site has therefore gained much interest in recent years.

Further, site-specific drug delivery has been enhanced by technologies that allow controlled release of one or more drugs from an implanted device or article. Controlled release can relate to the duration of time drug is released from the device or article, and/or the rate at which the drug is released.

For example, EP0244178 shows an injectable composition for drug delivery into the eye comprising a viscous solution of natural polysaccharides and a drug. The polysaccharide implantable drug release composition is preferably based on hyaluronic acid or a mixture of hyaluronic acid and chondroitin sulfate.

Several challenges confront the use of medical devices or articles that release bioactive agents into a patient's body. For example, treatment may require release of the bioactive agent(s) over an extended period of time (for example, weeks, months, or even years), and it can be difficult to sustain the desired release rate of the bioactive agent(s) over such long periods of time. Further, the device or article surface is preferably biocompatible and non-inflammatory, as well as durable, to allow for extended residence within the body.

Generally speaking, a bioactive agent can be associated with the surface of a medical device or article by surface modification, embedded and released from within polymeric materials (matrix-type), or surrounded by and released through a carrier (reservoir-type). The polymeric materials in such applications should optimally act as a biologically inert barrier and not induce further inflammation within the body. However, the molecular weight, porosity of the polymer, a greater percentage of coating exposed on the medical device or article, and the thickness of the polymer coating can contribute to adverse reactions to the medical device or article.

Another way to deliver bioactive agents from the surface of a medical device or article is by using a coating that has a biodegradable polymer, such as polylactic acid. As the coating degrades, the bioactive agent is released from the surface of the device or article. Some concerns exist that regard the use of biodegradable materials that degrade into materials that are not typically found in the body, or that are found at particularly low levels in the body. These types of biodegradable materials have the potential to degrade into products that cause unwanted side effects in the body by virtue of their presence or concentration *in vivo.* These unwanted side effects can include immune reactions, toxic buildup of the degradation products in the body, or the initiation or provocation of other adverse effects on cells or tissue in the body.

Another problem is that preparations of some biodegradable materials may not be obtained at consistent purity due to variations inherent in natural materials. This is relevant at least with regard to biodegradable materials derived from animal sources. Inconsistencies in preparations of biodegradable materials can result in problematic coatings.

Additional concerns are that preparations from animal sources may provide other unwanted contaminants, such as antigenic factors. These antigenic factors may promote a localized immune response in the vicinity of the implanted article and foul its function. These factors may also cause infection as well as local inflammation.

In particular, placement of implantable devices or articles in limited access regions of the body can present additional challenges. Limited access regions of the body can be characterized in terms of physical accessibility as well as therapeutic accessibility. For example, the relatively small size and sensitive tissues surrounding the eye can contribute to physical accessibility difficulties. In addition, ocular absorption of systemically administered pharmacologic agents is limited by the blood ocular barrier, namely the tight junctions of the retinal pigment epithelium and vascular endothelial cells. These can make accessing the eye with therapeutics difficult. High systemic doses of bioactive agents can penetrate this blood ocular barrier in relatively small amounts, but expose the patient to the risk of systemic toxicity. Intravitreal injection of bioactive agents (such as drugs) is an effective means of delivering a drug to the posterior segment of the eye in high concentrations. However, these repeated injections carry the risk of such complications as infection, hemorrhage, and retinal detachment. Patients also often find this procedure somewhat difficult to endure.

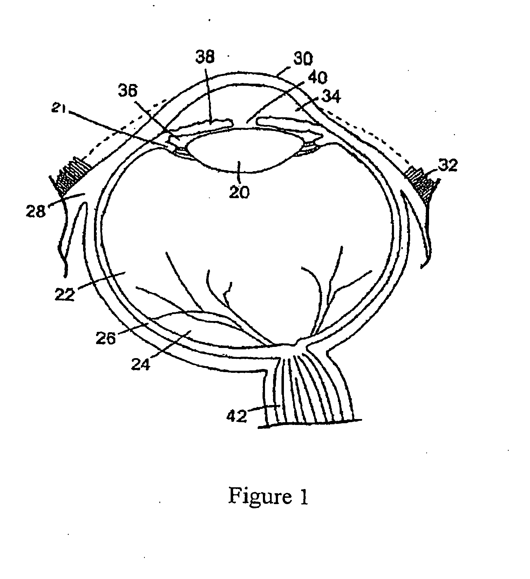

Because description of the invention will involve treatment of the eye as an illustrative embodiment, basic anatomy of the eye will now be described in some detail with reference to Figure 1, which illustrates a cross-sectional view of the eye. Beginning from the exterior of the eye, the structure of the eye includes the iris 38 that surrounds the pupil 40. The iris 38 is a circular muscle that controls the size of the pupil 40 to control the amount of light allowed to enter the eye. A transparent external surface, the cornea 30, covers both the pupil 40 and the iris 38. Continuous with the cornea 30, and forming part of the supporting wall of the eyeball, is the sclera 28 (the white of the eye). The pars plana is a region of the eye approximately 4 mm posterior to the point on the globe where the colored iris 38 meets the white sclera 28. The pars plana encircles the iris and is not constant in width, but rather typically varies between 2-3 mm in width around the iris (with the largest width of the pars plana typically lying on the temporal side and measuring about 3 mm in width).

The conjunctiva 32 is a clear mucous membrane covering the sclera 28. Within the eye is the lens 20, which is a transparent body located behind the iris 38. The lens 20 is suspended by ligaments attached to the anterior portion of the ciliary body 21. Light rays are focused through the transparent cornea 30 and lens 20 upon the retina 24. The central point for image focus (the visual axis) in the human retina is the fovea (not shown in the figures). The optic nerve 42 is located opposite the lens.

There are three different layers of the eye, the external layer, formed by the sclera 28 and cornea 30; the intermediate layer, which is divided into two parts, namely the anterior (iris 38 and ciliary body 21) and posterior (the choroid 26); and the internal layer, or the sensory part of the eye, formed by the retina 24. The sclera 28 is composed of dense, fibrous tissue and is composed of collagen fiber. Scleral thickness is approximately 1 mm posteriorly near the optic nerve and approximately 0.3 mm anteriorly. At the pars plana, the eye tissues are composed of sclera only; there is no choroidal or retinal tissue layer within this region. For this reason, the avascular pars plana is typically selected for implantation and/or injection of materials into the interior (vitreous) of the eye.

The lens 20 divides the eye into the anterior segment (in front of the lens) and the posterior segment (behind the lens). More specifically, the eye is composed of two chambers of fluid: the anterior chamber 34 (between the cornea 30 and the iris 38), and the vitreous chamber 22 (between the lens 20 and the retina 24). The anterior chamber 34 is filled with aqueous humor whereas the vitreous chamber 22 is filled with a more viscous fluid, the vitreous humor.

The vitreous chamber 22 is the largest chamber of the eye, consisting of approximately 4.5 ml of fluid. The vitreous chamber is filled with a transparent gel composed of a random network of thin collagen fibers in a highly dilute solution of salts, proteins and hyaluronic acid (the vitreous humor comprises approximately 98% water).

### Summary of the Invention

The present invention according to claims 1 and 15 provides compositions for preparing biodegradable articles within a patient's eye. These medical articles deliver bioactive agents to the eye. These bioactive agent delivery compositions include a natural biodegradable polysaccharide as a component that can be crosslinked *in situ* to form a matrix from which a therapeutic material such as a drug, a biomolecule, or cells (referred to herein as a "bioactive agents") can be released or retained. In some embodiments of the invention, a bioactive agent is present in and can be released from the biodegradable matrix; in other embodiments a bioactive agent is present in a biodegradable microparticle, the microparticle being immobilized within the matrix.

The natural biodegradable polysaccharide is used to prepare an article within the body by *in situ* formation, In some aspects, the article can be amorphous, such as a polymerized mass of natural biodegradable polysaccharides that is formed within or on a portion of the body, by using an *in vivo* matrix-forming composition.

In some aspects, the *in vivo* formed matrix is used in methods for the treatment of any one or more of a variety of medical conditions or indications, including retinal detachment; occlusions; proliferative retinopathy; proliferative vitreoretinopathy; diabetic retinopathy; inflammations such as uveitis, choroiditis, and retinitis; degenerative disease (such as age-related macular degeneration, also referred to as AMD); vascular diseases; and various tumors including neoplasms. In yet further embodiments, the biodegradable medical article can be used post-operatively, for example, as a treatment to reduce or avoid potential complications that can arise from ocular surgery. In one such embodiment, the medical article can be provided to a patient after cataract surgical procedures, to assist in managing (for example, reducing or avoiding) post-operative inflammation.

Illustrative bioactive agents include antiproliferative agents, anti-inflammatory agents, anti-angiogenic agents, hormonal agents, antibiotics, neurotrophic factors, or combinations thereof. Exemplary antiproliferative agents include 13-cis retinoic acid, retinoic acid derivatives, taxol, sirolimus (rapamycin), analogues of rapamycin, tacrolimus, ABT-578, everolimus, paclitaxel, taxane, and vinorelbine. Exemplary anti-inflammatory agents include hydrocortisone, hydrocortisone acetate, dexamethasone 21-phosphate, fluocinolone; medrysone, methylprednisolone, prednisolone 21-phosphate, prednisolone acetate, fluoromethalone, betamethasone, triamcinolone, and triamcinolone acetonide. Exemplary inhibitors of angiogensis include angiostatin, anecortave acetate, thrombospondin, anti-VEGF antibody, and anti-VEGF fragment. Exemplary hormonal agents include estrogens, estradiol, progesterol, progesterone, insulin, calcitonin, parathyroid hormone, peptide, and vasopressin hypothalamus releasing factor.

In some aspects, the biodegradable medical article can include a radiopacifying agent.

In preparing the biodegradable compositions, a plurality of natural biodegradable polysaccharides are crosslinked to each other via coupling groups that are pendent from the natural biodegradable polysaccharide (i.e., one or more coupling groups are chemically bonded to the polysaccharide). In some aspects, the coupling group on the natural biodegradable polysaccharide is a polymerizable group. In a free radical polymerization reaction the polymerizable group can crosslink natural biodegradable polysaccharides together in the composition, thereby forming a natural biodegradable polysaccharide matrix, which can be an *in-vivo* formed matrix.

The natural biodegradable polysaccharides described herein are non-synthetic polysaccharides that can be associated with each other to form a matrix, which can be used as an *in-vivo* formed matrix. The natural biodegradable polysaccharides can also be enzymatically degraded, but offer the advantage of being generally non-enzymatically hydrolytically stable. This is particularly advantageous for bioactive agent delivery, as in some aspects the invention provides articles capable of releasing the bioactive agent under conditions of enzyme-mediated degradation, but not by diffusion. Therefore, the kinetics of bioactive agent release from the articles of the invention are fundamentally different than those of coatings or medical implants prepared from synthetic biodegradable materials, such as poly(lactides).

Natural biodegradable polysaccharides include polysaccharide and/or polysaccharide derivatives that are obtained from natural sources, such as plants or animals. Exemplary natural biodegradable polysaccharides include amylose, maltodextrin, amylopectin, starch, dextran, hyaluronic acid, heparin, chondroitin sulfate, dermatan sulfate, heparan sulfate, keratan sulfate, dextran sulfate, pentosan polysulfate, and chitosan. Preferred polysaccharides are low molecular weight polymers that have little or no branching, such as those that are derived from and/or found in starch preparations, for example, amylose and maltodextrin.

Because of the particular utility of the amylose and maltodextrin polymers, in some aspects natural biodegradable polysaccharides are used that have an average molecular weight of 500,000 Da or less, 250,000 Da or less, 100,000 Da or less, or 50,000 Da or less. In some aspects the natural biodegradable polysaccharides have an average molecular weight of 500 Da or greater. In some aspects the natural biodegradable polysaccharides have an average molecular weight in the range of about 1000 Da to about 10,000 Da. Natural biodegradable polysaccharides of particular molecular weights can be obtained commercially or can be prepared, for example, by acid hydrolysis and/or enzymatic degradation of a natural biodegradable polysaccharide preparation, such as starch. The decision of using natural biodegradable polysaccharides of a particular size range may depend on factors such as the physical characteristics of the biodegradable composition (e.g., viscosity), the desired rate of degradation of the composition, the presence of other optional moieties in the composition (for example, bioactive agents, etc.), and the like.

The natural biodegradable polysaccharides that are used in accordance with the methods and compositions of the invention are readily available at a low cost and/or can be prepared easily using established techniques. This allows for a cost effective method of fabricating medical articles.

The use of natural biodegradable polysaccharides, such as maltodextrin or amylose, provides many advantages when used for the formation of an article, such as one that can be formed and used *in vivo.* Degradation of a natural biodegradable polysaccharide-containing article can result in the release of, for example, naturally occurring mono- or disaccharides, such as glucose, which are common components of bodily fluids, such as the vitreous humor. Furthermore, the use of natural biodegradable polysaccharides that degrade into common components found in bodily fluids, such as glucose, can be viewed as more acceptable than the use of synthetic biodegradable polysaccharides that degrade into non-natural compounds, or compounds that are found at very low concentrations in the body.

In some aspects of the invention, this advantageous feature is reflected in the use of natural biodegradable polysaccharides which are non-animal derived, such as amylose and maltodextrin, and that degrade into products that present little or no immunogenic or toxic risk to the individual. The invention provides improved, cost-efficient, natural biodegradable polysaccharide compositions for articles that can be used in a variety of medical treatments.

Another advantage of the invention is that the natural biodegradable polysaccharide-based compositions are more resistant to hydrolytic degradation than other biodegradable polymers, such as poly(lactides). Degradation of the natural biodegradable polysaccharides of the invention are primarily enzyme-mediated, with minimal or no hydrolysis of the natural biodegradable polysaccharide occurring when a natural biodegradable polysaccharide-containing composition is prepared under ambient conditions. This allows the natural biodegradable polysaccharide-based compositions to remain substantially stable (for example, resistant to degradation) prior to forming the medical article *in vivo.* For example, a natural biodegradable polysaccharide composition can be manipulated in a non-biological, aqueous-based-medium without risk that the composition will prematurely degrade due to non-enzyme-meditated hydrolysis. Other compositions that are based on biodegradable polymers such as poly(lactide) or poly(lactide-co-glycolide) are subject to hydrolysis even at relatively neutral pH ranges (e.g., pH 6.5 to 7.5) and therefore do not offer this advantage.

Therefore, the invention includes natural biodegradable polysaccharide-containing compositions, articles, and methods of preparing such that have the advantage of providing stability in the presence of an aqueous environment.

In one aspect, the invention provides a shelf-stable composition for preparing a biodegradable article, the shelf stable composition comprising a natural biodegradable polysaccharide comprising coupling groups. These compositions could be obtained or prepared, according to the details provided herein, and then stored for a period of time before the composition is used to form a biodegradable article, without significant degradation of the natural biodegradable polysaccharide occurring during storage. Accordingly, the invention also provides methods for preparing a biodegradable medical article comprising preparing a biodegradable composition comprising a natural biodegradable polysaccharide comprising coupling group; storing the biodegradable composition for an amount of time; and then using the biodegradable composition to prepare a biodegradable article. In some aspects, the biodegradable article is formed *in situ,* for example, by promoting the polymerization of the natural biodegradable polysaccharide within the body. Optionally, one or more bioactive agents and/or microparticles can be added before or after storage of the biodegradable composition.

In a related aspect, the invention also provides the advantage of being able to perform methods wherein the natural biodegradable polysaccharide is subject to exposure to an aqueous solution without risking significant degradation of the natural biodegradable polysaccharide. For example, the natural biodegradable polysaccharide may be contacted with an aqueous solution in a synthetic or post-synthetic step, including addition synthesis reactions and purification steps, or a composition that includes the natural biodegradable polysaccharide can be contacted with an aqueous solution in, for example, a sterilization step or a step that involves incorporation of a bioactive agent into the biodegradable composition.

The invention also provides a useful way to deliver larger hydrophilic bioactive agents, such as polypeptides, nucleic acids, and polysaccharides, as well as viral particles and cells from the biodegradable article. Comparatively, the use of non-degrading drug delivery matrices may not be useful for the delivery of these larger bioactive agents if they are too large to diffuse out of the matrix. However, according to some aspects of the invention, an article that includes a matrix of the natural biodegradable polysaccharide having a bioactive agent can be formed in the body, and as the matrix degrades the bioactive agent is gradually released from the matrix. In one aspect of the invention, the bioactive agent has a molecular weight of about 10,000 Da or greater.

In some aspects, the invention provides a bioactive agent-releasing biodegradable ophthalmic article or composition comprising (i) a natural biodegradable polysaccharide, preferably selected from amylose and maltodextrin, comprising an ethylenically unsaturated group, (ii) an initiator, and (iii) a bioactive agent selected from the group of polypeptides, polynucleotides, and polysaccharides.

Articles fabricated from the biodegradable polysaccharides can have favorable bioactive agent-releasing properties when the article is formed within the body. In this regard, the present invention provides an overall improvement in terms of providing implantable medical articles having bioactive agent delivery capabilities.

In another aspect of the invention, the natural biodegradable polysaccharide is modified with a hydrophobic moiety in order to provide a biodegradable matrix having hydrophobic properties. Therefore, a biodegradable article can be formed from natural biodegradable polysaccharide comprising one or more pendent coupling groups and one or more pendent hydrophobic moieties. Exemplary hydrophobic moieties include fatty acids and derivatives thereof, and C₂-C₁₈ alkyl chains.

Therefore, in some aspects of the invention, modification of the natural biodegradable polysaccharide allows for preparation of articles that are biodegradable and that can release a hydrophobic bioactive agent.

In other aspects, the hydrophobic moiety pendent from the natural biodegradable has properties of a bioactive agent. Upon degradation of the matrix, the hydrophobic moiety can be hydrolyzed from the natural biodegradable polymer and released to provide a therapeutic effect. Illustrative therapeutically useful hydrophobic moieties include butyric acid, valproic acid, retinoic acid, and the like.

In yet another aspect, the invention provides articles for improving the stability of a bioactive agent that is delivered from an article by utilizing a natural biodegradable non-reducing polysaccharide. The non-reducing polysaccharide can provide an inert matrix and thereby improve the stability of sensitive bioactive agents, such as proteins and enzymes. The article can include a matrix having a plurality of natural biodegradable non-reducing polysaccharides along with a bioactive agent, such as a polypeptide. An exemplary non-reducing polysaccharide comprises polyalditol. Biodegradable non-reducing polysaccharides can be useful for formulating articles that release the bioactive agent over a prolonged period of time.

While it is desirable to make articles that provide desired properties (for example, bioactive agent release, wettability, and the like), their actual preparation can be challenging. In particular, the use of some polysaccharides for preparing coatings or articles may result in products that are unsuitable for use. For example, some polysaccharide-based compositions, including those made from starch-based materials, have the potential to be overly brittle and inflexible. While these properties may be suitable for pharmaceutical capsules or tablets, they are generally undesirable as properties for medical articles, such as bioactive agent releasing medical implants.

Despite this, the present invention demonstrates the preparation of articles that include natural biodegradable polysaccharides that are suitable for *in vivo* formation and use. These products display excellent physical characteristics and are suitable for use in applications wherein a particular function, such as bioactive agent delivery is desired. For example, articles can be prepared having viscoelastic properties. In one aspect of the invention, the article has an elastic modulus value in the range of 27 kPa to 30 kPa.

Methods of preparing the compositions for fabrication of articles do not require the use of organic solvents. The use of organic solvents can be physically hazardous. Use of organic solvents can potentially destroy the activity of a bioactive agent in the natural biodegradable polysaccharide-based composition.

Many of the advantageous features of the present natural biodegradable polysaccharide-containing articles are thought to be provided by the starting materials, in particular the natural biodegradable polysaccharides having pendent coupling groups. In some aspects the natural biodegradable polysaccharides have pendent polymerizable groups, such as ethylenically unsaturated groups. In a preferred aspect, the degradable polymerizable polymers (macromers) are formed by reacting a natural biodegradable polysaccharide with a compound comprising an ethylenically unsaturated group. For example, in some cases, a natural biodegradable polysaccharide is reacted with a compound including an ethylenically unsaturated group and an isocyanate group. In another example of synthesis, a natural biodegradable polysaccharide is treated with an oxidizing agent to form a reactive aldehyde species on the polysaccharide and then reacted with a compound comprising an ethylenically unsaturated group and an amine group. Polysaccharide macromers were shown to have excellent matrix forming capabilities.

Synthesis can be carried out to provide the natural biodegradable polysaccharide with a desired quantity of pendent coupling groups. It has been found that use of a natural biodegradable polysaccharide having a predetermined amount of the coupling groups allows for the formation of an article having desirable physical characteristics (for example, the articles are not brittle). Therefore, in some aspects, the invention provides natural biodegradable polysaccharides having an amount of pendent coupling groups of about 0.7 µmoles of coupling group per milligram of natural biodegradable polysaccharide. Preferably the amount of coupling group per natural biodegradable polysaccharide is in the range of about 0.3 µmoles/mg to about 0.7 µmoles/mg. For example, amylose or maltodextrin can be subject to a synthesis reaction with a compound having an ethylenically unsaturated group to provide an amylose or maltodextrin macromer having a ethylenically unsaturated group load level in the range of about 0.3 µmoles/mg to about 0.7 µmoles/mg.

In the invention an initiator is used to promote the formation of the natural biodegradable polysaccharide matrix for article formation. The initiator can be an independent compound or a pendent chemical group used to activate the coupling group pendent from the natural biodegradable polymer and promote coupling of a plurality of natural biodegradable polymers. When the coupling group pendent from the natural biodegradable polysaccharide is a polymerizable group, the initiator can be used in a free radical polymerization reaction to promote crosslinking of the natural biodegradable polysaccharides together in the composition.

Therefore, in one aspect, the invention provides a biodegradable composition for forming an ophthalmic article comprising (i) a natural biodegradable polysaccharide, preferably selected from amylose and maltodextrin, comprising a coupling group, (ii) an initiator, and (iii) a bioactive agent, wherein the coupling group is able to be activated by the initiator and promote crosslinking of a plurality of natural biodegradable polysaccharides. In some aspects of the invention the initiator is independent of the natural biodegradable polysaccharide and in other aspects the initiator is pendent from the natural biodegradable polysaccharide. Preferably, the natural biodegradable polysaccharide comprises an ethylenically unsaturated group. In some aspects a photoinitiator is used, such as a photoinitiator that is activated by light wavelengths having no or a minimal effect on the bioactive agent resent in the composition and/or tissues of the eye.

In some aspects, the invention provides compositions as defined in claim 1 for use in forming a biodegradable implant *in situ*, in an eye of a patient, the use comprising steps of:
(a) administering a composition to a patient, the composition comprising
   (i) a natural biodegradable polysaccharide comprising a coupling group,
   (ii) an initiator, and
   (iii) a bioactive agent;
(b) activating the initiator to couple the natural biodegradable polysaccharides present in the composition, thereby forming a solid implant within the eye of the patient.

In another aspect, the initiator includes an oxidizing agent/reducing agent pair, a "redox pair," to drive polymerization of the biodegradable polysaccharide. In preparing the biodegradable article the oxidizing agent and reducing agent are combined in the presence of the biodegradable polysaccharide. One benefit of using a redox pair is that, when combined, the oxidizing agent and reducing agent can provide a particularly robust initiation system. This is advantageous as it can promote the formation of a matrix, for example, useful for medical article preparation, from biodegradable polysaccharide compositions having a relatively low viscosity. This can be particularly useful in many applications, especially when the biodegradable polysaccharide composition is used for the formation of an *in situ* polymerized article. For example, a low viscosity composition can be passed through a delivery conduit having a small inner diameter with relative ease to provide the composition that can polymerize *in situ.*

In some aspects of the invention, the viscosity of the composition is above about 5 centi Poise (cP), or about 10 cP or greater. In other aspects of the invention the viscosity of the composition is between about 5 cP or 10 cP and about 700 cP, and in some aspects between about 5 cP or 10 cP and about 250 cP. In some aspects the viscosity of the composition is above about 5 cP or 10 cP and the biodegradable polysaccharides in the composition have an average molecular weight of 500,000 Da or less, 250,000 Da or less, 100,000 Da or less, or 50,000 Da or less.

In some aspects of the invention, the composition is injectable through a cannula having an outer diameter of about 0.5 mm or less. This can be particularly beneficial when it is desirable to minimize the size of any incision in the body, thereby reducing or avoiding the use of sutures or other closure devices.

Polymerization of the composition can be induced by a variety of means such as irradiation with light of suitable wavelength or by contacting members of a reactive pair (e.g., a redox pair). When irradiation is employed, UV irradiation is preferred. UV irradiation can be accomplished in the visible or long ultraviolet (LWUV) wavelength range using standard ophthalmic light sources. With standard ophthalmic light sources having wavelengths in the visible or long ultraviolet wavelength range, polymerization generally occurs in about two (2) seconds to about three (3) minutes, usually in about five (5) seconds to about thirty (30) seconds, typically at an exposure distance of about 2 cm or less.

In some aspects, the power and wavelength of light are selected to provide a suitable curing time for the biodegradable polysaccharide composition. Suitable curing time is generally a time sufficient so that matrix is cured into a stable polymeric network within a suitable working time for a surgeon.

When polymerization is initiated by a reactive pair (such as a redox pair), typical curing times can be in the range of about one (1) second to about ten (10) minutes. Depending upon the particular redox pair selected, polymerization can be initiated almost instantaneously upon contact of the members of the redox pair.

A method for preparing a medical article *in situ* in an eye of a patient can include the steps of (a) providing a first composition that includes a natural biodegradable polysaccharide comprising a polymerizable group and a first member of a redox pair (for example, the oxidizing agent); (b) providing a second composition comprising a natural biodegradable polysaccharide comprising a polymerizable group, and a second member of a redox pair; (c) administering the first composition, the second composition, or a mixture of the first and second composition in liquid form into the eye of a patient; and (d) contacting the first composition with the second composition where, in the step of contacting, the redox pair initiates polymerization of the natural biodegradable polysaccharides, thereby forming a solid implant within the eye. For example, the first composition can include (a) a natural biodegradable polysaccharide having a coupling group and an oxidizing agent and the second composition can include a (b) natural biodegradable polysaccharide having a coupling group and a reducing agent. In some aspects, when the first composition is combined with the second composition, the viscosity of the finial composition can be about 5 cP or greater.

The oxidizing agent can be selected from inorganic or organic oxidizing agents, including enzymes; the reducing agent can be selected from inorganic or organic reducing agents, including enzymes. Exemplary oxidizing agents include peroxides, including hydrogen peroxide, metal oxides, and oxidases, such as glucose oxidase. Exemplary reducing agents include salts and derivatives of electropositive elemental metals such as Li, Na, Mg, Fe, Zn, Al, and reductases. In one aspect, the reducing agent is present in the composition at a concentration of 2.5 mM or greater when mixed with the oxidizing agent. Other reagents, such as metal or ammonium salts of persulfate, can be present in the composition to promote polymerization of the biodegradable polysaccharide.

An article formed using redox polymerization can therefore comprise a plurality of natural biodegradable polysaccharides associated via polymerized groups, a reduced oxidizing agent, and an oxidized reducing agent.

For example, an alternative method for forming an article can include combining (a) a natural biodegradable polysaccharide comprising a first coupling group with (b) a natural biodegradable polysaccharide comprising a second coupling group that is reactive with the first coupling group, and (c) a bioactive agent. The article can be partially or fully formed when reagent (a) reacts with (b) to link the natural biodegradable polysaccharides together to form the article, which includes reagent (c), the bioactive agent.

In some aspects, the present invention employs the use of biodegradable microparticles that include a bioactive agent and a natural biodegradable polysaccharide, such as amylose and maltodextrin that have pendent coupling groups. The microparticles are used in association with the natural biodegradable polysaccharides to prepare a biodegradable, bioactive agent-releasing medical article.

According to this aspect of the invention, a medical article that includes a crosslinked matrix of natural biodegradable polysaccharides and biodegradable microparticles having a bioactive agent can be formed in the body, and as the biodegradable microparticles degrade the bioactive agent is gradually released from the medical article.

The natural biodegradable polysaccharide matrix provides the ability to associate the biodegradable microparticles with the medical article. For example, microparticles can be included in an implantable medical article that is formed *in situ.* In some arrangements, the biodegradable microparticles are dispersed in the natural biodegradable polysaccharide matrix. Such coatings can be formed by forming a mixture of (a) biodegradable microparticles having a bioactive agent and (b) natural biodegradable polysaccharides having pendent coupling groups, and then treating the composition to form a biodegradable matrix wherein the biodegradable microparticles are dispersed within the matrix.

By including microparticles having a bioactive agent in the natural biodegradable polysaccharide-containing matrix, the invention also provides a way to effectively and efficiently prepare a variety of drug-delivery medical articles. The use of microparticles offers the ability to easily prepare medical articles having one or more bioactive agents present in desired amounts in the article: Such medical articles can be prepared by obtaining biodegradable microparticles that have a bioactive agent and then forming a medical article that includes the microspheres associated with the natural biodegradable polysaccharide matrix. In some aspects, different microparticles having different bioactive agents can be included in the medical article in desired amounts to provide a bioactive agent-releasing medical article that is able to release a desired combination of bioactive agents in desired amounts. This is a particular advantage when using bioactive agents that are typically not compatible in the same composition (for example, bioactive agents that have different physical properties).

These and other aspects and features of the invention will now be described in more detail.

### Brief Description of the Drawings

Figure 1 is an illustration of a cross-sectional view of the eye.
Figure 2 is a graph of cumulative BSA release from maltodextrin-acrylate filaments treated with amylase, over a period of time.
Figure 3 is a graph of cumulative absorbance values of active and total IgG Fab fragment release from maltodextrin-acrylate filaments treated with amylase, over a period of time.
Figure 4 is a graph of cumulative absorbance values of active and total IgG release from a maltodextrin-acrylate filament treated with amylase and percent degradation of the filament, over a period of time.
Figure 5 is a graph of modulus of a maltodextrin-acrylate matrix formed via redox polymerization, over a period of time.

### Detailed Description

The invention provides flowable compositions for forming a biodegradable implant in situ, in an eye of a patient, according to claims 1 and 15.

The composition is thus sufficiently flowable to be administered (e.g., by injection) to a targeted site within a patient, where it is subsequently treated to form a solid implant at the targeted site. The composition includes a natural biodegradable polysaccharide having a coupling group. Exemplary natural biodegradable polysaccharides include amylose and maltodextrin. In some aspects, the present invention provides biodegradable medical articles having excellent physical characteristics (such as optical transparency, elasticity, and the like) and that can provide a suitable vehicle for the delivery of bioactive agents. Components of the composition will now be described.

As referred to herein, a "natural biodegradable polysaccharide" refers to a non-synthetic polysaccharide that is capable of being enzymatically degraded but that is generally non-enzymatically hydrolytically stable. Natural biodegradable polysaccharides include polysaccharide and/or polysaccharide derivatives that are obtained from natural sources, such as plants or animals. Natural biodegradable polysaccharides include any polysaccharide that has been processed or modified from a natural biodegradable polysaccharide (for example, maltodextrin is a natural biodegradable polysaccharide that is processed from starch). Exemplary natural biodegradable polysaccharides include hyaluronic acid, starch, dextran, heparin, chondroitin sulfate, dermatan sulfate, heparan sulfate, keratan sulfate, dextran sulfate, pentosan polysulfate, and chitosan. Preferred polysaccharides are low molecular weight polymers that have little or no branching, such as those that are derived from and/or found in starch preparations, for example, amylose and maltodextrin. Therefore, the natural biodegradable polysaccharide can be a substantially non-branched or non-branched poly(glucopyranose) polymer.

Because of the particular utility of the amylose and maltodextrin polymers, it is preferred that natural biodegradable polysaccharides having an average molecular weight of 500,000 Da or less, 250,000 Da or less, 100,000 Da or less, or 50,000 Da or less. It is also preferred that the natural biodegradable polysaccharides have an average molecular weight of 500 Da or greater. A particularly preferred size range for the natural biodegradable polysaccharides is in the range of about 1000 Da to about 10,000 Da. Natural biodegradable polysaccharides of particular molecular weights can be obtained commercially or can be prepared. The decision of using natural biodegradable polysaccharides of a particular size range may depend on factors such as the physical characteristics of the biodegradable composition (e.g., viscosity), the desired rate of degradation of the medical article, the presence of other optional moieties in the biodegradable composition, for example, bioactive agents, etc.

As used herein, "amylose" or "amylose polymer" refers to a linear polymer having repeating glucopyranose units that are joined by α-1,4 linkages. Some amylose polymers can have a very small amount of branching via α-1,6 linkages (about less than 0.5% of the linkages) but still demonstrate the same physical properties as linear (unbranched) amylose polymers do. Generally amylose polymers derived from plant sources have molecular weights of about 1 X 10⁶ Da or less. Amylopectin, comparatively, is a branched polymer having repeating glucopyranose units that are joined by α-1,4 linkages to form linear portions and the linear portions are linked together via α-1,6 linkages. The branch point linkages are generally greater than 1% of the total linkages and typically 4% - 5% of the total linkages. Generally amylopectin derived from plant sources have molecular weights of 1 X 10⁷ Da or greater.

Amylose can be obtained from, or is present in, a variety of sources. Typically, amylose is obtained from non-animal sources, such as plant sources. In some aspects, a purified preparation of amylose is used as starting material for the preparation of the amylose polymer having coupling groups. In other aspects, as starting material, amylose can be used in a mixture that includes other polysaccharides.

For example, in some aspects, starch preparations having a high amylose content, purified amylose, synthetically prepared amylose, or enriched amylose preparations can be used in the preparation of amylose having the coupling groups. In starch sources, amylose is typically present along with amylopectin, which is a branched polysaccharide. According to the invention, it is preferred to use coating compositions that include amylose, wherein the amylose is present in the composition in an amount greater than amylopectin, if present in the composition. For example, in some aspects, starch preparations having high amylose content, purified amylose, synthetically prepared amylose, or enriched amylose preparations can be used in the preparation of amylose polymer having the coupling groups. In some embodiments the composition includes a mixture of polysaccharides including amylose wherein the amylose content in the mixture of polysaccharides is 50% or greater, 60% or greater, 70% or greater, 80% or greater, or 85% or greater by weight. In other embodiments the composition includes a mixture of polysaccharides including amylose and amylopectin and wherein the amylopectin content in the mixture of polysaccharides is 30% or less, or 15% or less.

In some cases it may be desirable to use non-retrograding starches, such as waxy starch, in the current invention. The amount of amylopectin present in a starch may also be reduced by treating the starch with amylopectinase, which cleaves α-1,6 linkages resulting in the debranching of amylopectin into amylose.

In some cases a synthesis reaction can be carried out to prepare an amylose polymer having pendent coupling groups (for example, amylose with pendent ethylenically unsaturated groups) and steps may be performed before, during, and/or after the synthesis to enrich the amount of amylose, or purify the amylose.

Amylose of a particular size, or a combination of particular sizes can be used. The choice of amylose in a particular size range may depend on the application, for example, the type of surface coated or the porosity of the surface. In some embodiments amylose having an average molecular weight of 500,000 Da or less, 250,000 Da or less, 100,000 Da or less, 50,000 Da or less, preferably greater than 500 Da, or preferably in the range of about 1000 Da to about 10,000 Da is used. Amylose of particular molecular weights can be obtained commercially or can be prepared. For example, synthetic amyloses with average molecular masses of 70, 110, 320, and 1,000 kDa can be obtained from Nakano Vinegar Co., Ltd. (Aichi, Japan). The decision of using amylose of a particular size range may depend on factors such as the physical characteristics of the biodegradable composition (e.g., viscosity), the desired rate of degradation of the medical article, the presence of other optional moieties in the biodegradable composition (for example, bioactive agents, etc.), etc.

Maltodextrin is typically generated by hydrolyzing a starch slurry with heat-stable α-amylase at temperatures at 85 - 90°C until the desired degree of hydrolysis is reached and then inactivating the α-amylase by a second heat treatment. The maltodextrin can be purified by filtration and then spray dried to a final product. Maltodextrins are typically characterized by their dextrose equivalent (DE) value, which is related to the degree of hydrolysis defined as: DE = MW dextrose/number-averaged MW starch hydrolysate x 100.

A starch preparation that has been totally hydrolyzed to dextrose (glucose) has a DE of 100, where as starch has a DE of about zero. A DE of greater than 0 but less than 100 characterizes the mean-average molecular weight of a starch hydrolysate, and maltodextrins are considered to have a DE of less than 20. Maltodextrins of various molecular weights, for example, in the range of about 500 - 5000 Da are commercially available (for example, from CarboMer, San Diego, CA).

Another contemplated class of natural biodegradable polysaccharides is natural biodegradable non-reducing polysaccharides. A non-reducing polysaccharide can provide an inert matrix thereby improving the stability of sensitive bioactive agents, such as proteins and enzymes. A non-reducing polysaccharide refers to a polymer of non-reducing disaccharides (two monosaccharides linked through their anomeric centers) such as trehalose (α-D-glucopyranosyl α-D-glucopyranoside) and sucrose β-D-fructofuranosyl α-D-glucopyranoside). An exemplary non-reducing polysaccharide comprises polyalditol which is available from GPC (Muscatine, Iowa). In another aspect, the polysaccharide is a glucopyranosyl polymer, such as a polymer that includes repeating (1→3)O-β-D-glucopyranosyl units.

In some aspects, the biodegradable compositions can include natural biodegradable polysaccharides that include chemical modifications other than the pendent coupling group. To exemplify this aspect, modified amylose having esterified hydroxyl groups can be prepared and used in biodegradable compositions in association with the methods of the invention. Other natural biodegradable polysaccharides having hydroxyl groups may be modified in the same manner. These types of modifications can change or improve the properties of the natural biodegradable polysaccharide making for a biodegradable composition that is particularly suitable for a desired application. Many chemically modified amylose polymers, such as chemically modified starch, have at least been considered acceptable food additives.

As used herein, "modified natural biodegradable polysaccharides" refers to chemical modifications to the natural biodegradable polysaccharide that are different than those provided by the coupling group or the initiator group. Modified amylose polymers having a coupling group (and/or initiator group) can be used in the compositions and methods of the invention.

To exemplify this aspect, modified amylose is described. By chemically modifying the hydroxyl groups of the amylose, the physical properties of the amylose can be altered. The hydroxyl groups of amylose allow for extensive hydrogen bonding between amylose polymers in solution and can result in viscous solutions that are observed upon heating and then cooling amylose-containing compositions such as starch in solution (retrograding). The hydroxyl groups of amylose can be modified to reduce or eliminate hydrogen bonding between molecules thereby changing the physical properties of amylose in solution.

Therefore, in some embodiments the natural biodegradable polysaccharides, such as amylose, can include one or more modifications to the hydroxyl groups wherein the modifications are different than those provided by coupling group. Modifications include esterification with acetic anhydride (and adipic acid), succinic anhydride, 1-octenylsuccinic anhydride, phosphoryl chloride, sodium trimetaphosphate, sodium tripolyphosphate, and sodium monophosphate; etherification with propylene oxide, acid modification with hydrochloric acid and sulfuric acids; and bleaching or oxidation with hydrogen peroxide, peracetic acid, potassium permanganate, and sodium hypochlorite.

Examples of modified amylose polymers include carboxymethyl amylose, carboxyethyl amylose, ethyl amylose, methyl amylose, hydroxyethyl amylose, hydroxypropyl amylose, acetyl amylose, amino alkyl amylose, allyl amylose, and oxidized amylose. Other modified amylose polymers include succinate amylose and oxtenyl succinate amylose.

In another aspect of the invention, the natural biodegradable polysaccharide is modified with a hydrophobic moiety in order to provide a biodegradable matrix having hydrophobic properties. Exemplary hydrophobic moieties include those previously listed, fatty acids and derivatives thereof, and C₂-C₁₈ alkyl chains. A polysaccharide, such as amylose or maltodextrin, can be modified with a compound having a hydrophobic moiety, such as a fatty acid anhydride. The hydroxyl group of a polysaccharide can also cause the ring opening of lactones to provide pendent open-chain hydroxy esters.

In some aspects, the hydrophobic moiety pendent from the natural biodegradable has properties of a bioactive agent. The hydrophobic moiety can be hydrolyzed from the natural biodegradable polymer and released from the matrix to provide a therapeutic effect. One example of a therapeutically useful hydrophobic moiety is butyric acid, which has been shown to elicit tumor cell differentiation and apoptosis, and is thought to be useful for the treatment of cancer and other blood diseases. Other illustrative hydrophobic moieties include valproic acid and retinoic acid. Retinoic acid is known to possess antiproliferative effects and is thought to be useful for treatment of proliferative vitreoretinopathy (PVR). The hydrophobic moiety that provides a therapeutic effect can also be a natural compound (such as butyric acid, valproic acid, and retinoic acid). Therefore, degradation of the matrix having a coupled therapeutic agent can result in all natural degradation products.

In further aspects, the natural biodegradable polysaccharide can be modified with a corticosteroid. In these aspects, a corticosteroid, such as triamcinolone, can be coupled to the natural biodegradable polymer. One method of coupling triamcinolone to a natural biodegradable polymer is by employing a modification of the method described in Cayanis, E. et al., *Generation of an Auto-anti-idiotypic Antibody that Binds to Glucocorticoid Receptor,* The Journal of Biol. Chem., 261(11): 5094-5103 (1986). Triamcinolone hexanoic acid is prepared by reaction of triamcinolone with ketohexanoic acid; an acid chloride of the resulting triamcinolone hexanoic acid can be formed and then reacted with the natural biodegradable polymer, such as maltodextrin or polyalditol, resulting in pendent triamcinolone groups coupled via ester bonds to the natural biodegradable polymer.

Optionally, when the natural biodegradable polymer includes a pendent hydrophobic moiety and/or corticosteroid, the inventive compositions can further include an enzyme, such as lipase, to accelerate degradation of the bond between the hydrophobic moiety and the polysaccharide (e.g., ester bond).

According to the invention, a natural biodegradable polysaccharide that includes a coupling group is used to form a medical article *in vivo.* Other polysaccharides can also be present in the biodegradable composition. For example, the two or more natural biodegradable polysaccharides are used to form a medical article. Examples include amylose and one or more other natural biodegradable polysaccharide(s), and maltodextrin and one or more other natural biodegradable polysaccharide(s); in one aspect the composition includes a mixture of amylose and maltodextrin, optionally with another natural biodegradable polysaccharide.

In one preferred embodiment, amylose or maltodextrin is the primary polysaccharide. In some embodiments, the composition includes a mixture of polysaccharides including amylose or maltodextrin and the amylose or maltodextrin content in the mixture of polysaccharides is 50% or greater, 60% or greater, 70% or greater, 80% or greater, or 85% or greater by weight.

Purified or enriched amylose preparations can be obtained commercially or can be prepared using standard biochemical techniques such as chromatography. In some aspects, high-amylose cornstarch can be used.

In accordance with the invention, the natural biodegradable polysaccharides comprises a coupling group. As used herein, "coupling group" can include (1) a chemical group that is able to form a reactive species that can react with the same or similar chemical group to form a bond that is able to couple the natural biodegradable polysaccharides together (for example, wherein the formation of a reactive species can be promoted by an initiator); or (2) a pair of two different chemical groups that are able to specifically react to form a bond that is able to couple the natural biodegradable polysaccharides together. The coupling group can be attached to any suitable natural biodegradable polysaccharide, including the amylose and maltodextrin polymers as exemplified herein. The natural biodegradable polysaccharide, once coupled, forms a natural biodegradable polysaccharide matrix.

Contemplated reactive pairs include Reactive Group A and corresponding Reactive Group B as shown in the Table 1 below. For the preparation of a biodegradable composition, a reactive group from Group A can be selected and coupled to a first set of natural biodegradable polysaccharides and a corresponding reactive Group B can be selected and coupled to a second set of natural biodegradable polysaccharides. Reactive Groups A and B can represent first and second coupling groups, respectively. At least one and preferably two, or more than two reactive groups are coupled to an individual natural biodegradable polysaccharide polymer. The first and second sets of natural biodegradable polysaccharides can be combined and reacted, for example, thermochemically, if necessary, to promote the coupling of natural biodegradable polysaccharides and the formation of a natural biodegradable polysaccharide matrix.

**Table 1**

| Reactive groups A | Reactive groups B |
|---|---|
| amine, hydroxyl, sulfhydryl | N-oxysuccinimide ("NOS") |
| amine | Aldehyde |
| amine | Isothiocyanate |
| amine, sulfhydryl | Bromoacetyl |
| amine, sulfhydryl | Chloroacetyl |
| amine, sulfhydryl | Iodoacetyl |
| amine, hydroxyl | Anhydride |
| aldehyde | Hydrazide |
| amine, hydroxyl, carboxylic acid | Isocyanate |
| amine, sulfhydryl | Maleimide |
| sulfhydryl | Vinylsulfone |

Amine also includes hydrazide (R-NH-NH₂)

For example, a suitable coupling pair would be a natural biodegradable polysaccharide having an electrophilic group and a natural biodegradable polysaccharide having a nucleophilic group. An example of a suitable electrophilic-nucleophilic pair is N-hydroxysuccinimide-amine pair, respectively. Another suitable pair would be an oxirane-amine pair.

In some aspects, the natural biodegradable polysaccharides of the invention include at least one, and more typically more than one, coupling group per natural biodegradable polysaccharide, allowing for a plurality of natural biodegradable polysaccharides to be coupled in linear and/or branched manner. In some preferred embodiments, the natural biodegradable polysaccharide includes two or more pendent coupling groups.

In some aspects, the coupling group on the natural biodegradable polysaccharide is a polymerizable group. In a free radical polymerization reaction the polymerizable group can couple natural biodegradable polysaccharides together in the composition, thereby forming a natural biodegradable polysaccharide matrix.

A preferred polymerizable group is an ethylenically unsaturated group. Suitable ethylenically unsaturated groups include vinyl groups, acrylate groups, methacrylate groups, ethacrylate groups, 2-phenyl acrylate groups, acrylamide groups, methacrylamide groups, itaconate groups, and styrene groups. Combinations of different ethylenically unsaturated groups can be present on a natural biodegradable polysaccharide, such as amylose or maltodextrin.

In preparing the natural biodegradable polysaccharide having pendent coupling groups any suitable synthesis procedure can be used. Suitable synthetic schemes typically involve reaction of, for example, hydroxyl groups on the natural biodegradable polysaccharide, such as amylose or maltodextrin. Synthetic procedures can be modified to produce a desired number of coupling groups pendent from the natural biodegradable polysaccharide backbone. For example, the hydroxyl groups can be reacted with a coupling group-containing compound or can be modified to be reactive with a coupling group-containing compound. The number and/or density of coupling groups (such as acrylate groups) can be controlled using the present method, for example, by controlling the relative concentration of reactive moiety to saccharide group content.

In some modes of practice, the biodegradable polysaccharides have an amount of pendent coupling groups of about 0.7 µmoles of coupling group per milligram of natural biodegradable polysaccharide. In a preferred aspect, the amount of coupling group per natural biodegradable polysaccharide is in the range of about 0.3 µmoles/mg to about 0.7 µmoles/mg. For example, amylose or maltodextrin can be reacted with an acrylate groups-containing compound to provide an amylose or maltodextrin macromer having a acrylate group load level in the range of about 0.3 µmoles/mg to about 0.7 µmoles/mg.

In accordance with the invention, the composition administered to a patient includes a natural biodegradable polysaccharide comprising a coupling group, and an initiator. As used herein, an "initiator" refers to a compound, or more than one compound, that is capable of promoting the formation of a reactive species from the coupling group. For example, the initiator can promote a free radical reaction of natural biodegradable polysaccharide having a coupling group. In some embodiments, the initiator can be an "initiator polymer" that includes a polymer having a backbone and one or more initiator groups pendent from the backbone of the polymer.

Generally speaking, the initiator can be provided as a photoreactive group (photoinitiator) that is activated by radiation, or a redox initiator that is activated when members of a redox pair contact each other. Each of these aspects will now be described.

In some aspects the initiator is a compound that is light sensitive and that can be activated to promote the coupling of the polysaccharide polymer via a free radical polymerization reaction. These types of initiators are referred to herein as "photoinitiators" In some aspects it is preferred to use photoinitiators that are activated by light wavelengths that have no or a minimal effect on a bioactive agent if present in the composition. A photoinitiator can be present in a biodegradable polysaccharide composition independent of the polysaccharide polymer or pendent from the polysaccharide polymer.

In ophthalmology, many diagnostic and therapeutic devices are equipped with a bright light source to illuminate the fundus of the eye. Thus, the compositions of the invention are particularly suitable in connection with ophthalmic procedures because they can be used along with equipment that is commonly available in ophthalmology offices where procedures utilizing light sources (e.g., PDT lasers) are performed. Such equipment includes light sources that can be used to initiate the photopolymerization of the inventive compositions. In this regard, the compositions of the invention are advantageously used because the activation systems such as metal halide, halogen and zenon ophthalmic light sources are typically in possession of the user. In some aspects, photoinitiators that have activation wavelengths in the visible light range or long wavelength UV (LWUV) range can be used in the compositions and methods of the invention.

In some embodiments, photoinitiation occurs using groups that promote an intra- or intermolecular hydrogen abstraction reaction. This initiation system can be used without additional energy transfer acceptor molecules and utilizing nonspecific hydrogen abstraction, but is more commonly used with an energy transfer acceptor, typically a tertiary amine, which results in the formation of both aminoalkyl radicals and ketyl radicals. Examples of molecules exhibiting hydrogen abstraction reactivity and useful in a polymeric initiating system, include analogs of benzophenone, thioxanthone, and camphorquinone.

In some preferred embodiments the photoinitiator includes one or more charged groups. The presence of charged groups can increase the solubility of the photoinitiator (which can contain photoreactive groups such as aryl ketones) in an aqueous system and therefore provide for an improved biodegradable composition. Suitable charged groups include, for example, salts of organic acids, such as sulfonate, phosphonate, carboxylate, and the like, and onium groups, such as quaternary ammonium, sulfonium, phosphonium, protonated amine, and the like. According to this embodiment, a suitable photoinitiator can include, for example, one or more aryl ketone photogroups selected from acetophenone, benzophenone, anthraquinone, anthrone, anthrone-like heterocycles, and derivatives thereof; and one or more charged groups, for example, as described herein. Examples of these types of water-soluble photoinitiators have been described in U.S. Patent No. 6,077,698.

In some aspects the photoinitiator is a compound that is activated by long-wavelength ultraviolet (LWUV) and visible light wavelengths. For example, in some aspects, the initiator includes a photoreducible or photo-oxidizable dye. Photoreducible dyes can also be used in conjunction with a compound such as a tertiary amine. The tertiary amine intercepts the induced triplet producing the radical anion of the dye and the radical cation of the tertiary amine. Examples of molecules exhibiting photosensitization reactivity and useful as an initiator include acridine orange, camphorquinone, ethyl eosin, eosin Y, erythrosine, fluorescein, methylene green, methylene blue, phloxime, riboflavin, rose bengal, thionine, and xanthine dyes. Use of these types of photoinitiators can be particularly advantageous when a light-sensitive bioactive agent is included in the biodegradable composition.

In some aspects, the photoinitiator is a water soluble photoinitiator. A "water soluble" photoinitiator has a solubility in the composition of about 0.5% or greater.

In some embodiments, a water-soluble derivative of camphorquinone is utilized. Camphor or camphorquinone can be derivatized by techniques known in the art to add, for example, charged groups. See, for example, G. Ullrich et al. (2003) Synthesis and photoactivity of new camphorquinone derivatives"; Austrian Polymer Meeting 21, International H. F. Mark-Symposium, 131.

In some aspects of the invention, the water soluble photoinitiator is a diketone, which can be selected from water-soluble derivatives of camphoroquinone, 9,10-phenanthrenequinone, and naphthoquinone having an absorbance of 400 nm and greater. In some aspects of the invention, for example, the photoinitiator is a water-soluble non-aromatic alpha diketones, selected from water-soluble derivatives of camphorquinone.

Other suitable long-wave ultra violet (LWUV) or light-activatable molecules include, but are not limited to, [(9-oxo-2-thioxanthanyl)-oxy]acetic acid, 2-hydroxythioxanthone, and vinyloxymethylbenzoin methyl ether. Suitable visible light activatable molecules include, but are not limited to water soluble forms of initiators comprising acridine orange, ethyl eosin, eosin Y, Eosin B, erythrosine, fluorescein, methylene green, methylene blue, phloxime, riboflavin, rose bengal, thionine, xanthine dyes, and the like.

As mentioned above, the initiator can comprise a photoinitiator or a redox initiator. Thus, in some aspects, the initiator includes an oxidizing agent/reducing agent pair, a "redox pair," to drive polymerization of the biodegradable polysaccharide. In this case, polymerization of the biodegradable polysaccharide is carried out upon combining one or more oxidizing agents with one or more reducing agents. In general, combinations of organic and inorganic oxidizers, and organic and inorganic reducing agents are used to generate radicals for polymerization. A description of redox initiation can be found in Principles of Polymerization, 2nd Edition, Odian G., John Wiley and Sons, pgs 201-204, (1981). Other compounds can be included in the composition to promote polymerization of the biodegradable polysaccharides.

When combined, the oxidizing agent and reducing agent can provide a particularly robust initiation system and can drive the formation of a polymerized matrix of polysaccharides from a composition having a low viscosity. A polysaccharide composition with a low viscosity may be due to a low concentration of polysaccharide in the composition, a polysaccharide having a low average molecular weight, or combinations thereof. Matrix formation from a polysaccharide composition having a low viscosity is particularly advantageous in many applications, especially for *in situ* polymerization. In some aspects of the invention, a low viscosity polysaccharide composition is passed through a small delivery conduit (e.g., having a small inner diameter), such as a needle, wherein the redox pair causes the polymerization of the polysaccharides *in situ.*

In some aspects of the invention, the viscosity of the composition is above about 5 cP, or about 10 cP or greater. In other aspects of the invention the viscosity of the composition is between about 5 cP or 10 cP and about 700 cP, or between about 5 cP or 10 cP and about 250 cP.

In order to promote polymerization of the biodegradable polysaccharides in a composition to form a matrix, the oxidizing agent is added to the reducing agent in the presence of the one or more biodegradable polysaccharides. For example, a composition including a biodegradable polysaccharide and a reducing agent is added to a composition including an oxidizing agent, or a composition including a biodegradable polysaccharide and an oxidizing agent is added to a composition containing a reducing agent. One desirable method of preparing a matrix is to combine a composition including a biodegradable polysaccharide and an oxidizing agent with a composition including a biodegradable polysaccharide and a reducing agent. For purposes of describing this method, the terms "first composition" and "second composition" can be used.

The viscosities of biodegradable polysaccharide in the first and second compositions can be the same or can be different. Generally, though, it has been observed that good mixing and subsequent matrix formation is obtained when the compositions have the same or similar viscosities. In this regard, if the same biodegradable polymer is used in the first and second compositions, the concentration of the biodegradable polymer may be the same or different.

The oxidizing agent can be selected from inorganic or organic oxidizing agents, including enzymes; the reducing agent can be selected from inorganic or organic reducing agents, including enzymes. Exemplary oxidizing agents include peroxides, including hydrogen peroxide, metal oxides, and oxidases, including glucose oxidase. Exemplary reducing agents include salts and derivatives of electropositive elemental metals such as Li, Na, Mg, Fe, Zn, Al, and reductases. In one mode of practice, the reducing agent is present at a concentration of about 2.5 mM or greater when the reducing agent is mixed with the oxidizing agent. Prior to mixing, the reducing agent can be present in a composition at a concentration of, for example, 5 mM or greater.

Other reagents can be present in the composition to promote polymerization of the biodegradable polysaccharide. Other polymerization promoting compounds can be included in the composition, such as metal or ammonium salts of persulfate.

In some aspects the polymerization initiator (photoinitiator or redox initiator) is a polymer that includes an initiator group (herein referred to as an "initiator polymer"). The polymeric portion of the initiator polymer can be obtained or prepared to have particular properties or features that are desirable for use with a biodegradable composition. For example, the polymeric portion of the initiator polymer can have hydrophilic or amphoteric properties, or it can include pendent charged groups. Optionally, or additionally, the polymer can change or improve the properties of the biodegradable matrix that is formed by the amylose polymer having coupling groups. For example, the initiator polymer can change the elasticity, flexibility, wettability, or softness (or combinations thereof) of the biodegradable matrix. Certain polymers, as described herein, are useful as plasticizing agents for compositions that include natural biodegradable polysaccharides. Initiator groups can be added to these plasticizing polymers and used in the compositions and methods of the invention.

For example, in some aspects an initiator can be pendent from a natural biodegradable polysaccharide. Therefore, the natural biodegradable polysaccharide is able to promote activation of polymerizable groups that are pendent from other natural biodegradable polysaccharides and promote the formation of a natural biodegradable polysaccharide matrix.

In other cases, the polymeric portion of the initiator polymer can include, for example, acrylamide and methacrylamide monomeric units, or derivatives thereof. In some embodiments, the coating composition includes an initiator polymer having a photoreactive group and a polymeric portion selected from the group of acrylamide and methacrylamide polymers and copolymers.

In still further embodiments, the initiator can be present as an independent component of the composition. The initiator can be present in the composition at a concentration sufficient for matrix formation. In some aspects, the initiator (for example, a water soluble non-aromatic alpha diketone such as a water soluble camphorquinone derivative) is used at a concentration of about 0.5 mg/ml or greater. In some aspects, the water soluble photoinitiator can be present at a concentration in the range of about 0.1 mg/ml to about 10 mg/ml.

Optionally, the compositions and methods of the invention can include polymerization accelerants that can improve the efficiency of polymerization. Examples of useful accelerants include N-vinyl compounds, particularly N-vinyl pyrrolidone and N-vinyl caprolactam. Such accelerants can be used, for instance, at a concentration of between about 0.01% and about 5%, and preferably between about 0.05% and about 0.5%, by weight, based on the volume of the coating composition.

According to the invention, the natural biodegradable polysaccharide that includes a coupling group is used to form a medical article. Other polysaccharides can also be present in the biodegradable composition. For example, the composition can include two different natural biodegradable polysaccharides, or more than two different natural biodegradable polysaccharides. For example, in some cases the natural biodegradable polysaccharide (such as amylose or maltodextrin) can be present in the composition along with another biodegradable polymer (i.e., a secondary polymer), or more than one other biodegradable polymer. An additional polymer or polymers can be used to alter the properties of the matrix, or serve as bulk polymers to alter the volume of the matrix formed from the biodegradable composition. For example, other biodegradable polysaccharides can be used in combination with the amylose polymer. These include hyaluronic acid, dextran, starch, amylose (for example, non-derivatized), amylopectin, cellulose, xanthan, pullulan, chitosan, pectin, inulin, alginates, and heparin.

In some aspects of the invention, a composition that includes at least the natural biodegradable polysaccharide, such as amylose or maltodextrin having a coupling group and a bioactive agent, is used to form a medical article *in vivo.* In some embodiments the composition includes the natural biodegradable polysaccharide, a bioactive agent, and an initiator. In other embodiments, a medical article is formed by combining the natural biodegradable polysaccharide and biodegradable microparticles.

The concentration of the natural biodegradable polysaccharide in the composition can be chosen to provide a medical article having a desired density of crosslinked natural biodegradable polysaccharide. In some embodiments, the concentration of natural biodegradable polysaccharide in the composition can depend on the type or nature of the bioactive agent that is included in the composition. In some embodiments the natural biodegradable polysaccharide having the coupling groups is present in the coating composition at a concentration in the range of about 5% to about 100% (w/v), and about 5% to about 50%, and in more specific embodiments in the range of about 10% to about 20% and in other embodiments in the range of about 20% to about 50% (w/v).

The concentration of the natural biodegradable polysaccharide may be higher to provide a more structurally rigid implant.

Other polymers or non-polymeric compounds can be included in the composition that can change or improve the properties of the medical article that is formed by the natural biodegradable composition having coupling groups in order to change the elasticity, flexibility, wettability, or adherent properties, (or combinations thereof) of the medical article.

In accordance with some aspects of the invention, the biodegradable composition includes a natural biodegradable polysaccharide comprising a coupling group, an initiator, and one or more bioactive agents. The bioactive agent can be dispersed within the natural biodegradable article itself. Alternatively, the bioactive agent can be present in microparticles that are associated with the natural biodegradable polysaccharide matrix. The bioactive agent can be delivered from the medical article upon degradation of the natural biodegradable polysaccharide and/or biodegradable microparticles.

The term "bioactive agent" refers to an agent that affects physiology of biological tissue. Bioactive agents include peptides, proteins, carbohydrates, nucleic acids, lipids, polysaccharides, synthetic inorganic or organic molecules, viral particles, cells, or combinations thereof, that cause a biological effect when administered *in vivo* to an animal, including but not limited to birds and mammals, including humans. Bioactive agents useful according to the invention include virtually any substance that possesses desirable therapeutic and/or prophylactic characteristics for application to the implantation site. Nonlimiting examples are antigens, enzymes, hormones, receptors, peptides, and gene therapy agents. Examples of suitable gene therapy agents include (a) therapeutic nucleic acids, including antisense DNA, antisense RNA, and interference RNA, and (b) nucleic acids encoding therapeutic gene products, including plasmid DNA and viral fragments, along with associated promoters and excipients. Examples of other molecules that can be incorporated include nucleosides, nucleotides, vitamins, minerals, and steroids.

For ease of discussion, reference will repeatedly be made to a "bioactive agent." While reference will be made to a "bioactive agent," it will be understood that the invention can provide any number of bioactive agents to a treatment site. Thus, reference to the singular form of "bioactive agent" is intended to encompass the plural form as well.

Although not limited to such, the biodegradable compositions of the invention are particularly useful for delivering bioactive agents that are large hydrophilic molecules, such as polypeptides (including proteins and peptides), nucleic acids (including DNA and RNA), polysaccharides (including heparin), as well as particles, such as viral particles, and cells. In one aspect, the bioactive agent has a molecular weight of about 10,000 or greater.

Classes of bioactive agents which can be incorporated into biodegradable medical articles (both the natural biodegradable matrix and/or the biodegradable microparticles) of this invention include, but are not limited to: ACE inhibitors, actin inhibitors, analgesics, anesthetics, anti-angiogenic agents (such as VEGF receptor antagonists, receptor tyrosine kinase inhibitors, VEGF antagonists, and IL-1beta inhibitors), anti-hypertensives, anti polymerases, antisecretory agents, anti-AIDS substances, antibiotics, anti-cancer substances, anti-cholinergics, anti-coagulants, anti-convulsants, anti-depressants, anti-emetics, antifungals, anti-glaucoma compounds, antihistamines, antihypertensive agents, anti-inflammatory agents (such as NSAIDs), anti metabolites, antimitotics, antioxidizing agents, anti-parasite and/or anti-Parkinson substances, antiproliferatives (including antiangiogenesis agents; also including 13-cis retinoic acid, retinoic acid derivatives, taxol, 5-fluorouracii, sirolimus (rapamycin), analogues of rapamycin, tacrolimus, ABT-578, everolimus, paclitaxel, taxane, genistein, and vinorelbine), anti-protozoal solutes, anti-psychotic substances, anti-pyretics, antiseptics, anti-spasmodics, antiviral agents, calcium channel blockers, cell response modifiers, chelators, chemotherapeutic agents, complement inhibitors, dopamine agonists, extracellular matrix components, fibrinolytic agents, free radical scavengers, growth hormone antagonists, hypnotics, immunosuppressive agents, immunotoxins, inhibitors of surface glycoprotein receptors, microtubule inhibitors, miotics, muscle contractants, muscle relaxants, neurotoxins, neurotransmitters, neuroprotective agents, opioids, photodynamic therapy agents, prostaglandins, remodeling inhibitors, statins, steroids, thrombolytic agents, tranquilizers, vasodilators, and vasospasm inhibitors.

Antibiotics are art recognized and are substances that inhibit the growth of or kill microorganisms. Examples of antibiotics include penicillin, tetracycline, chloramphenicol, minocycline, doxycycline, vancomycin, bacitracin, kanamycin, neomycin, gentamycin, erythromycin, cyclosporine, cephalosporins, geldanamycin, and analogs thereof. Examples of cephalosporins include cephalothin, cephapirin, cefazolin, cephalexin, cephradine, cefadroxil, cefamandole, cefoxitin, cefaclor, cefuroxime, cefonicid, ceforanide, cefotaxime, moxalactam, ceftizoxime, ceftriaxone, and cefoperazone.

Antiseptics are recognized as substances that prevent or arrest the growth or action of microorganisms, generally in a nonspecific fashion, e.g., by inhibiting their activity or destroying them. Examples of antiseptics include silver sulfadiazine, chlorhexidine, glutaraldehyde, peracetic acid, sodium hypochlorite, phenols, phenolic compounds, iodophor compounds, quaternary ammonium compounds, and chlorine compounds.

Anti-viral agents are substances capable of destroying or suppressing the replication of viruses. Examples of anti-viral agents include α-methyl-P-adamantane methylamine, hydroxy-ethoxymethylguanine, adamantanamine, 5-iodo-2'-deoxyuridine, trifluorothymidine, interferon, and adenine arabinoside.

Enzyme inhibitors are substances that inhibit an enzymatic reaction. Examples of enzyme inhibitors include edrophonium chloride, N-methylphysostigmine, neostigmine bromide, physostigmine sulfate, tacrine HCl, tacrine, 1-hydroxymaleate, iodotubercidin, p-bromotetramisole, 10-(α-diethylaminopropionyl)-phenothiazine hydrochloride, calmidazolium chloride, hemicholinium-3, 3,5-dinitrocatechol, diacylglycerol kinase inhibitor I, diacylglycerol kinase inhibitor II, 3-phenylpropargylamine, N-monomethyl-L-arginine acetate, carbidopa, 3-hydroxybenzylhydrazine HCl, hydralazine HCl, clorgyline HCl, deprenyl HCl, L(-), deprenyl HCl, D(+), hydroxylamine HCl, iproniazid phosphate, 6-MeO-tetrahydro-9H-pyrido-indole, nialamide, pargyline HCl, quinacrine HCl, semicarbazide HCl, tranylcypromine HCl, N,N-diethylaminoethyl-2,2-diphenylvalerate hydrochloride, 3-isobutyl-1-methylxanthine, papaverine HCl, indomethacin, 2-cyclooctyl-2-hydroxyethylamine hydrochloride, 2,3-dichloro-α-methylbenzylamine (DCMB), 8,9-dichloro-2,3,4,5-tetrahydro-1H-2-benzazepine hydrochloride, p-aminoglutethimide, p-aminoglutethimide tartrate, R(+), p-aminoglutethimide tartrate, S(-), 3-iodotyrosine, alpha-methyltyrosine, L(-) alpha-methyltyrosine, D L(-), cetazolamide, dichlorphenamide, 6-hydroxy-2-benzothiazolesulfonamide, and allopurinol.

Anti-pyretics are substances capable of relieving or reducing fever. Anti-inflammatory agents are substances capable of counteracting or suppressing inflammation. Examples of such agents include aspirin (salicylic acid), indomethacin, sodium indomethacin trihydrate, salicylamide, naproxen, colchicine, fenoprofen, sulindac, diflunisal, diclofenac, indoprofen and sodium salicylamide. Local anesthetics are substances that have an anesthetic effect in a localized region. Examples of such anesthetics include procaine, lidocaine, tetracaine and dibucaine.

Cell response modifiers are chemotactic factors such as platelet-derived growth factor (pDGF). Other chemotactic factors include neutrophil-activating protein, monocyte chemoattractant protein, macrophage-inflammatory protein, SIS (small inducible secreted) proteins, platelet factor, platelet basic protein, melanoma growth stimulating activity, epidermal growth factor, transforming growth factor (alpha), fibroblast growth factor, platelet-derived endothelial cell growth factor, insulin-like growth factor, nerve growth factor, and bone growth/cartilage-inducing factor (alpha and beta). Other cell response modifiers are the interleukins, interleukin inhibitors or interleukin receptors, including interleukin 1 through interleukin 10; interferons, including alpha, beta and gamma; hematopoietic factors, including erythropoietin, granulocyte colony stimulating factor, macrophage colony stimulating factor and granulocyte-macrophage colony stimulating factor; tumor necrosis factors, including alpha and beta; transforming growth factors (beta), including beta-1, beta-2, beta-3, inhibin, activin, and DNA that encodes for the production of any of these proteins.

Examples of statins include lovastatin, pravastatin, simvastatin, fluvastatin, atorvastatin, cerivastatin, rousvastatin, and superstatin.

Imaging agents are agents capable of imaging a desired site, e.g., tumor, *in vivo,* can also be included in the biodegradable compositions. Examples of imaging agents include substances having a label that is detectable *in vivo,* e.g., antibodies attached to fluorescent labels. The term antibody includes whole antibodies or fragments thereof.

Exemplary ligands or receptors include antibodies, antigens, avidin, streptavidin, biotin, heparin, type IV collagen, protein A, and protein G.

Exemplary antibiotics include antibiotic peptides.

The bioactive agent can be also be selected from mono-2-(carboxymethyl) hexadecanamidopoly (ethylene glycol)₂₀₀ mono-4-benzoylbenzyl ether, mono-3-carboxyheptadecanamidopoly (ethylene glycol)₂₀₀ mono-4-benzoylbenzyl ether, mono-2-(carboxymethyl) hexadecanamidotetra (ethylene glycol) mono-4-benzoylbenzyl ether, mono-3-carboxyheptadecanamidotetra (ethylene glycol) mono-4-benzoylbenzyl ether, N-[2-(4-benzoylbenzyloxy) ethyl]-2-(carboxymethyl) hexadecanamide, N-[2-(4-benzoylbenzyloay)ethyl]-3-carboxyheptadecanamide, N-[12-(benzoylbenzyloxy) dodecyl]-2-(carboxymethyl) hexadecanamide, N-[12-(benzoylbenzyloxy) dodecyl]-3-carboxy-heptadecanamide, N-[3-(4-benzoylbenzamido) propyl]-2-(carboxymethyl) hexadecanamide, N-[3-(4-benzoylbenzamido) propyl]-3-carboxyheptadecanamide, N-(3-benzoylphenyl)-2-(carboxymethyl) hexadecanamide, N-(3-benzoylphenyl)-3-carboxyheptadecanamide, N-(4-benzoylphenyl)-2-(carboxymethyl) hexadecanamide, poly(ethylene glycol)₂₀₀ mono-15-carboxypentadecyl mono-4-benzoylbenzyl ether, and mono-15-carboxypentadecanamidopoly (ethylene glycol)₂₀₀ mono-4-benzoylbenzyl ether.

Additional examples of contemplated bioactive agents include analogues of rapamycin ("rapalogs"), ABT-578 from Abbott, dexamethasone, betamethasone, vinblastine, vincristine, vinorelbine, poside, teniposide, daunorubicin, doxorubicin, idarubicin, anthracyclines, mitoxantrone, bleomycins, plicamycin (mithramycin), mitomycin, mechlorethamine, cyclophosphamide and its analogs, melphalan, chlorambucil, ethylenimines and methyhnelainines, alkyl sulfonates-busulfan, nitrosoureas, carmustine (BCNU) and analogs, streptozocin, trazenes-dacarbazinine, methotrexate, fluorouracil, floxuridine, cytarabine, mercaptopurine, thioguanine, pentostatin, 2-chlorodeoxyadenosine, cisplatin, carboplatin, procarbazine, hydroxyurea, mitotane, estrogen, ticlopidine, clopidogrel, abciximab, breveldin, cortisol, cortisone, fludrocortisone, prednisone, prednisolone, 6U-methylprednisolone, triamcinolone, acetaminophen, etodalac, tolmetin, ketorolac, ibuprofen and derivatives, mefenamic acid, meclofenamic acid, piroxicam, tenoxicam, phenylbutazone, oxyphenthatrazone, nabumetone, auranofin, aurothioglucose, gold sodium thiomalate, azathioprine, mycophenolate mofetil; angiotensin receptor blockers; nitric oxide donors; and mTOR inhibitors.

Viral particles and viruses include those that may be therapeutically useful, such as those used for gene therapy, and also attenuated viral particles and viruses that can promote an immune response and generation of immunity. Useful viral particles include both natural and synthetic types. Viral particles include, but are not limited to, adenoviruses, baculoviruses, parvoviruses, herpesviruses, poxviruses, adeno-associated viruses, vaccinia viruses, and retroviruses.

Other bioactive agents that can be used for altering gene function include plasmids, phages, cosmids, episomes, and integratable DNA fragments, antisense oligonucleotides, antisense DNA and RNA, modified DNA and RNA, iRNA, ribozymes, siRNA, and shRNA.

Other bioactive agents include cells such as platelets, stem cells, T lymphocytes, B lymphocytes, acidophils, adipocytes, astrocytes, basophils, hepatocytes, neurons, cardiac muscle cells, chondrocytes, epithelial cells, dendrites, endrocrine cells, endothelial cells, eosinophils, erythrocytes, fibroblasts, follicular cells, ganglion cells, hepatocytes, endothelial cells, Leydig cells, parenchymal cells, lymphocytes, lysozyme-secreting cells, macrophages, mast cells, megakaryocytes, melanocytes, monocytes, myoid cells, neck nerve cells, neutrophils, oligodendrocytes, oocytes, osteoblasts, osteochondroclasts, osteoclasts, osteocytes, plasma cells, spermatocytes, reticulocytes, Schwann cells, Sertoli cells, skeletal muscle cells, and smooth muscle cells. Bioactive agents can also include genetically modified, recombinant, hybrid, mutated cells, and cells with other alterations.

Additives such as inorganic salts, BSA (bovine serum albumin), and inert organic compounds can be used to alter the profile of bioactive agent release, as known to those skilled in the art.

The concentration of the bioactive agent or agents dissolved or suspended in the biodegradable composition can range from about 0.01 to about 90 percent, by weight, based on the weight of the final biodegradable composition.

The particular bioactive agent, or combination of bioactive agents, can be selected depending upon one or more of the following factors: the application of the controlled delivery device, the medical condition to be treated, the anticipated duration of treatment, characteristics of the implantation site, the number and type of bioactive agents to be utilized, and the like.

Any of the polymer compositions described herein can be utilized to form a medical article *in situ* and can include any number of desired bioactive agents, depending upon the final application of the medical device.

A comprehensive listing of bioactive agents can be found in The Merck Index Thirteenth Edition, Merck & Co. (2001). Bioactive agents are commercially available from Sigma Aldrich Fine Chemicals, Milwaukee, WI.

In some aspects of the invention, a microparticle is used to deliver the bioactive agent from the natural biodegradable polysaccharide-based medical article. The microparticles of the invention can comprise any three-dimensional structure that can be associated with the matrix formed by the polysaccharide polymer. The term "microparticle" is intended to reflect that the three-dimensional structure is very small but not limited to a particular size range, or not limited to a structure that has a particular shape. According to the invention, microparticles typically have a size in the range of 5 nm to 100 µm in diameter. Generally microparticles are spherical or somewhat spherical in shape, but can have other shapes as well. In preferred embodiments of the invention, the biodegradable microparticles have a size in the range of 100 nm to 20 µm in diameter, and even more preferable in the range of 400 nm to 20 µm in diameter.

The microparticle being "biodegradable" refers to the presence of one or more biodegradable materials in the microparticle. The biodegradable microparticles include at least a biodegradable material (such as a biodegradable polymer) and a bioactive agent. The biodegradable microparticles can gradually decompose and release bioactive agent upon exposure to an aqueous environment, such as body fluids.

The biodegradable microparticle can also include one or more biodegradable polymers. Examples of biodegradable polymers that can be included in the biodegradable microparticle include, for example, polylactic acid, poly(lactide-co-glycolide), polycaprolactone, polyphosphazine, polymethyldienemalonate, polyorthoesters, polyhydroxybutyrate, polyalkeneanhydrides, polypeptides, polyanhydrides, and polyesters, and the like.

Biodegradable polyetherester copolymers can be used. Generally speaking, the polyetherester copolymers are amphiphilic block copolymers that include hydrophilic (for example, a polyalkylene glycol, such as polyethylene glycol) and hydrophobic blocks (for example, polyethylene terephthalate). Examples of block copolymers include poly(ethylene glycol)-based and poly(butylene terephthalate)-based blocks (PEG/PBT polymer). Examples of these types of multiblock copolymers are described in, for example, U.S. Patent No. 5,980,948. PEG/PBT polymers are commercially available from Octoplus BV, under the trade designation PolyActive™.

Biodegradable copolymers having a biodegradable, segmented molecular architecture that includes at least two different ester linkages can also be used. The biodegradable polymers can be block copolymers (of the AB or ABA type) or segmented (also known as multiblock or random-block) copolymers of the (AB)ₙ type. These copolymers are formed in a two (or more) stage ring opening copolymerization using two (or more) cyclic ester monomers that form linkages in the copolymer with greatly different susceptibilities to transesterification. Examples of these polymers are described in, for example, in U.S. Patent No. 5,252,701 (Jarrett et al., "Segmented Absorbable Copolymer").

Other suitable biodegradable polymer materials include biodegradable terephthalate copolymers that include a phosphorus-containing linkage. Polymers having phosphoester linkages, called poly(phosphates), poly(phosphonates) and poly(phosphites), are known. See, for example, Penczek et al., Handbook of Polymer Synthesis, Chapter 17: "Phosphorus-Containing Polymers," 1077-1132 (Hans R. Kricheldorf ed., 1992), as well as U.S. Patent Nos. 6,153,212, 6,485,737, 6,322,797, 6,600,010, 6,419,709. Biodegradable terephthalate polyesters can also be used that include a phosphoester linkage that is a phosphite. Suitable terephthalate polyester-polyphosphite copolymers are described, for example, in U.S. patent No. 6,419,709 (Mao et al., "Biodegradable Terephthalate Polyester-Poly(Phosphite) Compositions, Articles, and Methods of Using the Same). Biodegradable terephthalate polyester can also be used that include a phosphoester linkage that is a phosphonate. Suitable terephthalate polyester-poly(phosphonate) copolymers are described, for example, in U.S. Patent Nos. 6,485,737 and 6,153,212 (Mao et al., "Biodegradable Terephthalate Polyester-Poly(Phosphonate) Compositions, Articles and Methods of Using the Same). Biodegradable terephthalate polyesters can be used that include a phosphoester linkage that is a phosphate. Suitable terephthalate polyester-poly(phosphate) copolymers are described, for example, in U.S. Patent Nos. 6,322,797 and 6,600,010 (Mao et al., "Biodegradable Terephthalate Polyester-Poly(Phosphate) Polymers, Compositions, Articles, and Methods for Making and Using the Same).

Biodegradable polyhydric alcohol esters can also be used (See U.S. Patent No. 6,592,895). This patent describes biodegradable star-shaped polymers that are made by esterifying polyhydric alcohols to provide acyl moieties originating from aliphatic homopolymer or copolymer polyesters. The biodegradable polymer can be a three-dimensional crosslinked polymer network containing hydrophobic and hydrophilic components which forms a hydrogel with a crosslinked polymer structure, such as that described in U.S. Patent No. 6,583,219. The hydrophobic component is a hydrophobic macromer with unsaturated group terminated ends, and the hydrophilic polymer is a polysaccharide containing hydroxy groups that are reacted with unsaturated group introducing compounds. The components are convertible into a one-phase crosslinked polymer network structure by free radical polymerization. In yet further embodiments, the biodegradable polymer can comprise a polymer based upon α-amino acids (such as elastomeric copolyester amides or copolyester urethanes, as described in U.S. Patent No. 6,503,538).

The biodegradable microparticle can include one or more biodegradable polymers obtained from natural sources. In some preferred aspects the biodegradable polymer is selected from hyaluronic acid, dextran, starch, amylose, amylopectin, cellulose, xanthan, pullulan, chitosan, pectin, inulin, alginates, and heparin. One, or combinations of more than one of these biodegradable polymers, can be used. A particular biodegradable polymer can also be selected based on the type of bioactive agent that is present in the microparticle. Therefore, in some aspects of the invention, the biodegradable matrix can include a natural biodegradable polysaccharide matrix and a natural biodegradable polysaccharide-containing microparticle.

Therefore, in some embodiments, the microparticles include a natural biodegradable polysaccharide such as amylose or maltodextrin. In some embodiments the natural biodegradable polysaccharide can be the primary biodegradable component in the microparticle. In some embodiments, both the biodegradable matrix and the microparticle include amylose and/or maltodextrin as components.

Dextran-based microparticles can be particularly useful for the incorporation of bioactive agents such as proteins, peptides, and nucleic acids. Examples of the preparation of dextran-based microparticles are described in U.S. Patent No. 6,303,148.

The preparation of amylose and other starch-based microparticles have been described in various references, including, for example, U.S. Patent No. 4,713,249; U.S. Patent No. 6,692,770; and U.S. Patent No. 6,703,048. Biodegradable polymers and their synthesis have been also been described in various references including Mayer, J.M., and Kaplan, D.L. (1994) Trends in Polymer Science 2:pages 227-235; and Jagur-Grodzinski, J., (1999) Reactive and Functional Polymers: Biomedical Application of Functional Polymers, Vol. 39, pages 99-138.

In some aspects of the invention, the biodegradable microparticle contains a biologically active agent (a "bioactive agent"), such as a pharmaceutical or a prodrug. Microparticles can be prepared incorporating various bioactive agents by established techniques, for example, by solvent evaporation (see, for example, Wichert, B. and Rohdewald, P. J Microencapsul. (1993) 10:195). The bioactive agent can be released from the biodegradable microparticle (the microparticle being present in the natural biodegradable polysaccharide composition) upon degradation of the biodegradable microparticle *in vivo*. Microparticles having bioactive agent can be formulated to release a desired amount of the agent over a predetermined period of time. It is understood that factors affecting the release of the bioactive agent and the amount released can be altered by the size of the microparticle, the amount of bioactive agent incorporated into the microparticle, the type of degradable material used in fabricating the microparticle, the amount of biodegradable microparticles immobilized per unit area within the biodegradable medical article, and the like.

The microparticles can also be treated with a porogen, such as salt, sucrose, PEG, or an alcohol, to create pores of a desired size for incorporation of the bioactive agent.

The quantity of bioactive agents provided in the biodegradable microparticle can be adjusted by the user to achieve the desired effect. Biologically active compounds can be provided by the microparticles in a range suitable for the application. In another example, protein molecules can be provided by biodegradable microparticles. For example, the amount of protein molecules present can be in the range of 1-250,000 molecules per 1 µm diameter microparticle.

Generally, the concentration of the bioactive agent present in the biodegradable microparticles can be chosen based on any one or a combination of a number of factors, including, but not limited to, the release rate from the medical article, the type of bioactive agent(s) in the biodegradable matrix, the desired local or systemic concentration of the bioactive agent following release, and the half life of the bioactive agent. In some cases the concentration of bioactive agent in the microparticle can be about 0.001% or greater, or in the range of about 0.001% to about 50 percent, or greater, by weight, based on the weight of the microparticle.

The particular bioactive agent to be included in the biodegradable microparticle, or combination of bioactive agents in microparticles, can be selected depending upon factors such as the application of the medical article, the medical condition to be treated, the anticipated duration of treatment, characteristics of the implantation site, the number and type of bioactive agents to be utilized, the chemical composition of the microparticle, size of the microparticle, crosslinking, and the like.

In one embodiment, the invention advantageously allows for preparation of medical articles having two, or more than two, different bioactive agents, wherein the bioactive agents are mutually incompatible in a particular environment, for example, as hydrophobic and hydrophilic drugs are incompatible in either a polar or non-polar solvent. Different bioactive agents may also demonstrate incompatibility based on protic/aprotic solvents or ionic/non-ionic solvents. For example, the invention allows for the preparation of one set of biodegradable microparticles containing a hydrophobic drug and the preparation of another set of biodegradable microparticles containing a hydrophilic drug; the mixing of the two different sets of microparticles into a polymeric material used to form the matrix; and the disposing of the mixture on the surface of a substrate. Both hydrophobic and hydrophilic drugs can be released from the medical article at the same time as the biodegradable microparticles degrade, or the composition of the biodegradable microparticles or the natural biodegradable polysaccharide matrix can be altered so that one bioactive agent is released at a different rate or time than the other one.

Biodegradable microparticles can be prepared having compositions that are suitable for either hydrophobic or hydrophilic drugs. For example, polymers such as polylactide or polycaprolactone can be useful for preparing biodegradable microparticles that include hydrophobic drugs; whereas polymers such as amylose or glycolide can be useful for preparing microparticles that include hydrophilic drugs.

Various factors can influence the delivery of bioactive agents from the biodegradable medical articles of the invention. These include the concentration of the natural biodegradable polysaccharide and the extent of natural biodegradable polysaccharide coupling in the biodegradable matrix, the amount and location of biodegradable microparticles associated with the medical article, the concentration of bioactive agent in the microparticles, and the like. For example, the rate of delivery of the drug can be decreased by increasing the concentration of polymeric material or the relative amount of coupling or crosslinking of the polymeric material in the polymeric matrix or in the microparticle. Based on the description provided herein and the general knowledge in this technical area, one can alter properties of the coating to provide a desired release rate for one or more particular bioactive agents from the inventive biodegradable matrix.

Portions of the degradable medical implant can be prepared to degrade at the same or different rates. For example, the biodegradable microparticles can be prepared or obtained to have a faster rate of degradation than the natural biodegradable polysaccharide matrix. In this case, the bioactive agent can be released into the natural biodegradable polysaccharide matrix and/or diffuse out of the natural biodegradable polysaccharide matrix.

In preferred aspects of the following methods, the natural biodegradable polysaccharide can be selected from the group of amylose and maltodextrin. In other preferred aspects of the following methods, the natural biodegradable polysaccharide has a molecular weight of 500,000 Da or less, 250,000 Da or less, 100,000 Da or less, or 50,000 Da or less. It is also preferred that the natural biodegradable polysaccharides have an average molecular weight of 500 Da or greater. A particularly preferred size range for the natural biodegradable polysaccharides is in the range of about 1000 Da to about 10,000 Da.

In accordance with the invention, a biodegradable implant is formed in *situ,* in an eye of a patient, by (a) administering a composition to a patient, the composition comprising a natural biodegradable polysaccharide comprising a coupling group, an initiator, and a bioactive agent; and (b) activating the initiator to couple the natural biodegradable polysaccharides present in the composition, thereby forming a solid implant within the eye of the patient.

In other aspects, the invention provides methods for forming a biodegradable implant *in situ,* in an eye of a patient, the methods including steps of: (a) providing a first composition comprising: (i) a natural biodegradable polysaccharide comprising a pendent polymerizable group, and (ii) a first member of a redox pair; (b) providing a second composition comprising: (i) a natural biodegradable polysaccharide comprising a pendent polymerizable group, and (ii) a second member of a redox pair; (c) administering the first composition, the second composition, or a mixture of the first and second composition in liquid form into the eye of a patient; and (d) contacting the first composition with the second composition comprising a second member of the redox pair where, in the step of contacting, the redox pair initiates polymerization of the natural biodegradable polysaccharides, thereby forming a solid implant within the eye.

In accordance with the invention, one or more compositions are thus administered to the patient, depending upon the mode of polymerization initiation involved. When photoinitiation is involved, typically one composition is administered. When polymerization is initiated by contacting members of a reactive pair (such as a redox pair), two or more compositions may be administered to the patient. For purposes of describing the inventive methods below, reference will be made to administering a "composition" Use of the singular form of this word is not meant to limit the discussion to administration of a single composition; rather, it is understood that the term can include the appropriate number of compositions required for performance of the particular method employed.

In accordance with the invention, the biodegradable composition can be administered to the patient by any suitable method to introduce the composition to a targeted site within a patient. Typically, the composition is administered by injection to the targeted site, when the targeted site is located within the body of a patient. The composition can be administered by use of a suitable cannula or syringe, depending upon the particular site chosen and viscosity of the composition, for example. Suitable administration routes will be apparent upon review of this disclosure. In some aspects, the target site is the same as the implantation site for the formed medical article. The term "implantation site" refers to the site within a patient's body at which the implantable article is located during a treatment course according to the invention.

The inventive methods and compositions are particularly useful for forming medical articles *in situ* in limited access regions of the body, as discussed herein. Taking the eye as an example, the compositions are utilized to form ophthalmic articles at implantation sites within the eye tissues. Suitable ophthalmic articles in accordance with these aspects can perform a function and provide bioactive agent to any desired area of the eye. In some aspects, the articles can be utilized to deliver bioactive agent to an anterior segment of the eye (in front of the lens), and/or a posterior segment of the eye (behind the lens). In some desirable aspects, the composition comprises a flowable liquid with a sufficient viscosity to allow administration to the implantation site within the eye.

The compositions can be utilized to form ophthalmic articles located external to the globe, such as ophthalmic articles placed juxta-sclerally (under the conjunctival membrane).

Articles configured for placement at an internal site of the eye can reside within any desired area of the eye. In some aspects, the ophthalmic article can be configured for placement at an intraocular site, such as the vitreous or subretinal space.

As mentioned, the vitreous chamber is the largest chamber of the eye and contains the vitreous humor or vitreous. Generally speaking, the vitreous is bound interiorly by the lens, posterior lens zonules and ciliary body, and posteriorly by the retinal cup. The vitreous is a transparent, viscoelastic gel that is 98% water and has a viscosity of about 2-4 times that of water. The main constituents of the vitreous are hyaluronic acid (HA) molecules and type II collagen fibers, which entrap the HA molecules. The viscosity is typically dependent on the concentration of HA within the vitreous. The vitreous is traditionally regarded as consisting of two portions: a cortical zone, characterized by more densely arranged collagen fibrils, and a more liquid central vitreous.

Therefore, in some aspects, the invention provides method for forming medical articles at a site comprising a gel-like material, such as viscoelastic gel. Desirably, the viscosity of the biodegradable polysaccharide composition is selected such that the composition is sufficiently flowable to be administered through a delivery conduit (e.g., cannula or syringe), yet remains localized at the site of administration and prior to polymerization, such that the composition can be polymerized to form a solid implant. The viscosity of the biodegradable polysaccharide composition can be selected depending upon such factors as geometry and composition of the implantation site selected (e.g., vitreous humor, subretinal space, or other limited access region).

In many aspects of the invention, ocular administration is performed by injecting the biodegradable polysaccharide composition into the vitreous. In some aspects, the composition can be injected through the scleral tissue (trans-scleral injection). Typically, intravitreal delivery will be accomplished by direct intravitreal injection of the biodegradable polysaccharide composition, for example, using a 25 to 30-gauge needle (or smaller) having a length of about 0.5 inches to about 0.62 inches.

This methodology also yields a technique that can be implemented in an outpatient clinic setting. According to this embodiment, a delivery instrument or device is provided (e.g., a cannula or syringe), a portion of which is configured and arranged such that when the instrument is inserted into the eye, the opening formed in the sclera to receive the instrument is small enough so as to not require sutures to seal or close the opening in the sclera. In other words, the opening is small enough that the wound or opening is self-sealing, thereby preventing the vitreous humor from leaking out of the eye.

In addition, the step of inserting can further include inserting the insertable portion of the delivery instrument or device transconjunctivally so the operable end thereof is within the vitreous. In this regard, transconjunctival shall be understood to mean that the instrument's operable end is inserted through both the conjunctiva and through the sclera into the vitreous. More particularly, inserting the insertable portion that forms an opening in the sclera and the conjunctiva that is small enough so as to not require sutures or the like to seal or close the opening in the sclera. In conventional surgical techniques for the posterior segment of the eye, the conjunctiva is routinely dissected to expose the sclera, whereas according to the methodology of this embodiment, the conjunctiva need not be dissected or pulled back.

Consequently, when the instrument is removed from the eye, the surgeon does not have to seal or close the opening in the sclera with sutures to prevent leaking of the aqueous humor, since such an opening or wound in the sclera is self sealing. In addition, with the transconjunctival approach, the surgeon does not have to reattach the dissected conjunctiva. These features can further simplify the surgical procedure, as well as reduce (if not eliminate) suturing required under the surgical procedure.

It will be understood that the inventive methods do not require dissection of the conjunctiva. However, if such additional step is desired in a particular treatment, such conjunctival dissection could be performed.

After the insertable portion of the instrument is inserted into the eye, the operable end thereof is localized to the targeted site within the body. The "targeted site" is the site within the patient's body at which the biodegradable polysaccharide composition is to be delivered. As mentioned herein, the targeted site can be the same or different from the implantation site. As is known to those skilled in the art, surgical personnel typically mount a lens assembly onto the cornea of the eye in accordance with known and accepted practices and techniques. This lens assembly is provided so that the surgeon can view the interior of the eye as well as any instruments inserted therein. In addition, a light-transmitting apparatus as is known in the art can also be inserted into the vitreous so as to be capable of providing a source of light therein for the surgeon. Accordingly, the surgeon would determine the positioning of the operable end of the instrument by viewing the interior of the eye using the lens assembly and being illuminated by the light transmitting apparatus.

This procedure can be performed without vitrectomy and results in a self-sealing sclerotomy, eliminating the need for sutures and minimizing risk of infection. In some aspects, the small sclerotomy is leakage-free, thereby reducing risk of leakage of vitreous from the implantation site. Advantageously, the inventive methods can be performed as an office-based procedure.

Once the delivery instrument is located at a suitable position within the vitreous, the biodegradable polysaccharide composition is administered, for example, by injection of the composition into the vitreous. A suitable amount of the biodegradable polysaccharide composition is administered to provide the volume desired for the particular treatment. In some aspects, the biodegradable polysaccharide composition is administered to the vitreous in an amount of 200 µl or less.

In some aspects, ocular administration is performed by injecting the biodegradable polysaccharide composition into the subretinal space, to form an implant at a subretinal area within the eye. In these aspects, the instrument utilized for administration (e.g., needle or cannula) can be advanced transconjunctivally and trans-retinally, to reach the subretinal space within the eye. Once the tip of the instrument has reached the subretinal space, a limited or localized retinal detachment (e.g., a bleb detachment) can be formed using any of a number of devices and/or techniques known to those skilled in the art, thereby defining or forming a subretinal space. The biodegradable polysaccharide composition can then be administered to the subretinal space formed by the retinal detachment. The limited or local dome-shaped subretinal detachment is created in such a fashion that the detachment itself generally does not have an appreciable or noticeable long-term effect on the vision of the patient.

In some embodiments, the step of administering the biodegradable polysaccharide composition includes inserting a portion of a delivery instrument or device, such as the exemplary delivery device illustrated in U.S. Patent Application No. 2004/0133155 (Varner et al.), into the eye in a minimally invasive manner. This methodology also yields a technique that can be implemented in an outpatient clinic setting. According to this embodiment, a delivery instrument or device is provided, a portion of which is configured and arranged such that when the instrument is inserted into the eye, the opening formed in the sclera to receive the instrument is small enough so as to not require sutures to seal or close the opening in the sclera. In other words, the opening is small enough that the wound or opening is self-sealing, thereby preventing the vitreous humor from leaking out of the eye.

As discussed above for vitreal administration, the step of inserting can include inserting the insertable portion of the delivery instrument or device transconjunctivally so the operable end thereof is within the vitreous. The delivery instrument can be advanced through the vitreous to the retina. After the insertable portion of the instrument is inserted into the eye, the operable end thereof is localized to the targeted site including the tissues that are being targeted for treatment. As discussed above, surgical personnel typically utilize visualization techniques to view the instruments within the eye.

After localizing the operable end of the instrument to the targeted site, for example the surface of the retina proximal the implantation site, the surgeon forms the limited retinal detachment. In an illustrative exemplary embodiment, the surgeon forms the limited retinal detachment by injecting a fluid, such as liquid or gas, from the instrument's operable end. More specifically, the fluid is injected from the instrument's operable end in such a manner that the injected fluid is disposed between the retina and the choroid, thereby causing the retina to detach therefrom. In more specific embodiments, the instrument's operable end is positioned such that the stream of fluid flowing from the operable end of the instrument is directed towards the targeted site of the retina and the stream of fluid pierces the retina and flows beneath the retina. Using known techniques, an operator of the delivery instrument is able to determine that the distal portion of the instrument has entered, but not traveled completely through, the retina.

In accordance with the invention, the biodegradable polysaccharide composition is administered in the subretinal spaced defined by the limited retinal detachment. In some embodiments, the instrument forming the retinal detachment can be used to administer the biodegradable polysaccharide composition into the retinal detachment. Alternatively, a fluid including the biodegradable polysaccharide composition can be used to form the retinal detachment and thereby simultaneously form the detachment and inject the composition containing biodegradable polysaccharide (and optionally, bioactive agent). Thus, the forming of the detachment and the injection of the composition are performed essentially simultaneously, thereby further simplifying the procedure or process.

In some aspects, subretinal delivery of the composition will be accomplished by direct subretinal injection of the composition, for example, using a 30-gauge needle (or smaller). In some aspects, the needle can be about 42 gauge or less, particularly when a self-sealing retinotomy is desired.

When the inventive methods are utilized to form medical articles at other areas within the eye, similar techniques can be utilized to administer the composition to the desired implantation site. Techniques are known for administration of compositions to other areas within or adjacent to the eye, such as the capsular bag (for medical articles for implantation in the anterior region of the eye), juxtascleral locations, and the like.

The biodegradable polysaccharide composition is thus administered to a targeted site within the patient, where the composition is allowed to polymerize to form a solid implant. Regardless of the location of the targeted site, the formed implant can be relocated to a desired implantation site. Typically, the targeted site will be at or near the implantation site for the medical article. When the medical article is intended to reside at a location within the body that is different from the targeted site, the medical article can be formed into a solid implant, and the formed implant can be relocated to the implantation site. For example, a grasping member (such as forceps) can be used to relocate (for example, by pulling) a formed implant from a targeted site to a desired implantation site. The formed implant can then reside at the implantation site during a treatment course.

During administration of the biodegradable polysaccharide composition, while efforts are made at maintaining the administered polymeric material at the targeted site, it is conceivable that some leakage of unpolymerized material may occur. The biodegradable polysaccharide compositions of the invention are clearly advantageous in that any unpolymerized or partially polymerized material lost from the targeted site can be degraded into innocuous products elsewhere in the body.

Optionally, a securement element can be utilized in connection with the biodegradable polysaccharide composition. In these aspects, the securement element can be provided before, simultaneously with, or after, administration of the biodegradable polysaccharide composition. Typically, the securement element is provided prior to polymerization of the biodegradable polysaccharide composition, so that the securement element is incorporated into the biodegradable polysaccharide matrix, when formed. In these aspects, the securement element can be visualized as a "wick," similar to wicks commonly included in candles, in that the securement element passes through and extends from the body of the formed implant. The biodegradable polysaccharide composition can be solidified (cured) around the securement element, such that the formed biodegradable polymeric matrix surrounds the securement element. The formed implant thus comprises a biodegradable polymeric matrix in solid form, having a securement element disposed wherein. The securement element can extend a desirable distance from the biodegradable polymeric matrix, to allow securement (e.g., by suturing) of the formed implant to eye tissues.

The securement element can be any desirable configuration sufficient to provide an anchoring element for the formed implant. Illustrative securement elements include elements formed of known suture material and the like.

In some aspects of the invention, a partial vitrectomy can be performed to hollow out a portion of the vitreous and thereby contain the composition within this hollowed-out area. While this additional step is not required, it can be utilized to assist in minimizing leakage of unpolymerized material from the targeted site.

In some embodiments, the inventive composition can be utilized in combination with a casing for containing the composition. In accordance with the invention, the rate at which bioactive agent is delivered to the treatment site is controlled primarily by the composition of the polysaccharide matrix containing the bioactive agent. In other words, the casing itself does not provide a significant role in controlling the release rate of bioactive agent from the inventive implants. This can provide further benefits, since different biodegradable polysaccharide matrices can be utilized in connection with a single casing that is implanted in a patient, thereby allowing the interventionalist to tailor a release rate to a particular application, and to change that release rate when desired, by simply selecting the desirable biodegradable polysaccharide composition to be included in the casing.

In these aspects, the system can comprise a permeable casing configured for delivery to the targeted site, and a composition comprising a biodegradable polysaccharide. The system is minimally invasive since both the casing and the biodegradable polysaccharide are delivered to the eye with nominal tissue disruption. That is, the casing is delivered to a targeted site within an eye in a compact configuration, and then the biodegradable polysaccharide is delivered within the casing via one or more conduits. The casing is filled with the biodegradable polysaccharide composition *in situ,* and the composition is polymerized to form a matrix that can deliver bioactive agent to the patient. If desired, the biodegradable polysaccharide can be formed into a desired shape, for example, if the casing is formed in a desired shape and is sufficiently filled with the biodegradable polysaccharide.

The permeability of the casing can be achieved by fabricating the casing of a permeable material and/or by providing apertures in the material used to form the casing, the aperatures allowing passage of fluids therethrough. As used herein, "permeability" generally refers to the ability of bioactive agent to pass through the casing. In some particular aspects, the material can also be permeable to other materials, such as water.

The casing is typically relatively thin. In some aspects the casing has a thickness in the range of about 0.1 mm to about 0.5 mm. The relative thinness of the casing allows it to be significantly compacted, facilitating the minimally invasive method. In some aspects, the casing is in a compacted configuration of a scroll or volute. This allows the casing in the compacted configuration to have a cross-sectional diameter of about less about 1 cm, or less than about 0.5 cm. One preferred cross sectional diameter is in the range of about 0.2 to 0.5 cm. Following deployment to a targeted site within the eye, the casing can unroll from the scroll or volute configuration.

The construction and dimensions of the casing can provide an overall shape to the formed implant. In many cases, the casing construction will provide a formed implant having a length that is less than about 1 cm, for example, in the range of about 0.25 cm to about 1 cm. This can, in some embodiments, avoid or reduce risk of the device entering the central visual field. The formed implant (i.e., casing filled with the biodegradable polysaccharide) will have a height, width, and length. In some aspects, the formed implant can occupy a volume of about 200 µl or less within the eye.

The casing is permeable and allows fluid to flow in and out of the casing in the eye. The permeable casing is relatively thin, and preferably has a nominal thickness in the range of about 0.1 mm to about 0.5 mm. This thickness allows the casing to be compacted for insertion into the eye in a minimally invasive manner. Because the casing can be folded into a compact configuration for insertion into a patient, the casing is generally malleable.

The materials used in fabricating the casing are not particularly limited, provided these materials are biocompatible and allow delivery of the bioactive agent to the treatment site. Generally, the casing can be constructed from any suitable biomaterial, or combination of biomaterials, that allow for permeability of the bioactive agent. Preferably, as mentioned, the casing material does not significantly impact the rate of bioactive agent delivery to the treatment site. Rather, the casing functions to provide a reservoir (such as a defined area within the interior of the eye) into which the inventive biodegradable polysaccharide composition can be filled and optionally refilled for treatment of a patient. This can be beneficial, for example, when it is desired to provide an implant at a specific site within the eye that can be retained over time (e.g., be filled and refilled with desirable polysaccharide compositions containing a selected bioactive agent or agents).

In some aspects, the casing is formed of a material that is water-permeable. The casing can be formed from a fabric made of synthetic and/or natural polymeric materials. Exemplary synthetic polymeric materials that can be included in the casing include polyesters, such as Dacron™ or PET (Polyethylene terephthalate), or polytetrafluoroethylene (PTFE), such as Teflon™.

In some embodiments, the casing can be fabricated of an elastic material. Suitable materials for use in forming an elastic casing are well known and may be readily determined by one of skill in the art. For example, some suitable include thin-walled nondistensible materials, such as PET, and more elastomeric materials, such as polyurethane.

In other embodiments, the casing can be formed of a material that is permeable to the bioactive agent. By way of example, some suitable permeable materials may include polycarbonates, polyolefins, polyurethanes, copolymers of acrylonitrile, copolymers of polyvinyl chloride, polyamides, polysulphones, polystyrenes, polyvinyl fluorides, polyvinyl alcohols, polyvinyl esters, polyvinyl butyrate, polyvinyl acetate, polyvinylidene chlorides, polyvinylidene fluorides, polyimides, polyisoprene, polyisobutylene, polybutadiene, polyethylene, polyethers, polytetrafluoroethylene, polychloro ethers, polymethylmethacrylate, polybutylmethacrylate, polyvinyl acetate, nylons, cellulose, gelatin, silicone rubbers and porous rubbers

In some aspects, permeability of the casing arises due to apertures in the casing material. When included, apertures in the casing can be formed, for example, with a laser, hot wire, drilling device or similar mechanism.

In the method and system of the present invention, the casing can be compacted so that it can be delivered to the interior of the eye in a minimally invasive manner. For example, the casing can be in a compacted form of a scroll or volute, which allows it to pass through an incision or other opening during delivery to a targeted site within an eye. The casing in the compacted form can be flexible, which may allow flexion during the insertion process. In some aspects, the casing is compacted to have a cross-sectional diameter of about less about 1 cm, and more preferably less than 0.5 cm. In one aspect the cross sectional diameter is in the range of about 0.2 cm to about 0.5 cm.

Various compact configurations of the casing are contemplated. In some aspects the compact configuration comprises a cross sectional shape that is rounded or circular. For example, the casing can be formed into a scroll or volute. Upon delivery of the casing in a scrolled or volute compact configuration, the casing can be unrolled or unfurled to spread out the casing in the portion of the eye.

In another aspects, the casing in the compact configuration is folded. A casing that is folded may have a cross sectional shape of a square or rectangle. The casing in the compact configuration may include a plurality of folds (pleats). When the casing transitions from a compact to an uncompact configuration in the eye, the casing can expand in a manner similar to that of an expanding accordion.

The flowable form of the biodegradable polysaccharide composition is capable of being delivered to an interior portion of the casing *in situ,* for example, by injection.

After the casing has been deployed in the eye, it can transition from a compacted to an uncompacted configuration. In this transition, the casing essentially spreads out within the targeted site. The transition may be characterized by the unfolding, unrolling, or unfurling (or combinations of these events depending on the compacted configuration) of the casing within the targeted site.

The transition can be facilitated by, for example, the material properties and/or the construction of the casing, or by performing an action that will facilitate the transition. In many cases, the process of delivering the biodegradable polysaccharide composition via the delivery conduit can be sufficient to cause the transition to the uncompacted configuration. That is, the pressure exerted by the biodegradable polysaccharide composition on the inner walls of the casing will be sufficient for it to unfold or unravel.

The steps of delivering the casing and/or polysaccharide composition to the interior of the casing can be performed using standard ophthalmic visualization.

In some aspects, utilization of a casing can provide a refillable casing for the biodegradable polysaccharide composition. That is, the casing can remain in the eye after the biodegradable polysaccharide composition has degraded. If needed, another volume of biodegradable polysaccharide can be delivered to the interior of the casing, for example, by simply injecting the additional volume of composition into the casing. Such additional volume can be delivered at any suitable time, for example, when a portion or all of the original biodegradable polysaccharide composition has degraded from within the casing.

When a casing is utilized in combination with the biodegradable polysaccharide composition, the formed implant (i.e., casing with formed matrix of biodegradable polysaccharide contained within) can be completely contained within the interior of the eye. This can provide advantages over known reservoir-type devices implanted in the eye that include a conduit or lumen that passes to the exterior of the eye, to allow for refilling of the reservoir within the eye. In contrast, the inventive casing with biodegradable polysaccharide matrix contained therein can be attached to eye tissues (e.g., by a suitable suture or other securement element) without passing through the eye tissues to the exterior of the eye. This can reduce risk of infection, loss of pressure within the eye, tissue damage, and the like that can result from breaching the barrier between the interior and exterior of the eye by a conduit or other lumen.

Optionally, the casing can be secured to eye tissues, for example, the scleral tissues of the eye. In some embodiments, the casing can be secured to an internal scleral surface. Securement can be accomplished by using securement elements, such as sutures that are in contact with scleral tissues.

Once the biodegradable polysaccharide composition has been administered to the targeted site within the patient body, the composition is permitted to polymerize to form a solid implant. The biodegradable polysaccharide composition includes an initiator that is capable of promoting the formation of a reactive species from the coupling group. The initiator can be provided as a photoinitiator or a redox initiator. Polymerization initiation will thus depend upon the particular initiator(s) chosen. Polymerization of the composition can be induced by a variety of means such as irradiation with light of suitable wavelength, or by contacting the members of the redox pair.

In some aspects the initiator is a compound that is light sensitive and that can be activated to promote the coupling of the polysaccharide via a free radical polymerization reaction ("photoinitiators"). In some aspects it is preferred to use photoinitiators that are activated by light wavelengths that have no or a minimal effect on bioactive agent present in the composition. In some aspects, it is preferred to use photoinitiators that are activated by light wavelengths that pose minimal or no risk of damage to eye tissues (e.g., the retina) during application of the inventive methods.

When irradiation is employed, irradiation with light in the visible range is preferred. UV irradiation can be accomplished in the visible wavelength range using a standard ophthalmic source of light in the visible or LWUV wavelength range, polymerization generally occurs in about 2 seconds to about 3 minutes, usually in about 2 seconds to about 30 seconds, typically at an exposure distance in the range of about 2 cm or less.

The biodegradable polysaccharide composition can be treated to activate the photoinitiator and promote polymerization and matrix formation during and/or after the composition has been administered to the targeted site. For example, an amount of biodegradable polysaccharide composition can be administered and irradiated at the time of application, or administered and then irradiated after administration, or combinations thereof. The steps of administering and irradiating can be performed once, or more than one time during the overall process. For example, if it is desired to build up the thickness of the matrix, the steps of administering and irradiating can be performed multiple times during the overall process of matrix formation.

In performing the step of irradiating, any suitable visible light-emitting source can be used. In ophthalmology, many diagnostic and therapeutic devices are equipped with a bright light source to illuminate the fundus of the eye. In most instances, the light is applied through the intact eye (transpupillary, through the lens and cornea). In other instances, fiberoptic endoillumination can be utilized by trans pars plana insertion of the light source. However, during trans pars plana endoillumination, the procedure bypasses the eye media and the threshold for damage by visible radiation to the retina is substantially decreased. The safety of an endoilluminator light source is usually determined by measuring its aphakic retinal hazard function. Phototoxcitiy created by exposure to an endoilluminator can be either thermal or photochemical in nature. Thermal photoxicity is usually not a concern with endoilluminators (although if the light source touches the retina, thermal damage may occur). Phototoxicity is generally thought to occur when light within the ultraviolet or blue wavelengths is utilized. Therefore, when a light source is inserted into the vitreous for endoillumination, additional caution should be exercised regarding wavelength, power, and distance from the retina of the light source.

A standard fiberoptic endoillumination probe for vitreous surgery can include a 300 µm silica fiber embedded in a 20- to 25-gauge needle hand piece with a typical acceptance angle of 20 degrees in water. A commercially available endoillumination probe is the Fiberoptic Endoilluminator™ (Storz Ophthalmics, St. Louis, MO).

Commonly used visible light-emitting sources include metal halide sources, halogen sources, zenon sources, and conventional ophthalmic lasers. The visible light-emitting sources can be any sources that are capable of generating visible light within wavelengths that promote activation of the photoinitiator. Light sources having a wavelength in the range of about 250 nm to about 750 nm can be used, and preferably light sources having a more specific light emission, wherein primarily visible light is emitted, are utilized. For example, in many aspects light sources primarily emitting wavelengths of about 400 nm or greater are used.

Metal halide lamps are suitable sources and are commercially available (for example, as part of the Millennium™ microsurgical system from Bausch & Lomb, Rochester, NY).

Halogen lamps, such as are available as part of the Accurus™ systems from Alcon Canada (Mississauga, ON), can be used.

Xenon light sources have more recently become commercially available and can provide more powerful light sources as compared to the conventional halogen and metal halide sources. Commercially available xenon light sources include, for example, the Synergetics Photon™ light source (Synergetics USA, Inc. O'Fallon, MO) and Alcon Xenon system.

Activation of the photoinitiators can be accomplished using known ophthalmic laser systems to provide light of suitable wavelength. A wide variety of ophthalmic laser systems are commercially available. Selection of a particular laser can depend upon such factors as photoinitiator(s) selected, the desired wavelength for activation, desired curing time, power, location of the implantation site, and the like. Ophthalmic laser treatments comprise a variety of modalities to combat different diseases or indications. For example, ion or dye lasers, such as Argon and Krypton lasers, produce wavelengths of about 488 or 514 nm for Argon, 648 nm for Krypton and are commonly used in various photocoagulation procedures. Given the present teaching, one of skill in the art can readily select a suitable laser system for activation of selected photoinitiator(s) in a particular composition.

When the initiator comprises a photoinitiator, suitable curing time can be selected so that the matrix is cured into a stable polymeric matrix within a suitable working time for a surgeon. An illustrative curing time can be in the range of about 2 seconds to about 3 minutes.

Light from the light source is applied in an amount sufficient to promote formation of the matrix of the administered composition given the components of the matrix forming composition and the light source used. Generally, the amount of energy that is applied to the administered matrix will depend upon the light intensity and duration of the light treatment. Light intensity is the amount of power distributed over a given area. Light intensity can be increased or decreased by adjusting the amount of total power, or adjusting the area of distribution of the light (for example, by the distance the light is placed from the disposed composition). Light intensity values can be obtained for any particular light source by measurement with a radiometer.

The light source can be placed a desired distance from the disposed composition. Generally, the distance that the light source is placed will depend upon the spot size of the applied light, the area of the administered composition, and whether illumination will be performed endoscopically or trans-pupilary (through the lens and cornea). Typically, it is desirable to optimize the distance from the tip of the light to the disposed composition to provide the maximum intensity at the composition, thereby minimizing the cure time (i.e., time for matrix formation).

In some embodiments, the biodegradable polysaccharide composition can be administered through the pars plana at a first site, and the activating light can be administered through the pupil. In these aspects, a single injection site is utilized, thereby minimizing injection sites at the surface of the body.

Alternatively, the biodegradable polysaccharide composition can be administered through the pars plana at a first site, and the activating light can be administered by creating a second administration site (e.g., incision) at a location separate from the first site. In these aspects, a second instrument is passed through the pars plana at a second site, the second instrument being placed internally within the eye to provide the activating light source (e.g., endoluminal illumination procedures). This approach can also minimize risk of damage to the macula, as the activating light can be more easily aimed toward the peripheral retina.

During the step of activating, the initiator is activated to promote the crosslinking of two or more natural biodegradable polysaccharides via their coupling groups. In preferred aspects the natural biodegradable polysaccharide includes a polymerizable group, such as an ethylenically unsaturated group, and initiator is capable of initiating free radical polymerization of the polymerizable groups.

In some modes of practice, in order to promote polymerization of the biodegradable polysaccharides in a composition to form a matrix, an oxidizing agent is added to a reducing agent in the presence of the one or more biodegradable polysaccharides. These methodologies thus involve the use of a redox pair to initiate polymerization of the polysaccharides, thereby forming a polysaccharide matrix. The polysaccharide matrix comprises a solid implant within the patient body that is capable of delivering bioactive agent as described herein. For example, a composition including a biodegradable polysaccharide and a reducing agent can be added to a composition including an oxidizing agent, or a composition including a biodegradable polysaccharide and an oxidizing agent is added to a composition containing a reducing agent. One desirable method of preparing a matrix is to combine a composition including a biodegradable polysaccharide and an oxidizing agent with a composition including a biodegradable polysaccharide and a reducing agent. For purposes of describing this method, the terms "first composition" and "second composition" can be used.

In one aspect, polymerization of the composition is promoted *in situ,* such as at a targeted site for forming a biodegradable implant with the polymerized mass of material.

A biodegradable implant formed of the inventive polysaccharide composition including bioactive agent can provide enhanced site-specific bioactive agent delivery to tissues of the eye.

In the process, first and second compositions are delivered to the ocular targeted site via a delivery device (e.g., cannula or syringe). The delivery device will generally have a very small diameter, such as described herein. The inventive compositions, which can be used at low viscosities to form biodegradable ocular implants, can be delivered through delivery devices of these sizes at an acceptable flow rate without risk of clogging the lumen of the delivery device.

Commencement of polymeric matrix formation can occur before, during, and/or after the composition is delivered to the targeted site. Depending upon the particular redox initiators selected, polymerization initiation can commence immediately upon contact of the members of the redox pair, or at a time period subsequent to initial contact of the members of the redox pair. In accordance with the latter embodiments, polymerization initiation can be delayed for a desired amount of time after initial contact of the members of the redox pair, for example on the order of seconds or minutes after initial contact.

In one mode of practice, a dual lumen delivery device can be inserted into the eye of a patient and navigated to place the distal end of the delivery device at the targeted site. First and second compositions that include natural biodegradable polysaccharides and, individually, an oxidizing agent, and a reducing agent can be delivered to and mixed within the targeted site. Based upon the polymerizable compositions described herein, it has been found that these compositions can be delivered through very small diameter delivery devices. While the compositions are particularly suitable for being delivered via a small diameter delivery device, the compositions can also be delivered via larger diameter delivery devices. Larger diameter delivery devices (e.g., catheters) can be used to deliver the compositions to other areas of the body that would accommodate larger delivery devices.

In alternative embodiments, a polymerization initiator system is activated prior to delivering the composition to the targeted site via the delivery device. For example, in some modes of practice, a first composition including an oxidizing agent, and a second composition including a reducing agent are combined, mixed, and then injected into the targeted site. One type of suitable mixing device comprises injection ports and a chamber having a series of baffles in which the compositions are mixed (Mixpac™; commercially available from Mixpac™ Systems AG, Rotkreuz, CH). Mixing of the composition occurs immediately prior to introduction of the mixed compositions into the targeted site.

Mixing of the first composition and second composition at the targeted site results in crosslinking and formation of the biodegradable polysaccharide matrix. In accordance with these aspects of the invention, the polysaccharide matrix can be formed into a solid implant in time frames on the order of about 1 second to about ten minutes, when the targeted site is within a patient eye. The time for formation of the matrix can depend upon such factors as, for example, the concentration of oxidizing and reducing agents within the compositions, the composition of the particular targeted site (e.g., vitreous humor, subretinal space, or other aqueous or non-aqueous site), and the like.

Ophthalmic articles can also be configured for placement within any desired tissues of the eye. For example, ophthalmic devices can be configured for placement at a subconjunctival area of the eye, such as devices positioned extrasclerally but under the conjunctiva, such as glaucoma drainage devices and the like. The above-described methods and equipment can be modified for such procedures.

In some aspects of the invention, the biodegradable polysaccharide composition is placed in contact with an aqueous solution. The biodegradable polysaccharide composition is designed to be stable in the presence of the aqueous solution provided that an enzyme that causes the degradation of the natural biodegradable polysaccharide (or another degrading agent) is not present in an amount sufficient to cause substantial degradation of the composition.

For example, the invention provides a shelf stable composition comprising a natural biodegradable polysaccharide comprising coupling groups. These compositions could be obtained or prepared, according to the details provided herein, and then stored for a period of time before the composition is used to form a biodegradable medical article, without the significant degradation of the natural biodegradable polysaccharide occurring during storage.

Accordingly, the invention also provides methods for preparing a biodegradable medical article comprising preparing a biodegradable composition comprising a natural biodegradable polysaccharide comprising coupling group; storing the biodegradable composition for an amount of time; and then using the biodegradable composition to prepare a medical article *in vivo* in an eye of a patient. Optionally, one or more bioactive agents and/or microparticles can be added before or after storage of the biodegradable composition.

In a related aspect, the invention also provides the advantage of being able to perform synthetic and post-synthetic procedures wherein the natural biodegradable polysaccharide is contacted with an aqueous composition, and there is minimal risk of degradation of the polysaccharide. For example, the natural biodegradable polysaccharide may be contacted with an aqueous solution for purification without risking significant degradation of the natural biodegradable polysaccharide.

A carbohydrase that contacts the article can specifically degrade the natural biodegradable polysaccharide causing release of the bioactive agent. Examples of carbohydrases that can specifically degrade natural biodegradable polysaccharide matrices include α-amylases, such as salivary and pancreatic α-amylases; disaccharidases, such as maltase, lactase and sucrase; trisaccharidases; and glucoamylase (amyloglucosidase).

Vitreal concentrations for amylase are estimated to be in the range of about 50 -100 U per liter, and serum concentrations also fall within this range (Varela, R.A., and Bossart, G.D. (2005) J Am Vet Med Assoc 226:88-92).

In some aspects, the carbohydrase can be administered to a subject to increase the local concentration, for example in the tissue or serum surrounding the implanted device, so that the carbohydrase may promote the degradation of the medical article. Exemplary routes for introducing a carbohydrase include local injection, intravenous (IV) routes, and the like. Alternatively, degradation can be promoted by indirectly increasing the concentration of a carbohydrase in the vicinity of the medical article, for example, by a dietary process, or by ingesting or administering a compound that increases the systemic levels of a carbohydrase.

In other cases, the carbohydrase can be provided on a portion of the medical article itself. For example the carbohydrase can be present in a microparticle in one or more portions the biodegradable matrix. As the carbohydrase is released from the microparticle, it causes degradation of the matrix and promotes the release of the bioactive agent.

The biodegradable polysaccharide compositions as described herein can be used to fabricate a variety of implantable medical articles *in vivo.* The medical article can be any article that is introduced into a mammal for the prophylaxis or treatment of a medical condition. In particular, these devices can be devices suitable for ophthalmic use, as mentioned herein. The particular form of the implant (e.g., filament, capsule, rod, disc, and the like) can be determined based upon the configuration of the implantation site. For example, for a subretinal implantable device, the implant can be formed as a filament or rod, to accommodate the subretinal space. When a casing is utilized, the dimensions and/or shape of the casing can impact the form of the implant.

The biodegradable polysaccharide compositions are particularly useful for forming biodegradable medical articles that will come in contact with aqueous systems. The body fluids typically have enzymes that allow for the degradation of the natural biodegradable polysaccharide-based composition. The aqueous system (such as bodily fluids) allows for the degradation of the biodegradable composition and release of the bioactive agent from the article. In some cases, depending on the bioactive agent and the matrix, the bioactive agent can diffuse out of the matrix. For example, it has been demonstrated that a loosely formed matrix may allow some diffusion of bioactive agents, particularly smaller bioactive agents. More desirably, well-formed matrices having signification polysaccharide association via coupling groups are able to retain bioactive agents. Release of bioactive agents from these matrices is mediated by enzymatic degradation.

The polymeric compositions are be utilized to form an ophthalmic article *in vivo.* The ophthalmic article is configured for placement at an internal site of the eye. Suitable ophthalmic articles in accordance with these aspects can provide bioactive agent to any desired area of the eye. In some aspects, the articles can be utilized to deliver bioactive agent to an anterior segment of the eye (in front of the lens), and/or a posterior segment of the eye (behind the lens). Suitable ophthalmic articles can also be utilized to provide bioactive agent to tissues in proximity to the eye, when desired.

Suitable external articles can be configured for topical administration of bioactive agent. Such external articles can reside juxtasclerally (e.g., subconjunctivally, yet exterior to the globe).

Articles configured for placement at an internal site of the eye can reside within any desired area of the eye. In some aspects, the ophthalmic article can be formed at an intraocular site, such as the vitreous.

In some aspects, the ophthalmic article can be formed at a subretinal area within the eye. In some embodiments, the outer diameter (or maximum cross-sectional dimension) of the ophthalmic article is no greater than about 1000 µm in order to minimize retinal detachments and hemorrhaging. In other embodiments, the outer diameter (or maximum cross-sectional dimension) of the device is 900 µm or less, in other embodiments 800 µm or less, in other embodiments 700 µm or less, in other embodiments 600 µm or less, in other embodiments 500 µm or less, in other embodiments 400 µm or less, in other embodiments 300 µm or less, in other embodiments 200 µm or less, in other embodiments 100 µm or less, in other embodiments 100 µm or less. Typically, the diameter (or maximum cross-sectional dimension) ranges from about 200 µm to about 500 µm.

In some embodiments, the length of the ophthalmic article for subretinal application is about 5.0 mm or less, in other embodiments about 4.5 mm or less, in other embodiments about 4.0 mm or less, in other embodiments about 3.5 mm or less. In a specific embodiment, the article is about 3.0 mm or less in length, as such lengths have been found to provide the additional benefit of coming to a resting point in the eye that does not cross multiple tissue layers. However, it is possible to provide articles longer than about 3.0 mm that can be inserted with special care so as to minimize multiple tissue layer crossing. In other embodiments, the length of the article is 2.9 mm or less, in other embodiments about 2.8 mm or less, in other embodiments about 2.7 mm or less, in other embodiments about 2.6 mm or less, in other embodiments about 2.5 mm or less, in other embodiments about 2.4 mm or less, in other embodiments about 2.3 mm or less, in other embodiments about 2.2 mm or less, in other embodiments about 2.1 mm or less, in other embodiments about 2.0 mm or less. In some embodiments, the length of the article is in the range of about 2.0 to about 3.0 mm.

In some aspects, the invention provides a biodegradable implant that is formed from the biodegradable polysaccharide composition and that includes a bioactive agent, such as a high molecular weight bioactive agent useful for treating an ocular condition.

The invention provides a method for forming an article from the biodegradable polysaccharide composition, wherein the method includes polymerizing a composition that includes the biodegradable polysaccharide within the eye, such as in a subretinal area or within the vitreous. For example, the methods can utilize a low viscosity composition including a natural biodegradable polysaccharide and a redox pair to promote polymerization for *in situ* matrix formation.

Ophthalmic articles can also be configured for placement within any desired tissues of the eye. For example, ophthalmic articles can be configured for placement at a subconjunctival area of the eye, such as devices positioned extrasclerally but under the conjunctiva, such as glaucoma drainage devices and the like.

In some aspects of the invention the natural biodegradable polymer is used to form the body member of a medical implant, wherein the body member has a wet weight of about 10 g or less, or a dry weight of about 2.5 g or less.

In some aspects, the medical article formed by the natural biodegradable polysaccharide matrix can be a medical device that performs a function within the eye (that is, a function in addition to, or in substitution of, bioactive agent delivery). For example, the compositions can be utilized to form a mechanical tamponade for treatment of retinal detachment. Mechanical tamponades are known, for example, composed of biologically inert fluids such as an oil (e.g., silicone oil or a silicone-fluorosilicone copolymer oil) or gas.

Thus the invention provides methods for forming a medical device within the eye. In an illustrative embodiment, the medical device is a tamponade that can be used to treat retinal detachment. The inventive compositions can provide advantages over known treatment options for retinal detachment.

Conventional techniques for management of retinal detachment include application of an extra-scleral device and/or injection of a material to tamponade the retina while reattachment can occur. One conventional treatment option is a scleral buckle, which is a type of silicone explant that is mounted over the sclera 360 degrees and tightened in order to indent the sclera and make it apposed to the underlying detached retina. A second conventional technique, pneumatic retinopexy, involves intra-ocular injection of gas (air or expandable gas) in order to tamponade the retinal detachment and break while the choroidal adhesions form. However, for pneumatic retinopexy, each procedure requires location of the tear and treating the retina around its edges by cryotherapy or laser in order to create firm adhesions between the sensory retina and the RPE layer and preventing detachment. The gas bubble will expand, and being lighter than the ocular fluids, will migrate upward to tamponade superior breaks. Hence positioning post-op is critical - if the break is in the posterior pole (close to the macula), the patient should remain face down. If the break was in the right temporal retina, he should lie flat on his left side. Positioning should be applied for the first 2 weeks. A third conventional technique involves vitrectomy with oil injection. The oil (e.g., silicone oil) is injected to tamponade the break and detachment for a prolonged time, in fear of recurrence. Moreover, silicone oil should be removed subsequently after 3 to 12 months to prevent toxicity to the cornea, lens (cataract), trabecular meshwork (glaucoma), etc..

In contrast to known tamponade materials, the inventive biodegradable polysaccharide composition can form medical devices that are biocompatible, can remain in the body for extended periods of time without eliciting an adverse response, can degrade into materials acceptable to the ocular environment, and thus do not require removal. Further, the biodegradable polysaccharide matrix can be formulated to provide a specific gravity substantially equivalent to the specific gravity of the vitreous. In these aspects, the medical device formed by the biodegradable polysaccharide matrix can remain in place once formed *in situ* in the eye, and does not require immobilization of the patient subsequent to formation *in situ.*

Thus, in some aspects, the invention provides an *in situ* formed medical device for treating a detached retina in an eye, the medical device comprising a crosslinked natural biodegradable polysaccharide

Optionally, a portion of the vitreal humor can be removed prior to, or simultaneously with, administration of the composition to the vitreal chamber.

The medical devices (such as a tamponade) include one or more bioactive agents, as described elsewhere herein.

In some aspects of the invention, the natural biodegradable polysaccharide compositions can be used to form an optically clear matrix. For example, maltodextrin and polyalditol can be formed into optically clear matrices using either redox or photoinitiation. Factors that can affect the ability of the formed matrix to be optically clear include the water solubility of the macromers utilized to form the matrix, and/or transparency of the initiating reagents. It will be readily appreciated that optically clear matrices formed in accordance with the invention can provide significant benefits, since such matrices can form implants that will not adversely impact the patient's vision (e.g., by creating blind spots by virtue of interference from the implant material). In turn, this can allow more flexibility as to the size and/or location of a formed implant within the interior of the eye.

The invention will be further described with reference to the following non-limiting Examples.

Unless otherwise indicated, all percentages are by weight.

### Example 1

### Synthesis of acrylated-amylose

Amylose having polymerizable vinyl groups was prepared by mixing 0.75g of amylose (A0512; Aldrich) with 100 mL of methylsulfoxide (JT Baker) in a 250 mL amber vial, with stirring. After one hour, 2 mL of triethylamine (TEA; Aldrich) was added and the mixture was allowed to stir for 5 minutes at room temperature. Subsequently, 2 mL of glycidyl acrylate (Polysciences) was added and the amylose and glycidyl acrylate were allowed to react by stirring overnight at room temperature. The mixture containing the amylose-glycidyl acrylate reaction product was dialyzed for 3 days against distiled (DI) water using continuous flow dialysis. The resultant acrylated-amylose (0.50g; 71.4% yield) was then lyophilized and stored desiccated at room temperature with protection from light.

### Example 2

### Synthesis of MTA-PAAm

A polymerization initiator was prepared by copolymerizing a methacrylamide having a photoreactive group with acrylamide.

A methacrylamide-oxothioxanthene monomer (N-[3-(7-Methyl-9-oxothioxanthene-3-carboxamido) propyl]methacrylamide (MTA-APMA)) was first prepared. N-(3-aminopropyl)methacrylamide hydrochloride (APMA), 4.53 g (25.4 mmol), prepared as described in U.S. Patent No. 5,858,653, Example 2, was suspended in 100 mL of anhydrous chloroform in a 250 mL round bottom flask equipped with a drying tube. 7-methyl-9-oxothioxanthene-3-carboxylic acid (MTA) was prepared as described in U.S. Patent No. 4,506,083, Example D. MTA-chloride (MTA-Cl) was made as described in U.S. Patent No. 6,007,833, Example 1. After cooling the slurry in an ice bath, MTA-Cl (7.69 g; 26.6 mmol) was added as a solid with stirring to the APMA-chloroform suspension. A solution of 7.42 mL (53.2 mmol) of TEA in 20 mL of chloroform was then added over a 1.5 hour time period, followed by a slow warming to room temperature. The mixture was allowed to stir 16 hours at room temperature under a drying tube. After this time, the reaction was washed with 0.1 N HCl and the solvent was removed under vacuum after adding a small amount of phenothiazine as an inhibitor. The resulting product was recrystallized from tetrahydrofuran (THF)/toluene (3/1) and gave 8.87 g (88.7% yield) of product after air drying. The structure of MTA-APMA was confirmed by NMR analysis.

MTA-APMA was then copolymerized with acrylamide in DMSO in the presence of 2-mercaptoethanol (a chain transfer agent), N,N,N',N'-tetramethyl-ethylenediamine (a cocatalyst), and 2,2'-azobis(2-methyl-propionitrile) (a free radical initiator) at room temperature. The solution was sparged with nitrogen for 20 minutes, sealed tightly, and incubated at 55°C for 20 hours. The solution was dialyzed for 3 days against DI water using continuous flow dialysis. The resultant MTA-PAAm was lyophilized, stored desiccated, and protected from light at room temperature.

### Example 3

### Preparation of 1-(6-oxo-6-hydroxyhexyl)maleimide (Mal-EACA)

A maleimide functional acid was prepared in the following manner, and was used in Example 4. EACA (6-aminocaproic acid), (100 g; 0.762 moles), was dissolved in 300 mL of acetic acid in a three-neck, three liter flask equipped with an overhead stirrer and drying tube. Maleic anhydride, (78.5 g; 0.801 moles), was dissolved in 200 mL of acetic acid and added to the EACA solution. The mixture was stirred one hour while heating on a boiling water bath, resulting in the formation of a white solid. After cooling overnight at room temperature, the solid was collected by filtration and rinsed two times with 50 mL of hexane each rinse. After drying, the yield of the (z)-4-oxo-5-aza-undec-2-endioic acid (Compound 1) was in the range of 158-165 g (90-95%) with a melting point of 160-165°C. Analysis on an NMR spectrometer was consistent with the desired product: ¹H NMR (DMSO-d₆, 400 MHz) δ 6.41, 6.24 (d, 2H, J = 12.6 Hz; vinyl protons), 3.6-3.2 (b, 1H; amide proton), 3.20-3.14 (m, 2H: methylene adjacent to nitrogen), 2.20 (t, 2H, J = 7.3; methylene adjacent to carbonyl), 1.53-1.44 (m, 4H; methylenes adjacent to the central methylene), and 1.32-1.26 (m, 2H; the central methylene).

(z)-4-oxo-5-aza-undec-2-endioic acid, (160 g; 0.698 moles), zinc chloride, 280 g (2.05 moles), and phenothiazine, 0.15 g were added to a two liter round bottom flask fitted with an overhead stirrer, condenser, thermocouple, addition funnel, an inert gas inlet, and heating mantle. Chloroform (CHCl₃), 320 mL was added to the 2 liter reaction flask, and stirring of the mixture was started. Triethylamine (480 mL; 348 g, 3.44 moles (TEA)) was added over one hour. Chlorotrimethyl silane (600 mL; 510 g, 4.69 moles) was then added over two hours. The reaction was brought to reflux and was refluxed overnight (~16 hours). The reaction was cooled and added to a mixture of CHCl₃ (500 mL), water (1.0 liters), ice (300 g), and 12 N hydrochloric acid (240 mL) in a 20 liter container over 15 minutes. After 15 minutes of stirring, the aqueous layer was tested to make sure the pH was less than 5. The organic layer was separated, and the aqueous layer was extracted three times with CHCl₃ (700 mL) each extraction. The organic layers were combined and evaporated on a rotary evaporator. The residue was then placed in a 20 liter container. A solution of sodium bicarbonate (192 g) in water (2.4 liters) was added to the residue. The bicarbonate solution was stirred until the solids were dissolved. The bicarbonate solution was treated with a solution of hydrochloric acid, (26 liters of 1.1 N) over 5 minutes to a pH of below 2. The acidified mixture was then extracted with two portions of CHCl₃, (1.2 liters and 0.8 liters) each extraction. The combined extracts were dried over sodium sulfate and evaporated. The residue was recrystallized from toluene and hexane. The crystalline product was then isolated by filtration and dried which produced 85.6 g of white N-(6-oxo-6-hydroxyhexyl)maleimide (Mal-EACA; Compound 2). Analysis on an NMR spectrometer was consistent with the desired product: ¹H NMR (CDCl₃, 400 MHz) δ 6.72 (s, 2H; maleimide protons), 3.52 (t, 2H, J = 7.2 Hz; methylene next to maleimide), 2.35 (t, 2H, J = 7.4; methylene next to carbonyl), 1.69 - 1.57 (m, 4H; methylenes adjacent to central methylene), and 1.39-1.30 (m, 2H; the central methylene). The product had a DSC (differential scanning calorimator) melting point peak at 89.9°C.

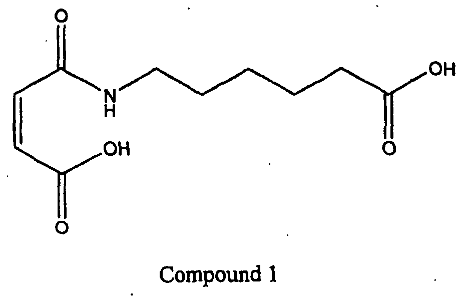

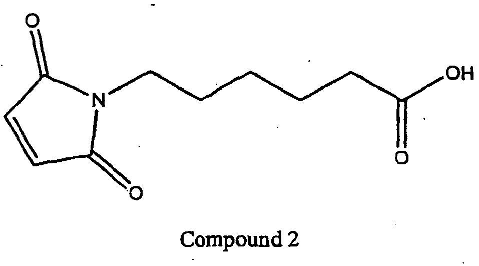

### Example 4

### Preparation of N-(5-isocyanatopentyl)maleimide (Mal-C5-NCO)

Mal-EACA from Example 3 (5.0 g; 23.5 mmole) and CHCl₃ (25 mL) were placed in a 100 mL round bottom flask and stirred using a magnetic bar with cooling in an ice bath. Oxalyl chloride (10.3 mL; ~15 g; 118 mmole) was added and the reaction was brought to room temperature with stirring overnight. The volatiles were removed on a rotary evaporator, and the residue was azetroped with three times with 10 mL CHCl₃ each time. The intermediate Mal-EAC-Cl [N-(6-oxo-6-chlorohexyl)maleimide] (Compound 3) was dissolved in acetone (10 mL) and added to a cold (ice bath) stirred solution of sodium azide (2.23 g; 34.3 mmole) in water (10 mL). The mixture was stirred one hour using an ice bath. The organic layer was set aside in an ice bath, and the aqueous layer was extracted three times with 10 mL CHCl₃. All operations of the acylazide were done at ice bath temperatures. The combined organic solutions of the azide reaction were dried for an hour over anhydrous sodium sulfate. The N-(6-oxo-6-azidohexyl)maleimide (Compound 4) solution was further dried by gentle swirling over molecular sieves over night. The cold azide solution was filtered and added to refluxing CHCl₃, 5 mL over a 10 minute period. The azide solution was refluxed for 2 hours. The weight of Mal-C5-NCO (Compound 5) solution obtained was 55.5 g, which was protected from moisture. A sample of the isocyanate solution, 136 mg was evaporated and treated with DBB (1,4-dibromobenzene), 7.54 mg and chloroform-d, 0.9 mL: ¹H NMR (CDCl₃, 400MHz) δ 6.72 (s,2H), 3.55 (t, 2H, J = 7.2 Hz), 3.32 (t, 2H, J = 6.6 Hz), 1.70-1.59 (m, 4H), 1.44-1.35 (m, 2H). The NMR spectra was consistent with desired product. The DBB internal standard δ at 7.38 (integral value was 2.0, 4H; per mole of product) was used to estimate the moles of Mal-C5-NCO in solution. The calculated amount of product in solution was 23.2 mmole for a yield of 98% of theory. NCO reagent (concentration was 0.42 mmole/g) was used to prepare a macromer in Example 14.

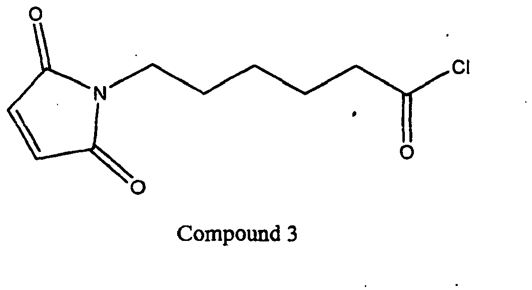

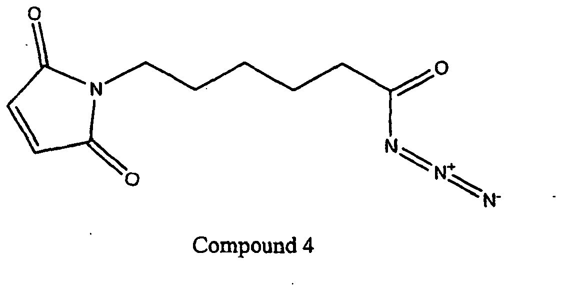

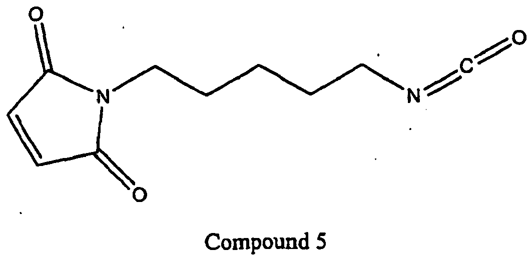

### Example 3

### Preparation of 3-(acryloyloxy)propanoic acid (2-carboxyethyl acrylate; CEA)

Acrylic acid (100 g; 1.39 mole) and phenothiazine (0.1 g) were placed in a 500 mL round bottom flask. The reaction was stirred at 92°C for 14 hours. The excess acrylic acid was removed on a rotary evaporator at 25°C using a mechanical vacuum pump. The amount of residue obtained was 51.3 g. The CEA (Compound 6) was used in Example 6 without purification.

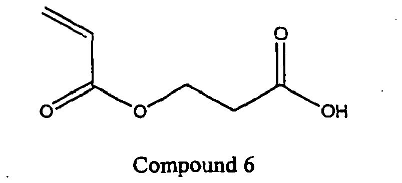

### Example 6

### Preparation of 3-chloro-3-oxopropyl acrylate (CEA-C1)

CEA from Example 5 (51 g; ~ 0.35 mole) and dimethyl formamide (DMF; 0.2 mL; 0.26 mmole) were dissolved in CH₂Cl₃ (100 mL). The CEA solution was added slowly (over 2 hours) to a stirred solution of oxalyl chloride (53 mL; 0.61 mole), DMF (0.2 mL; 2.6 mmole), anthraquinone (0.5 g; 2.4 mmole), phenothiazine (0.1 g, 0.5 mmole), and CH₂Cl₃ (75 mL) in a 500 mL round bottom flask in an ice bath at 200 mm pressure. A dry ice condenser was used to retain the CH₂Cl₃ in the reaction flask. After the addition was complete the reaction was stirred at room temperature overnight. The weight of reaction solution was 369 g. A sample of the CEA-Cl (Compound 7) reaction solution (124 mg) was treated with 1,4-dibromobenzene (DBB, 6.85 mg) evaporated and dissolved in CDCl₃: ¹H NMR (CDCl₃, 400 MHz) δ 7.38 (s, 4H; DBB internal std.), 6.45 (d, 1H, J=17.4 Hz), 6.13 (dd, 1H, J = 17.4, 10.4 Hz), 5.90 (d, 1H, J =10.4 Hz), 4.47 (t, 2H, J = 5.9 Hz), 3.28 (t, 2H, J = 5.9). The spectra was consistent with the desired product. There was 0.394 mole DBB for 1.0 mole CEA-Cl by integration, which gave a calculated yield of 61%. Commercially available CEA (426 g; Aldrich) was reacted with oxalyl chloride (532 mL) in a procedure similar to the one listed above. The residue CEA-Cl (490 g) was distilled using an oil bath at 140°C at a pressure of 18 mm Hg. The distillate temperature reached 98°C and 150 g of distillate was collected. The distillate was redistilled at 18 mm Hg at a maximum bath temperature of 120°C. The temperature range for the distillate was 30°C to 70°C which gave 11 g of material. The distillate appeared to be 3-chloro-3-oxopropyl 3-chloropropanoate. The residue of the second distillation (125 g; 26 % of theory) was used in Example 7.

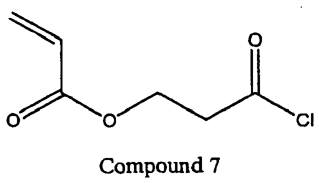

### Example 7

### Preparation of 3-azido-3-oxopropyl acrylate, (CEA-N3)

CEA-Cl from Example 10 (109.2 g; 0.671 mole) was dissolved in acetone (135 mL). Sodium azide (57.2 g; 0.806 mole) was dissolved in water (135 mL) and chilled. The CEA-Cl solution was then added to the chilled azide solution with vigorous stirring in an ice bath for 1.5 hours. The reaction mixture was extracted two times with 150 mL of CHCl₃ each extraction. The CHCl₃ solution was passed through a silica gel column 40 mm in diameter by 127 mm. The 3-azido-3-oxopropyl acrylate (Compound 8) solution was gently agitated over dried molecular sieves at 4°C overnight. The dried solution was used in Example 8 without purification.

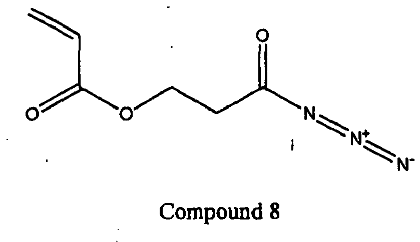

### Example 8

### Preparation of 2-isocyanatoethyl acrylate (EA-NCO)

The dried azide solution (from Example 7) was slowly added to refluxing CHCl₃, 75 mL. After the addition was completed, refluxing was continued 2 hours. The EA-NCO (Compound 9) solution (594.3 g) was protected from moisture. A sample of the EA-NCO solution (283.4 mg) was mixed with DBB (8.6 mg) and evaporated. The residue was dissolved in CDCl₃: ¹H NMR (CDCl₃, 400 MHz) δ 7.38 (s, 4H; DBB internal std.), 6.50 (d, 1H, J = 17.3 Hz), 6.19 (dd, 1H, J = 17.3, 10.5 Hz), 5.93 (d, 1H, J=10.5 Hz), 4.32 (t, 2H, J = 5.3 Hz), 3.59 (t, 2H, J = 5.3). The spectra was consistent with the desired EA-NCO. There was 0.165 mole DBB for 1.0 mole EA-NCO by integration, which gave a calculated concentration of 110 mg EA-NCO/g of solution. The EA-NCO solution was used to prepare a macromer in Example 9.

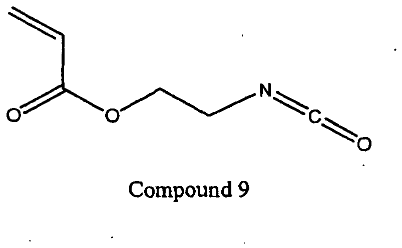

### Example 9

### Preparation of maltodextrin-acrylate macromer (MD-Acrylate)

Maltodextrin (MD; Aldrich; 9.64 g; ~3.21 mmole; DE (Dextrose Equivalent): 4.0 - 7.0) was dissolved in dimethylsulfoxide (DMSO) 60 mL. The size of the maltodextrin was calculated to be in the range of 2,000 Da - 4,000 Da. A solution of EA-NCO from Example 8 (24.73 g; 19.3 mmole) was evaporated and dissolved in dried DMSO (7.5 mL). The two DMSO solutions were mixed and heated to 55°C overnight. The DMSO solution was placed in dialysis tubing (1000 MWCO, 45 mm flat width x 50 cm long) and dialyzed against water for 3 days. The macromer solution was filtered and lyophilized to give 7.91 g white solid. A sample of the macromer (49 mg), and DBB (4.84 mg) was dissolved in 0.8 mL DMSO-d₆: ¹H NMR (DMSO-d₆, 400 MHz) δ 7.38 (s, 4H; internal std. integral value of 2.7815), 6.50, 6.19, and 5.93 (doublets, 3H; vinyl protons integral value of 3.0696). The calculated acrylate load of macromer was 0.616 µmoles/mg of polymer.

### Example 10

### Preparation of Maltodextrin-maleimide macromer (MD-Mal)

A procedure similar to Example 9 was used to make the MD-Mal macromer. A solution of Mal-C5-NCO from Example 4 (0.412 g; 1.98 mmole) was evaporated and dissolved in dried DMSO (2 mL). MD (0.991 g; 0.33 mmole) was dissolved in DMSO (5 mL). The DMSO solutions were combined and stirred at 55°C for 16 hours. Dialysis and lyophilization gave 0.566 g product. A sample of the macromer (44 mg), and DBB (2.74 mg) was dissolved in 00.8 mL DMSO-d₆: ¹H NMR (DMSO-d₆, 400 MHz) δ 7.38 (s, 4H; internal std. integral value of 2.3832), 6.9 (s, 2H; Maleimide protons integral value of 1.000). The calculated acrylate load of macromer was 0.222 µmoles/mg of polymer. The macromer was tested for its ability to make a matrix (see Example 13).

### Example 11

### Formation of Maltodextrin-acrylate biodegradable matrix using MTA-PAAm

250 mg of MD-Acrylate as prepared in Example 9 was placed in an 8 mL amber vial. To the MD-Acrylate was added 3 mg of MTA-PAAm (lyophilized), 2 µL of 2 NVP, and 1 mL of 1X phosphate-buffered saline (1X PBS), providing a composition having MD-Acrylate at 250 mg/mL. The reagents were then mixed for one hour on a shaker at 37°C. The mixture in an amount of 50 µL was placed onto a glass slide and illuminated for 40 seconds with an EFOS 100 SS illumination system equipped with a 400-500 nm filter. After illumination the polymer was found to form a semi-firm gel having elastomeric properties.

### Example 12

### Formation of MD-Acrylate biodegradable matrix using camphorquinone

250 mg of MD-acrylate as prepared in Example 9 was placed in an 8mL amber vial. To the MD-Acrylate was added 14 mg of camphorquinone-10-sulfonic acid hydrate (Toronto Research Chemicals, Inc.), 3 µL of 2-NVP, and I mL of distilled water. The reagents were then mixed for one hour on a shaker at 37°C. The mixture in an amount of 50 µL was placed onto a glass slide and illuminated for 40 seconds with a SmartliteIQ^{™} LED curing light (Dentsply Caulk). After illumination the polymer was found to form a semi-firm gel having with elastomeric properties.

### Example 13

### Formation of MD-Mal biodegradable matrix using MTA-PAAm

250 mg of MD-Mal as prepared in Example 10 was placed in an 8 mL amber vial. To the MD-Mal was added 3 mg of MTA-PAAm (lyophilized), 2µL of 2-NVP, and 1 mL of 1X phosphate-buffered saline (1X PBS). The reagents were then mixed for one hour on a shaker at 37°C. The mixture in an amount of 50 µL was placed onto a glass slide and illuminated for 40 seconds with an EFOS 100 SS illumination system equipped with a 400-500 nm filter. After illumination the polymer was found to form a semi-firm gel having elastomeric properties.

### Example 14

### Bioactive agent incorporation/release from a MD-Acrylate Matrix

500 mg of MD-Acrylate as prepared in Example 9 was placed in an 8 mL amber vial. To the MD-Acrylate was added 3 mg of MTA-PAAm (lyophilized), 2 µL of 2-NVP, and 1 mL of 1X phosphate-buffered saline (1X PBS). The reagents were then mixed for one hour on a shaker at 37°C. To this mixture was added either 5 mg 70kD FITC-Dextran or 5mg 10kD FITC-Dextran (Sigma) and vortexed for 30 seconds. The mixture in an amount of 200 µL was placed into a Teflon well plate (8mm diameter, 4mm deep) and illuminated for 40 seconds with an EFOS 100 SS illumination system equipped with a 400-500 nm filter. The formed matrix was loose, and not as well crosslinked as the formed MD-acrylate matrix in Example 13. After illumination, the matrix was transferred to a 12 well plate (Falcon) and placed in a well containing 0.6 mL PBS. At daily intervals for 6 days, 150 µL of PBS was removed from each well and placed into a 96 well plate. The remaining 850 µL were removed from the samples, and replaced with 1 mL fresh PBS. The 96 well plate was analyzed for FITC-Dextran on a spectrophotometer (Shimadzu) at 490 absorbance. Results showed that at least 70% of the detectable 10kd or 70kD FITC-Dextran was released from the matrix after 2 days. Visual observation showed that an unquantified amount of 10 kD or 70kD FITC-Dextran remained within the matrix after 6 days.

### Example 15

### Polyalditol-acrylate synthesis

Polyalditol (PA; GPC; 9.64 g; ~3.21 mmole) was dissolved in dimethylsulfoxide (DMSO) 60 mL. The size of the polyalditol was calculated to be in the range of 2,000 Da - 4,000 Da. A solution of EA-NCO from Example 8 (24.73 g; 19.3 mmole) was evaporated and dissolved in dried DMSO (7.5 mL). The two DMSO solutions were mixed and heated to 55°C overnight. The DMSO solution was placed in dialysis tubing (1000 MWCO, 45 mm flat width x 50 cm long) and dialyzed against water for 3 days. The polyalditol macromer solution was filtered and lyophilized to give 7.91 g white solid. A sample of the macromer (49 mg), and DBB (4.84 mg) was dissolved in 0.8 mL DMSO-d₆: ¹H NMR (DMSO-d₆, 400 MHz) 8 7.38 (s, 4H; internal std. integral value of 2.7815), 6.50, 6.19, and 5.93 (doublets, 3H; vinyl protons integral value of 3.0696). The calculated acrylate load of macromer was 0.616 µmoles/mg of polymer.

### Example 16

### Maltodextrin-acrylate Filaments

1,100 milligrams of MD-Acrylate as prepared in Example 9 was placed in an 8 mL amber vial. To the MD-Acrylate was added 1 mg of a photoinitiator 4,5-bis(4-benzoylphenyl-methyleneoxy) benzene-1,3-disulfonic acid (5 mg) (DBDS) and 1 mL of 1X .phosphate-buffered saline (PBS). The reagents were then mixed for one hour on a shaker at 37°C. The mixture in an amount of 10 uL was injected, using a 23 gauge needle, into a 22 mm length opaque silicone tube (P/N 10-447-01; Helix Medical, Carpinteria, CA). The tubing was placed into a Dymax Lightweld PC-2 illumination system (Dymax Corp.; light intensity 6.5 mW/cm²), 15 cm from light source, illuminated for 270 seconds, and then removed. After illumination, the filament was removed from the silicone tubing by rolling a pencil over the tubing, starting from the back. The filament was firm, which indicated complete polymerization of the MD-Acrylate. No excess liquid was observed. The filament was manipulated with forceps. Maltodextrin filaments were also made from a MD-acrylate solution having concentration of 200 mg/mL. These are physically firm and same as 1,100mg/ml.

### Example 17

### Polyalditol-acrylate Filaments

1,500 milligrams of polyalditol-acrylate as prepared in Example 15 was placed in an 8ml amber vial. To the polyalditol-acrylate was added 1 mg of DBDS (lyophilized), 15 mg Bovine Serum Albumin, and 200 uL of 1X phosphate-buffered saline (PBS). The reagents were then mixed for one hour on a shaker at 37°C. The mixture in an amount of 10 uL was injected, using a 23 gauge needle, into a 22 mm length opaque silicone tube (P/N 10-447-01; Helix Medical, Carpinteria, CA). The tubing was placed into a Dymax Lightweld PC-2 illumination system (Dymax Corp.; light intensity 6.5 mW/cm²), 15 cm from light source, illuminated for 270 seconds, and then removed. After illumination, the filament was removed from the silicone tubing by rolling a pencil over the tubing, starting from the back. The filament was firm, which indicated complete polymerization of the polyalditol-acrylate. No excess liquid was observed. The filament was manipulated with forceps

### Example 18

### Amylase Degradation of Maltodextrin-acrylate Filaments

Maltodextrin-acrylate filaments were synthesized using 200 mg/mL and 1100 mg/mL MD-acrylate as described in Example 16 and were tested for degradation in Amylase solutions. These filaments were placed in microcentrifuge tubes containing 1 mL of either 1X PBS (control), 1X PBS containing alpha-Amylase at 0.121 µg/mL (Sigma; catalog # A6814), or 1X PBS containing alpha-Amylase at 24 µg/mL. The tubes were then placed in an incubator at 37°C.

After 2 days in the PBS with the 0.121 µg/mL alpha-Amylase solution the 200 mg/mL filament was completely degraded, and no trace of the filament was observable. The 200 mg/mL filament in PBS (control) showed no signs of degradation.

After 33 days in the 1X PBS containing alpha-Amylase at 0.121 µg/mL, the 1100 mg/mL filament had lost some of its initial firmness (as noted by the slightly curled appearance of the filament), but was still completely intact. The 1,100 mg/mL filament in the PBS with 24 ug Amylase had completely degraded after 48 hours. The 1,100 mg/ml filament in the PBS showed no signs of degradation.

### Example 19

### Maltodextrin-acrylate Filaments with Bioactive Agent and Release

MD-Acrylate in an amount of 1,100 milligrams of as prepared in Example 9 was placed in an 8ml amber vial. To the MD-Acrylate was added 1 mg of DBDS (lyophilized), 15 mg Bovine Serum Albumin (representing the bioactive agent; and 1 mL of 1X phosphate-buffered saline (1X PBS). The reagents were then mixed for one hour on a shaker at 37°C. The mixture in an amount of 10 uL was injected, using a 23 gauge needle, into a 22 mm length opaque silicone tube (P/N 10-447-01; Helix Medical, Carpinteria, CA). The tubing was placed into a Dymax Lightweld PC-2 illumination system (Dymax Corp.; light intensity 6.5 mW/cm²), 15 cm from light source, illuminated for 270 seconds, and then removed. After illumination, the filament was removed from the silicone tubing by rolling a pencil over the tubing, starting from the back. The filament was firm, which indicated complete polymerization of the MD-Acrylate. No excess liquid was observed.

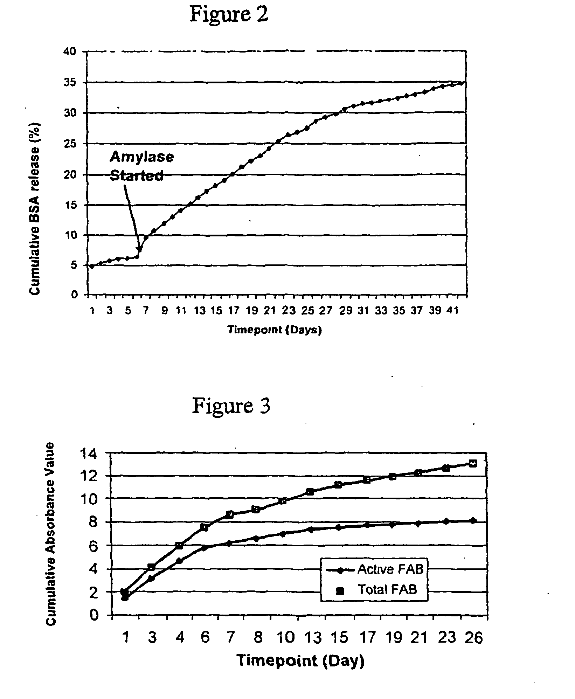

The filament was placed in a 1.7 ml microcentrifuge tube with 1 ml 1X PBS. At daily intervals for 6 days, 150 µL of PBS was removed from each well and placed into a 96 well plate for subsequent analysis. The remaining 850 µL was removed from the sample, and to the tube was added 1 ml of 1X PBS. After 6 days, the filament was placed in a 1.7 ml microcentrifuge tube with 1X PBS containing alpha-Amylase at 0.121 µg/mL. At daily intervals for 35 days, 150 µL of PBS was removed from each well and placed into a 96 well plate for subsequent analysis. The remaining 850 µL was removed from the sample, and to the tube was added 1 ml of fresh 1X PBS containing alpha-Amylase at 0.121 µg/mL. The 96-well plate was analyzed for BSA using the Quanitpro Assay Kit (Sigma). For the first 6 days, there was an initial burst of BSA, followed by a very slow release. After the addition of PBS + Amylase, the rate of BSA release significantly increased, and was relatively constant over the next 35 days. Results are shown in Table 2 and Figure 2.

**Table 2**

| Timepoint | Cumulative BSA release (% of Total BSA) | Timepoint | Cumulative BSA release (% of Total BSA) |
|---|---|---|---|
| 1 | 4.8 | 22 | 25.35 |
| 2 | 5.35 | 23 | 26.31 |
| 3 | 5.7 | 24 | 26.91 |
| 4 | 5.98 | 25 | 27.51 |
| 5 | 6.19 | 26 | 28.63 |
| 6 | 6.36 | 27 | 29.19 |
| 7 | 9.46 | 28 | 29.75 |
| 8 | 10.7 | 29 | 30.44 |
| 9 | 11.82 | 30 | 31.11 |
| 10 | 12.94 | 31 | 31.43 |
| 11 | 14.01 | 32 | 31.63 |
| 12 | 15.06 | 33 | 31.83 |
| 13 | 16.11 | 34 | 32:07 |
| 14 | 17.23 | 35 | 32.31 |
| 15 | 18.11 | 36 | 32.72 |
| 16. | 19.04 | 37 | 32.95 |
| 17 | 19.92 | 38 | 33.27 |
| 18 | 21.26 | 39 | 33.83 |
| 19 | 22.15 | 40 | 34.15 |
| 20 | 23.04 | 41 | 34.43 |
| 21 | 24.06 | 42 | 34.71 |

### Example 20

### Polyalditol-acrylate Filaments with Bioactive Agent and Release

Polyaldtiol-acrylate in an amount of 1,500 mg of as prepared in Example 15 was placed in an 8ml amber vial. To the PA-Acrylate was added 1 mg of DBDS (lyophilized), 15 mg Bovine Serum Albumin, and 1 mL of 1X phosphate-buffered saline (1X PBS). The reagents were then mixed for one hour on a shaker at 37°C. The mixture in an amount of 10 uL was injected, using a 23 gauge needle, into a 22 mm length opaque silicone tube (P/N 10-447-01; Helix Medical, Carpinteria, CA). The tubing was placed into a Dymax Lightweld PC-2 illumination system (Dymax Corp.; light intensity 6.5 mW/cm²), 15 cm from light source, illuminated for 270 seconds, and then removed. After illumination, the filament was removed from the silicone tubing by rolling a pencil over the tubing, starting from the back. The filament was firm, which indicated complete polymerization of the polyalditol-acrylate. No excess liquid was observed. The filament was manipulated with forceps.

The filament was placed in a 1.7 ml microcentrifuge tube with 1 ml PBS containing alpha-Amylase at 0.121 µg/mL. At daily intervals for 15 days, 150 µl of PBS was removed from each well and placed into a 96 well plate for subsequent analysis. The remaining 850 µL was removed from the sample, and to the tube was added 1 ml of fresh PBS containing alpha-Amylase at 0.121 µg/mL. The 96-well plate was analyzed for BSA using the Quanitpro Assay Kit (Sigma).

### Example 21

### Maltodextrin-acrylate Filaments with Bioactive Agent and Release

Maltodextrin filaments were synthesized using a 1,100 mg/mL solution as described in Example 19 using an anti-horseradish peroxidase antibody (P7899; Sigma) instead of BSA. The filament contained 800 ug of the anti-horseradish peroxidase antibody. The filament was placed in a 1.7 ml microcentrifuge tube containing 1 ml of 1X PBS containing alpha-Amylase at 0.121 µg/mL. At daily intervals for 5 days, 100 µl of PBS was removed from the sample, placed into a 96 well plate and incubated for 60 minutes at 37°C. The remaining 850 µL was removed from the sample, and replaced with 1 ml fresh 1X PBS containing alpha-Amylase at 0.121 µg/mL. After 1 hour, the plate was washed three times with 1 ml PBS/Tween (Sigma). 150 ul StabilCoat™ Immunoassay Stabilizer (SurModics, Eden Prairie, MN) was added to the well and incubated for 30 minutes at room temperature. After 30 minutes, the 96-well plate was washed three times with PBS/Tween. A solution of 0.5 mg/ml Horseradish Peroxidase (Sigma) in 1X PBS (100 uL) was added to the well and incubated for 60 minutes. After 60 minutes, the 96-well plate was washed six times with PBS/Tween. A chromogenic assay was then performed. After 15 minutes, the 96 well plate was analyzed for HRP conjugate on a spectrophotometer (Tecan) at 560 nm absorbance. Detectable Antibody was found at each time point.

### Example 22

### Degradation of MD-Acrylate filament in Vitreal Fluid

A circumferential dissection of the anterior segment (cornea, aqueous humour, lens) of porcine eye was performed, and the vitreous was squeezed out from the globe into a 20 mL amber vial; approx 10 mL total was retrieved from a total of four eyes. 200 mg/mL and 1100 mg/mL Maltodextrin filaments, formed in Example 15, were placed into 2 mL of the vitreous solution, and placed at 37°C on a rotator plate. The 200 mg/mL filament had completely dissolved after 24 hours. The 1,100 mg/mL filaments completely degraded after 30 days in the vitreous.

### Example 23

### Formation of a Maltodextrin-acrylate biodegradable matrix using REDOX chemistry

Two solutions were prepared. Solution #1 was prepared as follows: 250 mg of MD-acrylate as prepared in Example 9 was placed in an 8 mL vial. To the MD-acrylate was added 15 mg ferrous gluconate hydrate (Sigma), 30 mg Ascorbic Acid (Sigma), 67 uL AMPS (Lubrizol) and 1,000 uL deionized water. Solution #2 was prepared as follows: 250 mg of MD-acrylate as prepared in Example 9 was placed in a second 8 mL vial. To this MD-acrylate was added 30 uL AMPS, 80 uL Hydrogen Peroxide (Sigma) and 890 uL 0.1 M Acetate buffer (pH 5.5).

50 uL of Solution #1 was added to a glass slide. 50 uL of solution #2 was added to Solution #1 with slight vortexing. After mixing for 2 seconds, the mixture polymerized and formed a semi-firm gel having elastomeric properties.

### Example 24

### Formation of Maltodextrin-acrylate Biodegradable Matrix using REDOX Chemistry

Two solutions were prepared, similar to Example 23, but in this Example Solution #1 different concentrations of ferrous gluconate hydrate (Sigma) and ascorbic acid were used. Solution #1 was prepared as follows: 250 mg of MD-acrylate (as prepared in Example 9) was placed in an 8 mL vial. To the MD-acrylate was added 5 mg ferrous gluconate hydrate (Sigma), 40 mg ascorbic acid (Sigma), 67 uL AMPS (Lubrizol) and 1,000 uL deionized water. Solution #2 was prepared as follows: 250 mg of MD-acrylate as prepared in Example 3 was placed in a second 8 mL vial. To this MD-acrylate was added 30 uL AMPS, 80 uL Hydrogen Peroxide (Sigma) and 890 uL 0.1 M Acetate buffer (pH 5.5).

50 uL of Solution #1 was added to a glass slide. 50 uL of solution #2 was added to Solution #1 with slight vortexing. After mixing for 8 seconds, the mixture polymerized and formed a semi-firm gel having elastomeric properties.

### Example 25

### Formation of Maltodextrin-acrylate Biodegradable Matrix using REDOX Chemistry

Two solutions were prepared. Solution #1 was prepared as follows: 250 mg of MD-acrylate (as prepared in Example 9) was placed in an 8 mL vial. To the MD-acrylate was added 15 mg Iron (II) L-Ascorbate (Sigma), 30 mg Ascorbic Acid (Sigma), 67 uL AMPS (Lubrizol) and 1,000 uL deionized water. Solution #2 was prepared as follows: 250 mg of MD-acrylate as prepared in Example 3 was placed in a second 8 mL vial. To this MD-acrylate was added 30 uL AMPS, 80 uL hydrogen peroxide (Sigma) and 890 uL 0.1 M Acetate buffer (pH 5.5).

50 uL of Solution #1 was added to a glass slide. 50 uL of solution #2 was added to Solution #1 with slight vortexing. After mixing for 2 seconds, the mixture polymerized and formed a semi-firm gel having elastomeric properties.

### Example 26

### Formation of Polyalditol-acrylate Biodegradable matrix using REDOX chemistry

Two solutions were prepared. Solution #1 was prepared as follows: 1,000 mg of Polyalditol-acrylate as prepared in Example 15 was placed in an 8 mL vial. To the Polyalditol-acrylate was added 15 mg Ferrous Sulfate Heptahydrate (Sigma), 30 mg Ascorbic Acid (Sigma), 67 uL AMPS (Lubrizol) and 1,000 uL deionized water. Solution #2 was prepared as follows: 1,000 mg of Polyalditol-acrylate as prepared in Example 15 was placed in a second 8 mL vial. To this Polyalditol-acrylate was added 30 uL AMPS, 80 uL Hydrogen Peroxide (Sigma) and 890 uL 0.1 M Acetate buffer (pH 5.5).

50 uL of Solution #1 was added to a glass slide. 50 uL of solution #2 was added to Solution #1 with slight vortexing. After mixing for 2 seconds, the mixture polymerized and formed a semi-firm gel having elastomeric properties.

### Example 27

### Bioactive agent incorporation into a MD-Acrylate Matrix

Two solutions were prepared. Solution #1 was prepared as follows: 250 mg of MD-acrylate (as prepared in Example 9) was placed in an 8 ml vial. To the MD-acrylate was added 15 mg Iron (II) Acetate (Sigma), 30 mg Ascorbic Acid (Sigma), 67 ul AMPS (Lubrizol), 75 mg Bovine Serum Albumin (BSA; representing the bioactive agent) and 1,000 µL deionized water. Solution #2 was prepared as follows: 250 mg of MD-acrylate was placed in a second 8 ml vial. To this MD-acrylate was added 30 µL AMPS, 80 µL Hydrogen Peroxide (Sigma), 75 mg BSA and 890 µL Acetate buffer (pH 5.5).

50 µL of Solution #1 was added to a glass slide. 50 µL of solution #2 was added to Solution #1 with slight vortexing. After mixing for 2 seconds, the mixture polymerized and formed a semi-firm gel having elastomeric properties.

### Example 28

### Enzyme Degradation of a MD-Acrylate Matrix formed by REDOX

Maltodextrin-acrylate filaments were prepared using the reagents at concentrations as described in Example 23. These filaments were placed in microcentrifuge tubes containing 1 ml either Phosphate Buffered Saline (PBS) or 1X PBS containing alpha-Amylase at 0.121 µg/mL. The tubes were then placed in an incubator at 37°C.

After 4 days in the 1X PBS containing alpha-Amylase at 0.121 µg/mL, the 250 mg/mL filament had completely degraded, leaving no trace of the matrix. The matrix in PBS showed no signs of degradation.

### Example 29

### FAB fragment incorporation and release from a MD-Acrylate Filament

600 milligrams of MD-Acrylate as prepared in Example 9 was placed in an 8 mL amber vial. To the MD-Acrylate was added 5 mg of DBDS (lyophilized), 10 mg Rabbit Anti-Goat Fragment Antibody (catalog # 300-007-003; Jackson Immunological Research, West Grove, PA) and 1 mL of 1X phosphate-buffered saline (PBS). The reagents were then mixed for one hour on a shaker at 37°C. The mixture in an amount of 10 µL was pipetted into a 22 mm length opaque silicone tube (P/N 10-447-01; Helix Medical, Carpinteria, CA). The tubing was placed into a Dymax Lightweld PC-2 illumination system (Dymax Corp.; light intensity 6.5 mW/cm²), 15 cm from light source, illuminated for 270 seconds, and then removed. After illumination, the filament was removed from the silicone tubing by rolling a pencil over the tubing, starting from the back. The filament was firm and completely crosslinked, with no excess liquid.

The filament was placed in a 1.7 mL microcentrifuge tube with 0.5 ml 1X PBS containing alpha-Amylase at 0.121 µg/mL (eluent solution). At predetermined intervals for 17 days, 200 µL of the eluent solution was removed from each tube, and 100 µL was placed into two 96 well plates. The remaining 300 µL were removed from the samples, and replaced with 0.5 mL fresh 1X PBS containing alpha-Amylase at 0.121 µg/mL. The 96 well plates were analyzed for total FAB molecule release and FAB activity using an Enzyme-Linked Immunosorbent Assay (ELISA). Briefly, the 100 µL eluent solution was incubated at 37°C for one hour and then washed 3x with 2 ml PBS/Tween 20 (Sigma). The wells were blocked with 100 µL StabilCoat™ for 1 hour at room temperature and then washed 3x with 2 mL PBS/Tween 20. 100 uL of either 0.1 ug/mL (in PBS/Tween) HRP-labeled Goat IgG (Jackson Immunological; catalog #005-030-003) for molecule activity or 0.08 ug/mL (in PBS/Tween) HRP-labeled Goat anti-Rabbit IgG (Jackson Immunological; catalog #111-305-003) was incubated for 1 hour at 37°C. The wells were washed 6x with 2 mL PBS/Tween 20. 100 µL of TMB Microwell Peroxidase Substrate System (KPL, Catalog #50-76-00; Gaithersburg, MD) as added to each well. After 15 minutes, the 96 well plate was analyzed for HRP conjugate on a spectrophotometer (Tecan) at 650 nm absorbance. Detectable Antibody was found at each timepoint. Results are shown in Table 3 and Figure 3.

**Table 3: Fab Fragment release ABS values**

| Timepoint (Day) | Cumulative Active FAB Abs at 650 nm | Cumulative Total Fab Abs at 650 nm |
|---|---|---|
| 1 | 1.37 | 1.97 |
| 3 | 3.12 | 4.07 |
| 4 | 4.54 | 5.87 |
| 6 | 5.69 | 7.54 |
| 7 | 6.12 | 8.60 |
| 8 | 6.53 | 9.01 |
| 10 | 6.94 | 9.79 |
| 13 | 7.34 | 10.64 |
| 15 | 7.54 | 11.18 |
| 17 | 7.71 | 11.62 |
| 19 | 7.81 | 11.92 |
| 21 | 7.90 | 12.28 |
| 23 | 8.00 | 12.68 |
| 26 | 8.09 | 13.11 |

### Example 30

### Rabbit Antibody incorporation and release from a MD-Acrylate Filament

600 milligrams of MD-Acrylate as prepared in Example 9 was placed in an 8ml amber vial. To the MD-Acrylate was added 5 mg of DBDS (lyophilized), 16 mg Rabbit Antibody Anti-HRP (Sigma; catalog # P7899) and 1 ml of 1X phosphate-buffered saline (PBS). The reagents were then mixed for one hour on a shaker at 37°C. The mixture in an amount of 10 µL was pipetted into a 22 mm length opaque silicone tube (P/N 10-447-01; Helix Medical, Carpinteria, CA). The tubing was placed into a Dymax Lightweld PC-2 illumination system (Dymax Corp.; light intensity 6.5 mW/cm²), 15 cm from light source, illuminated for 270 seconds, and then removed. After illumination, the filament was removed from the silicone tubing by rolling a pencil over the tubing, starting from the back. The filament was firm and completely crosslinked, with no excess liquid.

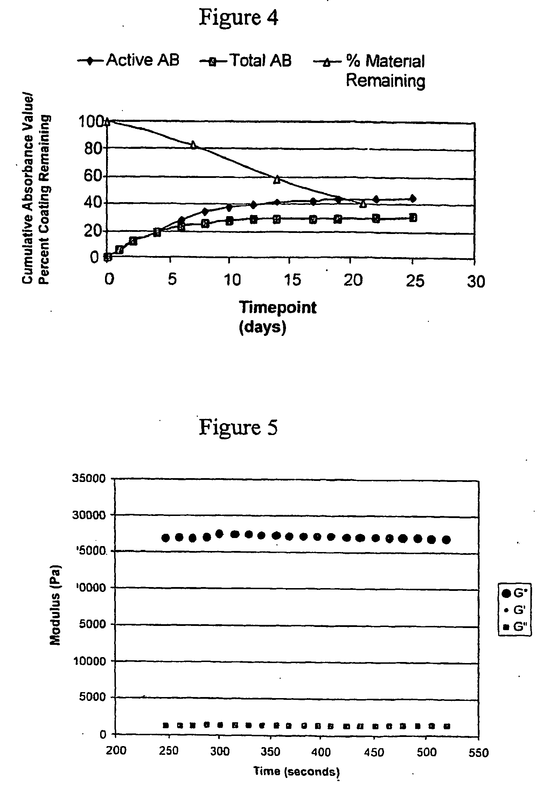

The filament was placed in a 1.7 ml microcentrifuge tube with 0.5 ml 1X PBS containing alpha-Amylase at 0.121 µg/mL (eluent solution). At predetermined intervals for 25 days, 200 µl of the eluent solution was removed from each tube, and 100 µL was placed into two 96 well plates. The remaining 300 µl were removed from the samples, and replaced with 0.5 ml fresh 1X PBS containing alpha-Amylase at 0.121 µg/mL. The 96 wellplates were analyzed for total Rabbit Antibody molecule release and activity using an Enzyme-Linked Immunosorbent Assay (ELISA). Briefly, the 100 µL eluent solution was added to the wells and incubated at 37 degrees C for one hour and then washed 3x with 2 ml PBS/Tween 20 (Sigma). The wells were blocked with 100 µL StabilCoat™ (SurModics) for 1 hour at room temperature and then washed 3x with 2 ml PBS/Tween 20. 100 µL of either 0.1 ug/ml (in PBS/Tween) HRP (Sigma; catalog # P8375) for molecule activity or 0.08 µg/ml (in PBS/Tween) HRP-labeled Goat anti-Rabbit IgG (Jackson Immunological; catalog # 111-305-003) was incubated for 1 hour at 37 degrees C. The wells were washed 6x with 2 ml PBS/Tween 20. 100 µL ofTMB Microwell Peroxidase Substrate System (KPL, Catalog # 50-76-00; Gaithersburg, MD) was added to each well. After 15 minutes, the 96 well plate was analyzed for HRP conjugate on a spectrophotometer (Tecan) at 650 nm absorbance. Detectable Antibody was found at each time point.
Results are shown in Table 4 and Figure 4.

**Table 4**

| Timepoint (Day) | Cumulative Active IgG release (%) (ELISA) | Cumulative Total IgG release (%) (ELISA) | MD-acrylate coating remaining (%) | Maximum theoretical total IgG release (%) |
|---|---|---|---|---|
| 1 | 5.56 | 5.31 | | |
| 2 | 12.13 | 11.94 | | |
| 4 | 18.38 | 19.13 | | |
| 6 | 27.75 | 22.88 | | |
| 7 | | | 83 | 17 |
| 8 | 33.50 | 25.44 | | |
| 10 | 37.63 | 27.44 | | |
| 12 | 39.50 | 28.31 | | |
| 14 | 40.75 | 28.57 | 59 | 31 |
| 17 | 41.75 | 28.76 | | |
| 19 | 42.75 | 28.98 | | |
| 21 | | | 40 | 60 |
| 22 | 43.44 | 29.67 | | |
| 25 | 44.31 | 30.67 | | |

### Example 31

### Mechanical testing of MD-acrylate discs formed via REDOX polymerization

MD-acrylate discs formed via redox polymerization of MD-acrylate coating solutions were tested for mechanical properties.

A first solution (#1) was prepared by placing 300 mg of MD-acrylate as prepared in Example 9 into an 8 ml vial and then adding 9 mg iron (II) ascorbate (Sigma), 30 mg ascorbic acid (Sigma), 67 µL AMPS (Lubrizol), and 1,000 µL deionized water. Solution #2 was prepared by placing 300 mg of MD-acrylate into a second 8 ml vial and then adding 30 µL AMPS, 80 µL if hydrogen peroxide (Sigma) and 890 µL of 0.1 M Acetate buffer (pH 5.5).

Viscosity of the first and second solutions were determined on a Brookfield Viscometer. The average viscosity for both solutions was 10.9 cP.

The modulus of the formed matrix was determined by rheological measurements. In order to perform rheological measurements, the first and second solutions were combined on the testing plate in the Rheometer (Rheometric Scientific; model # SR-2000) and the mixture was allowed to polymerize to form a matrix. Data recording began before sample was cured in plates. Briefly, 100 µL of solution #1 and 100 µL of solution #2 were mixed on the lower testing plate. As the matrix formed, the upper testing plate was lowered to fully contact the mixture of the first and second solutions as the mixture polymerized into a matrix. The sample was cured within 15 seconds. This curing method ensured maximum contact between the two testing plates resulting in more accurate testing compared to preformed matrices being placed between the testing plates.

The resulting MD-acrylate matrix had properties of an elastic solid with an elastic (storage) modulus ranging from 27 kPa to 30 kPa, and a viscous (loss) modulus of only about 1 kPa. Results are shown in Table 5 and Figure 5.

**Table 5: (Testing Conditions: Stress: 433 Pa; strain 1.6%; frequency: 1 radian/sec)**

| Time (seconds) | G' (Elastic Modulus; Pa) | G" (Storage Modulus; Pa) | G* (Loss Modulus; Pa) |
|---|---|---|---|
| 247 | 26820.2 | 1300.5 | 26851.7 |
| 261 | 26908.5 | 1294.55 | 26939.6 |
| 274 | 26872 | 1299.28 | 26903.4 |
| 288 | 26943.8 | 1343.69 | 26977.3 |
| 301 | 27376.6 | 1380.43 | 27411.4 |
| 315 | 27327.7 | 1373.31 | 27362.2 |
| 329 | 27319.8 | 1376.27 | 27354.5 |
| 342 | 27274.8 | 1362.35 | 27308.8 |
| 356 | 27246.6 | 1369.38 | 27281 |
| 369 | 27180.6 | 1373.6 | 27215.3 |
| 383 | 27174.4 | 1371.61 | 27209 |
| 397 | 27119.4 | 1366.76 | 27153.9 |
| 410 | 27105.4 | 1360.49 | 27139.5 |
| 424 | 27064.1 | 1358.45 | 27098.2 |
| 437 | 27019.9 | 1355.9 | 27053.9 |
| 451 | 27019.8 | 1355.39 | 27053.8 |
| 465 | 26972.3 | 1355.85 | 27006.4 |
| 478 | 26956.2 | 1361.11 | 26990.5 |
| 492 | 26918.2 | 1352.58 | 26952.2 |
| 505 | 26880.7 | 1355.85 | 26914.9 |
| 519 | 26840.4 | 1360.47 | 26874.9 |

### Example 32

### Preparation of Acylated Maltodextrin (Butyrylated-MD)

Maltodextrin having pendent butyryl groups were prepared by coupling butyric anhydride at varying molar ratios.

To provide butyrylated-MD (1 butyl/4 glucose units, 1:4 B/GU) the following procedure was performed. Maltodextrin (MD; Aldrich; 11.0 g; 3.67 mmole; DE (Dextrose Equivalent): 4.0 - 7.0) was dissolved in dimethylsulfoxide (DMSO) 600 mL with stirring. The size of the maltodextrin was calculated to be in the range of 2,000 Da - 4,000 Da. Once the reaction solution was complete, 1-methylimidazole (Aldrich; 2.0g, 1.9mls) and butyric anhydride (Aldrich; 5.0 g, 5.2 mls) was added with stirring. The reaction mixture was stirred for four hours at room temperature. After this time, the reaction mixture was quenched with water and dialyzed against DI water using 1,000 MWCO dialysis tubing. The butyrylated starch was isolated via lyophylization to give 9.315 g (85 % yield). NMR confirmed a butyrylation of 1:3 B/GU (1.99mmoles butyl/g sample).

To provide butyrylated-MD (1:8 B/GU), 2.5g (2.6 mL) butyric anhydride was substituted for the amount of butyric anhydride described above. A yield of 79% (8.741 g) was obtained. NMR confirmed a butyrylation of 1:5 B/GU (1.31 mmoles butyl/g sample).

To provide butyrylated-MD (1:2B/GU), 10.0g (10.4 mL) butyric anhydride was substituted for the amount of butyric anhydride described above. A yield of 96% (10.536 g) was obtained. NMR confirmed a butyrylation of 1:2 B/GU (3.42 mmoles butyl/g sample).

### Example 33

### Preparation of Acrylated Acylated Maltodextrin (Butyrylated-MD-Acrylate)

Preparation of an acylated maltodextrin macromer having pendent butyryl and acrylate groups prepared by coupling butyric anhydride at varying molar ratios.

To provide butyrylated-MD-acrylate (1 butyl/4 glucose units, 1:4 B/GU) the following procedure was performed. MD-Acrylate (Example 9; 1.1 g; 0.367 mmoles) was dissolved in dimethylsulfoxide (DMSO) 60 mL with stirring. Once the reaction solution was complete, 1-methylimidazole (0.20g, 0.19mls) and butyric anhydride (0.50 g, 0.52 mls) was added with stirring. The reaction mixture was stirred for four hours at room temperature. After this time, the reaction mixture was quenched with water and dialyzed against DI water using 1,000 MWCO dialysis tubing. The butyrylated starch acrylate was isolated via lyophylization to give 821 mg (75% yield, material lost during isolation). NMR confirmed a butyrylation of 1:3 B/GU (2.38 mmoles butyl/g sample).

### Example 34

### Preparation of Acrylated Acylated Maltodextrin (Butyrylated-MD-Acrylate

Maltodextrin having pendent butyryl and acrylate groups prepared by coupling butyric anhydride at varying molar ratios.

To provide butyrylated-MD-acrylate the following procedure is performed. Butyrylated-MD (Example 43; 1.0 g; 0.333 mmole) is dissolved in dimethylsulfoxide (DMSO) 60 mL with stirring. Once the reaction solution is complete, a solution ofEA-NCO from Example 8 (353 mg; 2.50 mmole) is evaporated and dissolved in dried DMSO 1.0 mL. The two DMSO solutions are mixed and heated to 55°C overnight. The DMSO solution is placed in dialysis tubing (1000 MWCO) and dialyzed against water for 3 days. The macromer solution is filtered and lyophilized to give a white solid.

### Example 35

### Formation of Polyalditol-acrylate Biodegradable Matrix Using REDOX Chemistry

Reductant and oxidant solutions including Polyalditol-acrylate (PD-A) were prepared (see Table 6). Oxidant solutions were prepared as follows: 500 mg of PD-A (as prepared in example 15) were individually placed in an 8 mL vial. To the PD-A was added various amounts of ammonium persulfate (Sigma) (see Table 6, rows A-H), potassium persulfate (see Table 6, rows M-P) or sodium persulfate (see Table 6, rows I-L) and 1,000 uL PBS. Reductant solutions were prepared as follows: 500 mg of PD-A was placed in a second 8 mL vial. To this PD-A was added either 20 uL or 40 uL TEMED, 40 uL 1N hydrochloric acid (VWR) and 960 uL Phosphate Buffered Saline (Sigma; pH 7.4).

Each polymerization experiment was performed as follows: 50 uL of oxidant solution was transferred to a glass slide; subsequently 50 uL of reductant solution was added to the oxidant solution. After mixing for 5 seconds at 23°C or 37°C, the mixture polymerized and formed gels having elastomeric properties.

**Table 6**

| | *PD-A* | *Oxidant* | *Oxidant Conc (mg*/*ml)* | *TEMED (u*/*ml)* | *Temperature (Celsius)* | *Crosslink time (secs)* | *Matrix properties* |
|---|---|---|---|---|---|---|---|
| A | 500 mg/mL | Ammonium | 10 | 20 ul | 23 | 240 s | Semi-firm gel |
| | | Persulfate | | | | | |
| B | 500 mg/mL | Ammonium | 15 | 20 | 23 | 120 s | Semi-firm gel |
| | | Persulfate | | | | | |
| C | 500 mg/mL | Ammonium | 50 | 20 | 23 | 60 s | Semi-firm gel |
| | | Persulfate | | | | | |
| D | 500mg/mL | Ammonium | 100 | 20 | 23 | 45 s | Semi-firm gel |
| | | Persulfate | | | | | |
| E | 500mg/mL | Ammonium | 10 | 40 | 23 | 120 s | Semi-firm gel |
| | | Persulfate | | | | | |
| F | 500mg/mL | Ammonium | 50 | 40 | 23 | 50 s | Semi-firm gel |
| | | Persulfate | | | | | |
| G | 500 mg/mL | Ammonium | 15 | 20 | 37 | 60 s | Semi-firm gel |
| | | Persulfate | | | | | |
| H | 500 mg/mL | Ammonium | 50 | 20 | 37 | 20 s | Semi-firm gel |
| | | Persulfate | | | | | |
| I | 500 mg/mL | Sodium | 5 | 20 | 23 | 600 s | Soft gel |
| | | Persulfate | | | | | |
| J | 500 mg/mL | Sodium | 10 | 20 | 23 | 360 s | Soft gel |
| | | Persulfate | | | | | |
| K | 500 mg/mL | Sodium | 5 | 20 | 37 | 90 s | Soft gel |
| | | Persulfate | | | | | |
| L | 500 mg/mL | Sodium | 10 | 20 | 37 | 90 s | Semi-firm gel |
| | | Persulfate | | | | | |
| M | 500 mg/mL | Potassium | 30 | 20 | 23 | 240 s | Semi-firm gel |
| | | Persulfate | | | | | |
| N | 500mg/mL | Potassium | 30 | 40 | 23 | 90 s | Semi-firm gel |
| | | Persulfate | | | | | |
| O | 500 mg/mL | Potassium | 30 | 20 | 37 | 75 s | Semi-firm gel |
| | | Persulfate | | | | | |
| P | 500mg/mL | Potassium | 30 | 40 | 37 | 30 s | Semi-firm gel |
| | | Persulfate | | | | | |

### Example 36

### Cell Viability Within Polyalditol-Acrylate REDOX Components

Solutions were prepared having the concentrations indicated in Table 7.

Cell suspensions were prepared as follows: PC-12 cells (ATCC; passage 5; 75% confluency) were harvested from a T-75 flask using Trypsin-EDTA for 2 minutes. The cells were placed in a 15 mL polyethylene conical (VWR) and centrifuged in F-12k media without serum for 4 minutes at 500 rpm. The cells were counted using a hemocytometer, centrifuged for 4 minutes at 500 rpm, and resuspended at 600,000 cells/mL in sterile PBS.

In order to determine the effect of individual components of the matrix forming compositions on cell viability, solutions A-G were independently mixed with PC-12 cells. 50 uL of cell suspension was added to 350 uL of solutions A-G (Table 7). After a 15 minute incubation, cell viability was assessed using a Live/Dead^{™} Viability/Cytotoxicity Kit (cat.# L3224; Molecular Probes, Eugene, OR).

The effect of a formed matrix on cell viability was also tested. To form the PD-A matrix, solution #1 (200 mg PD-A, 10 uL TEMED, 20 uL 1N HCl, 470 uL PBS) was added in the amount of 200 uL to a 1.6 ml eppendorf (VWR). Solution #2 (400 mg PD-A, 10 mg Potassium persulfate, 75K PC-12 cells, 500 uL PBS) was added in the amount of 200 uL to solution #1 in the eppendorf and mixed for 10 seconds. After a 15 minute incubation, cell viability was assessed using the Live/Dead^{™} Viability/Cytotoxicity Kit.

**Table 7**

| | *Component(s)* | *Concentration (in PBS)* | *Incubation time* | *Cell viability* (%) |
|---|---|---|---|---|
| A | TEMED/PBS | 0.2% (V/V) | 15 min | 10-30% |
| B | Sodium | 5 mg/ml | 15 min | 90% |
| | persulfate | | | |
| C | Potassium | 10 mg/ml | 15 min | 90% |
| | persulfate | | | |
| D | Ammonium | 10 mg/ml | 15 min | 90% |
| | persulfate | | | |
| E | Ammonium | 120 mg/ml | 15 min | 90% |
| | Persulfate | | | |
| F | PD-A | 400 mg/ml | 15 min | 90% |
| G | PD-A + TEMED | 400mg/ml +0.2% (v/v) | 15 min | 50% |
| H | PD-A matrix | | 15 min | 80% |
| I | PBS | | 15 min | 90% |

## Claims

1. A flowable composition comprising
(i) natural biodegradable polysaccharide comprising a coupling group,
(ii) an initiator, and
(iii) bioactive agent;
for forming a biodegradable implant *in situ,* in an eye of a patient by activation of the initiator to couple the natural biodegradable polysaccharides present in the composition, thereby forming a solid implant within the eye of the patient.

2. A composition according claim 1, wherein the biodegradable polysaccharide is selected from amylose and maltodextrin.

3. A composition according to claim 1, wherein the biodegradable polysaccharide comprises a non-reducing natural biodegradable polysaccharide.

4. A composition according to claim 1, wherein the biodegradable polysaccharide comprises a pendent retinoic acid group.

5. A composition according to claim 1, wherein the coupling group comprises a polymerizable group.

6. A composition according to claim 5, wherein the polymerizable group is selected from vinyl groups, acrylate groups, methacrylate groups, ethacrylate groups, phenyl acrylate groups, acrylamide groups, methacrylamide groups, itaconate groups, and styrene groups.

7. A composition according to claim 1, wherein the initiator comprises a photoinitiator.

8. A composition according to claim 1, for administration by injection of the composition into a targeted site within the eye of the patient.

9. A composition according to claim 8, wherein the targeted site is within the vitreous of the eye.

10. A composition according to claim 8, wherein the targeted site is a subretinal area of the eye.

11. A composition according to claim 1, wherein the activation of the initiator comprises applying light having a wavelength in a visible or long wavelength ultraviolet range.

12. A composition according to claim 11, wherein the activation comprises applying the light from a light source located within the interior of the eye.

13. A composition according to claim 11, wherein the activation comprises applying the light from a light source located externally from the eye.

14. A composition according to claim 1, wherein the activation of the initiator is performed after the composition has been administered to the patient.

15. A flowable mixture comprising a first composition comprising
(i) natural biodegradable polysaccharide comprising a pendent polymerizable group, and
(ii) a first member of a redox pair;
a second composition comprising
(i) natural biodegradable polysaccharide comprising a pendent polymerizable group, and
(ii) a second member of a redox pair;
for forming a biodegradable implant *in situ,* in an eye of a patient by administration of the first composition, the second composition, or a mixture of the first and second compositions in liquid form into the eye of a patient; and
contacting the first composition with the second composition where, in the contacting, the redox pair initiates polymerization of the natural biodegradable polysaccharides, thereby forming a solid implant within the eye.

16. A mixture according to claim 15, wherein the biodegradable polysaccharide is selected from amylose and maltodextrin.

17. A mixture according to claim 15, wherein the biodegradable polysaccharide comprises a non-reducing natural biodegradable polysaccharide.

18. A mixture according to claim 15, wherein the biodegradable polysaccharide of the first composition, the second composition, or both the first composition and second composition comprises a pendent retinoic acid group.

19. A mixture according to claim 15, wherein the administration comprises:
contacting the first composition with the second composition; and then
administering the mixture of the first and second compositions into the eye.

20. A mixture according to claim 15, wherein the administration comprises injecting the first composition, the second composition, or a mixture of the first and second composition in liquid form into the eye of a patient.

21. A mixture according to claim 15, wherein the natural biodegradable polysaccharide of the first composition is the same as the natural biodegradable polysaccharide of the second composition.

22. A mixture according to claim 15, wherein the redox pair comprises an oxidizing member selected from peroxides, metal oxides, and oxidases, and a reducing member selected from salts and derivatives of electropositive elemental metals and reductases.

23. A mixture according to claim 15, wherein the polymerizable group is selected from vinyl groups, acrylate groups, methacrylate groups, ethacrylate groups, 2-phenyl acrylate groups, acrylamide groups, methacrylamide groups, itaconate groups, and styrene groups.

24. A mixture according to claim 15, further comprising a bioactive agent included in the first composition, the second composition, or both the first composition and the second composition.

## Patentansprüche

1. Fließfähige Zusammensetzung, umfassend:
(i) ein natürliches, biologisch abbaubares Polysaccharid, das eine Bindungsgruppe umfasst,
(ii) einen Initiator und
(iii) ein bioaktives Mittel,
zur Ausbildung eines biologisch abbaubaren Implantats in situ in einem Auge eines Patienten durch Aktivierung des Initiators, sodass er die natürlichen, biologisch abbaubaren Polysaccharide, die in der Zusammensetzung vorhanden sind, bindet, wodurch ein festes Implantat im Auge des Patienten gebildet wird.

2. Zusammensetzung nach Anspruch 1, worin das biologisch abbaubare Polysaccharid aus Amylose und Maltodextrin ausgewählt ist.

3. Zusammensetzung nach Anspruch 1, worin das biologisch abbaubare Polysaccharid ein nichtreduzierendes, natürliches, biologisch abbaubares Polysaccharid umfasst.

4. Zusammensetzung nach Anspruch 1, worin das biologisch abbaubare Polysaccharid eine Retinsäure-Seitengruppe umfasst.

5. Zusammensetzung nach Anspruch 1, worin die Bindungsgruppe eine polymerisierbare Gruppe umfasst

6. Zusammensetzung nach Anspruch 5, worin die polymerisierbare Gruppe aus Vinylgruppen, Acrylatgruppen, Methacrylatgruppen, Ethacrylatgruppen, Phenylacrylatgruppen, Acrylamidgruppen, Methacrylamidgruppen, Itaconatgruppen und Styrolgruppen ausgewählt ist.

7. Zusammensetzung nach Anspruch 1, worin der Initiator einen Photoinitiator umfasst.

8. Zusammensetzung nach Anspruch 1 zur Verabreichung der Zusammensetzung mittels Injektion in eine Zielstelle innerhalb des Auges des Patienten.

9. Zusammensetzung nach Anspruch 8, wobei die Zielstelle sich innerhalb des Glaskörpers des Auges befindet.

10. Zusammensetzung nach Anspruch 8, wobei die Zielstelle ein subretinaler Bereich des Auges ist.

11. Zusammensetzung nach Anspruch 1, wobei die Aktivierung des Initiators das Anwenden von Licht mit einer Wellelänge im sichtbaren Bereich oder langwelligen Ultraviolettbereich umfasst.

12. Zusammensetzung nach Anspruch 11, wobei die Aktivierung das Anwenden von Licht von einer Lichtquelle umfasst, die sich im Inneren des Auges befindet.

13. Zusammensetzung nach Anspruch 11, wobei die Aktivierung das Anwenden von Licht von einer Lichtquelle umfasst, die sich außerhalb des Auges befindet.

14. Zusammensetzung nach Anspruch 1, wobei die Aktivierung des Initiators ausgeführt wird, nachdem die Zusammensetzung dem Patienten verabreicht wurde.

15. Fließfähiges Gemisch, das eine erste Zusammensetzung, umfassend
(i) ein natürliches, biologisch abbaubares Polysaccharid, das eine polymerisierbare Seitengruppe umfasst, und
(ii) ein erstes Element eines Redoxpaares,
eine zweite Zusammensetzung, umfassend:
(i) ein natürliches, biologisch abbaubares Polysaccharid, das eine polymerisierbare Seitengruppe umfasst, und
(iii) ein zweites Element eines Redoxpaares,
umfasst, zur Ausbildung eines biologisch abbaubaren Implantats in situ in einem Auge eines Patienten durch Verabreichung der ersten Zusammensetzung, der zweiten Zusammensetzung oder eines Gemischs aus der ersten und zweiten Zusammensetzung in flüssiger Form in das Auge eines Patienten und
durch Kontaktieren der ersten Zusammensetzung mit der zweiten Zusammensetzung, wenn beim Kontaktieren das Redoxpaar eine Polymerisation der natürlich abbaubaren Polysaccharide initiiert, wodurch ein festes Implantat im Auge gebildet wird.

16. Gemisch nach Anspruch 15, worin das biologisch abbaubare Polysaccharid aus Amylose und Maltrodextrin ausgewählt ist.

17. Gemisch nach Anspruch 15, worin das biologisch abbaubare Polysaccharid ein nichtreduzierendes, natürliches, biologisch abbaubares Polysaccharid umfasst.

18. Gemisch nach Anspruch 15, worin das biologisch abbaubare Polysaccharid der ersten Zusammensetzung, der zweiten Zusammensetzung oder sowohl der ersten als auch der zweiten Zusammensetzung eine Retinsäure-Seitengruppe umfasst.

19. Gemisch nach Anspruch 15, wobei die Verabreichung Folgendes umfasst:
Kontaktieren der ersten Zusammensetzung mit der zweiten Zusammensetzung und dann
Verabreichen des Gemischs aus der ersten und zweiten Zusammensetzung in das Auge.

20. Gemisch nach Anspruch 15, wobei die Verabreichung das Injizieren der ersten Zusammensetzung, der zweiten Zusammensetzung oder eines Gemischs aus der ersten und zweiten Zusammensetzung in flüssiger Form in das Auge eines Patienten umfasst.

21. Gemisch nach Anspruch 15, wobei das natürliche, biologisch abbaubare Polysaccharid der ersten Zusammensetzung das gleiche natürliche, biologisch abbaubare Polysaccharid wie in der zweiten Zusammensetzung ist.

22. Gemisch nach Anspruch 15, worin das Redoxpaar ein Oxidationsmittel, das aus Peroxiden, Metalloxiden und Oxidasen ausgewählt ist, und ein Reduktionsmittel, das aus Salzen und Derivaten von elektropositiven elementaren Metallen und Reductasen ausgewählt ist, umfasst.

23. Gemisch nach Anspruch 15, worin die polymerisierbare Gruppe aus Vinylgruppen, Acrylatgruppen, Methacrylatgruppen, Ethacrylatgruppen, 2-Phenylacrylatgruppen, Acrylamidgruppen, Methacrylamidgruppen, Itaconatgruppen und Styrolgruppen ausgewählt ist.

24. Gemisch nach Anspruch 15, das weiters ein bioaktives Mittel umfasst, das in der ersten Zusammensetzung, der zweiten Zusammensetzung oder sowohl der ersten als auch der zweiten Zusammensetzung enthalten ist.

## Revendications

1. Composition fluide comprenant
(i) un polysaccharide biodégradable naturel comprenant un groupe de couplage,
(ii) un initiateur, et
(iii) un agent bioactif ;
permettant de fabriquer un implant biodégradable *in situ* dans l'oeil d'un patient par activation de l'initiateur pour coupler les polysaccharides biodégradables naturels présents dans la composition, pour ainsi fabriquer un implant solide dans l'oeil du patient.

2. Composition selon la revendication 1, dans laquelle le polysaccharide biodégradable est choisi parmi l'amylose et la maltodextrine.

3. Composition selon la revendication 1, dans laquelle le polysaccharide biodégradable comprend un polysaccharide biodégradable naturel non réducteur.

4. Composition selon la revendication 1, dans laquelle le polysaccharide biodégradable comprend un groupe acide rétinoïque pendant.

5. Composition selon la revendication 1, dans laquelle le groupe de couplage comprend un groupe polymérisable.

6. Composition selon la revendication 5, dans laquelle le groupe polymérisable est choisi parmi les groupes vinyles, les groupes acrylate, les groupes méthacrylate, les groupes éthacrylate, les groupes phénylacrylate, les groupes acrylamide, les groupes méthacrylamide, les groupes itaconate et les groupes styrène.

7. Composition selon la revendication 1, dans laquelle l'initiateur comprend un photoinitiateur.

8. Composition selon la revendication 1, pour l'administration par injection de la composition dans un site ciblé de l'oeil du patient.

9. Composition selon la revendication 8, dans laquelle le site ciblé se situe dans le corps vitré de l'oeil.

10. Composition selon la revendication 8, dans laquelle le site ciblé est une région sous-rétinienne de l'oeil.

11. Composition selon la revendication 1, dans laquelle l'activation de l'initiateur comprend l'application d'une lumière ayant une longueur d'onde dans le domaine visible ou des ultraviolets à longueur d'onde élevée.

12. Composition selon la revendication 11, dans laquelle l'activation comprend l'application de lumière à partir d'une source lumineuse située à l'intérieur de l'oeil.

13. Composition selon la revendication 11, dans laquelle l'activation comprend l'application de lumière à partir d'une source lumineuse située à l'extérieur de l'oeil.

14. Composition selon la revendication 1, dans laquelle l'activation de l'initiateur est réalisée après que la composition a été administrée au patient.

15. Mélange fluide comprenant une première composition comprenant
(i) un polysaccharide biodégradable naturel comprenant un groupe polymérisable pendant, et
(ii) un premier élément d'une paire redox ;
une seconde composition comprenant
(i) un polysaccharide biodégradable naturel comprenant un groupe polymérisable pendant, et
(ii) un second élément d'une paire redox ;
permettant de fabriquer un implant biodégradable *in situ* dans l'oeil d'un patient par administration de la première composition, de la seconde composition ou d'un mélange des première et seconde compositions sous une forme liquide dans l'oeil d'un patient ; et
la mise en contact de la première composition avec la seconde composition où, lors de la mise en contact, la paire redox déclenche la polymérisation des polysaccharides biodégradables naturels, pour ainsi fabriquer un implant solide dans l'oeil.

16. Mélange selon la revendication 15, dans lequel le polysaccharide biodégradable est choisi parmi l'amylose et la maltodextrine.

17. Mélange selon la revendication 15, dans lequel le polysaccharide biodégradable comprend un polysaccharide biodégradable naturel non réducteur.

18. Mélange selon la revendication 15, dans lequel le polysaccharide biodégradable de la première composition, de la seconde composition, ou des première et seconde compositions comprend un groupe acide rétinoïque pendant.

19. Mélange selon la revendication 15, dans lequel l'administration comprend :
la mise en contact de la première composition avec la seconde composition ; puis l'administration du mélange des première et seconde compositions dans l'oeil.

20. Mélange selon la revendication 15, dans lequel l'administration comprend l'injection de la première composition, de la seconde composition ou d'un mélange des première et seconde compositions sous une forme liquide dans l'oeil d'un patient.

21. Mélange selon la revendication 15, dans lequel le polysaccharide biodégradable naturel de la première composition est identique au polysaccharide biodégradable naturel de la seconde composition.

22. Mélange selon la revendication 15, dans lequel la paire redox comprend un élément oxydant choisi parmi les peroxydes, les oxydes de métaux et les oxydases, et un élément réducteur choisi parmi les sels et les dérivés des métaux élémentaires électropositifs et des réductases.

23. Mélange selon la revendication 15, dans lequel le groupe polymérisable est choisi parmi les groupes vinyles, les groupes acrylate, les groupes méthacrylate, les groupes éthacrylate, les groupes 2-phénylacrylate, les groupes acrylamide, les groupes méthacrylamide, les groupes itaconate et les groupes styrène.

24. Mélange selon la revendication 15, comprenant en outre un agent bioactif inclus dans la première composition, la seconde composition, ou les première et seconde compositions.
